(19) Europäisches Patentamt
European Patent Office
Office européen des brevets

(11) EP 1 044 586 B1

(12) EUROPEAN PATENT SPECIFICATION

(45) Date of publication and mention of the grant of the patent:
**01.02.2006 Bulletin 2006/05**

(21) Application number: **98953300.5**

(22) Date of filing: **08.10.1998**

(51) Int Cl.:
***H05B 33/28*** *(2006.01)* ***H01L 51/50*** *(2006.01)*

(86) International application number:
**PCT/US1998/021171**

(87) International publication number:
**WO 1999/020081 (22.04.1999 Gazette 1999/16)**

# (54) METHOD FOR FABRICATING HIGHLY TRANSPARENT NON-METALLIC CATHODES

VERFAHREN ZUR HERSTELLUNG HOCHTRANSPARENTER NICHTMETALLISCHER KATHODEN

PROCEDE POUR LA FABRICATION DES CATHODES NON METALLIQUES HAUTEMENT TRANSPARENTES

(84) Designated Contracting States:
**DE FR GB**

(30) Priority:
**09.10.1997 US 948130**
**03.11.1997 US 64005 P**
**05.11.1997 US 964863**
**01.12.1997 US 980986**
**01.04.1998 US 53030**
**03.04.1998 US 54707**
**10.04.1998 US 58305**
**14.09.1998 US 152960**

(43) Date of publication of application:
**18.10.2000 Bulletin 2000/42**

(60) Divisional application:
**03025325.6 / 1 394 870**
**05004965.9 / 1 536 496**

(73) Proprietors:
- **THE TRUSTEES OF PRINCETON UNIVERSITY**
**Princeton, NJ 08544-0036 (US)**
- **THE UNIVERSITY OF SOUTHERN CALIFORNIA**
**Los Angeles,**
**California 90089 (US)**

(72) Inventors:
- **FORREST, Stephen, R.**
**Princeton, NJ 08540 (US)**
- **BURROWS, Paul**
**Princeton Junction, NJ 08550 (US)**
- **PARTHASARATHY, Gautam**
**Princeton, NJ 08540 (US)**
- **O'BRIEN, Diarmuid**
**Dublin 9 (IE)**
- **THOMPSON, Mark, E.**
**Anaheim, CA 92807 (US)**
- **YU, Yujian**
**North Wales, PA 19454 (US)**
- **SHOUSTIKOV, Andrei**
**Los Angeles, CA 90007 (US)**
- **PETASIS, Nicos, A.**
**Hacienda Heights, CA 91745 (US)**
- **SIBLEY, Scott**
**Baltimore, MD 21236 (US)**
- **LOY, Douglas,**
**Lakewood, CA 90715 (US)**
- **KOENE, Brian, E.**
**Burlington, MA 01803 (US)**
- **KWONG, Raymond, C.**
**Levittown,**
**PA 19054 (US)**

(74) Representative: **Beetz & Partner**
**Steinsdorfstrasse 10**
**80538 München (DE)**

(56) References cited:
**EP-A- 0 704 915**
**US-A- 5 203 974**
**US-A- 5 457 565**

- **WHITLOCK et al., "Investigations of Materials and Device Structures for Organic Semiconductor Solar Cells", OPTICAL ENG., Vol. 32, No. 8, (August 1993), pages 1921-1934, XP002918896**
- **BULOVIC et al., "Transparent Light-Emitting Devices", NATURE, Vol. 380, pp. 29, (1996), XP002918897**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

**Description**

## FIELD OF INVENTION

The present invention is directed to a method of fabricating a cathode that may be used in optoelectronic devices, particularly, highly transparent non-metallic cathodes. More specifically, the present invention is directed to a method of fabricating organic light emitting devices (OLEDs) that are comprised of highly transparent non-metallic cathodes.

## BACKGROUND OF THE INVENTION

Optoelectronic devices include those which convert electrical energy into optical energy, or vice versa, as well as those that detect optical signals through electronic processes. Such devices include photodetectors, phototransistors, solar cells, light emitting diodes and lasers. Such devices typically include a pair of electrodes with at least one charge-carrying layer between the electrodes. Dependent on the function of the device, the charge-carrying layer or layers may be comprised of a material or materials that are electroluminescent when a voltage is applied across the device or the layer or layers may form a heterojunction capable of generating a photovoltaic effect when exposed to optical radiation.

In particular, OLEDs are comprised of several organic layers in which one of the layers is comprised of an organic material that can be made to electroluminesce by applying a voltage across the device, *C. W. Tang et al., Appl Phys. Lett 51, 913 (1987).* Certain OLEDs have been shown to have sufficient brightness, range of color and operating lifetimes for use as a practical alternative technology to LCD-based full color flat-panel displays, *S.R. Forrest, P.E. Burrows and M.E. Thompson, Laser Focus World, Feb. 1995.* Since many of the thin organic films used in such devices are transparent in the visible spectral region, they allow for the realization of a completely new type of display pixel in which red (R), green (G), and blue (B) emitting OLEDs are placed in a vertically stacked geometry to provide a simple fabrication process, a small R-G-B pixel size, and a large fill factor, U.S. Patent No. 5,707,745. This patent disclosed a stacked OLED (SOLED) for which both intensity and color could be independently varied and controlled with external power supplies in a color tunable display device. Each layer in the integrated SOLED was independently addressable and emitted its own characteristic color. This colored emission could be transmitted through the adjacently stacked, transparent, independently addressable, organic layer or layers, the transparent contacts and the glass substrate, thus allowing the device to emit any color that could be produced by varying the relative output of the color-emitting layers. U.S. Patent No. 5,707,745, thus, illustrates a principle for achieving integrated, full color pixels that provide high image resolution, which is made possible by the compact pixel size. Furthermore, relatively low cost fabrication techniques, as compared with prior art methods, may be utilized for making such devices.

A transparent OLED (TOLED), *V. Bulovic. G. Gu, P.E. Burrows, M.E. Thompson, and S.R. Forrest, Nature 380, 29 (1996)*, which represents a further significant step toward realizing high resolution, independently addressable stacked R-G-B pixels, was reported in U.S. Patent No. 5,703.436, in which the TOLED had greater than 71% transparency when turned off and emitted light from both top and bottom device surfaces with high efficiency (approaching 1% quantum efficiency) when the device was turned on. The TOLED used transparent indium tin oxide (ITO) as the hole-injecting electrode and a Mg-Ag-ITO electrode layer for electron-injection. A device was disclosed in which the ITO side of the Mg-Ag-ITO electrode layer was used as a hole-injecting contact for a second, different color-emitting OLED stacked on top of the TOLED.

Such devices whose structure is based upon the use of layers of organic optoelectronic materials generally rely on a common mechanism leading to optical emission. Typically, this mechanism is based upon the radiative recombination of injected electrons and holes. Specifically, OLEDs are comprised of at least two thin organic layers separating the anode and cathode of the device. The material of one of these layers is specifically chosen based on the material's ability to assist in injecting and transporting holes, a "hole transporting layer" (HTL), and the material of the other layer is specifically selected according to its ability to assist in injecting and transporting electrons, an "electron transporting layer" (ETL). With such a construction, the device can be viewed as a diode with a forward bias when the potential applied to the anode is more positive than the potential applied to the cathode. Under these bias conditions, the anode injects holes (positive charge carriers) into the hole transporting layer, while the cathode injects electrons into the electron transporting layer. The portion of the luminescent medium adjacent to the anode thus forms a hole injecting and transporting zone while the portion of the luminescent medium adjacent to the cathode forms an electron injecting and transporting zone. The injected holes and electrons each migrate toward the oppositely charged electrode. When, for example, an electron and hole localize on the same molecule, a Frenkel exciton is formed. These excitons are trapped in the material which has the lowest energy. Recombination of this short-lived state may be visualized as an electron dropping from its conduction potential to a valence band, with relaxation occurring, under certain conditions, preferentially via a photoemissive mechanism. Under this view of the mechanism of operation of typical thin-layer organic devices, the electroluminescent layer comprises a luminescence zone receiving mobile charges carriers (electrons and holes)

from each electrode.

The materials that function as the electron transporting layer or as the hole transporting layer of the OLED are frequently the same materials that are incorporated into the OLED to produce the electroluminescent emission. Such devices in which the electron transporting layer or the hole transporting layer functions as the emissive layer are referred to as having a "single heterostructure" (SH). Alternatively, the electroluminescent material may be present in a separate emissive layer between the hole transporting layer and the electron transporting layer in what is referred to as a "double heterostructure" (DH).

In a single heterostructure OLED, either holes are injected from the HTL into the ETL where they combine with electrons to form excitons, or electrons are injected from the ETL into the HTL where they combine with holes to form excitons. Because excitons are trapped in the material having the lowest energy gap, and commonly used ETL materials generally have smaller energy gaps than commonly used HTL materials, the emissive layer of a single heterostructure device is typically the ETL. in such an OLED, the materials used for the ETL and HTL should be chosen such that holes can be injected efficiently from the HTL into the ETL. Also, the best OLEDs are believed to have good energy level alignment between the highest occupied molecular orbital (HOMO) levels of the HTL and ETL materials. In a double heterostructure OLED, holes are injected from the HTL and electrons are injected from the ETL into the separate emissive layer, where the holes and electrons combine to form excitons.

The material that is used as the cathode layer of an OLED has until now been comprised of a metal which has a low work function, for example, Mg:Ag. Such metallic cathode layers provide an electrically conductive path for current flow as well as a means of injecting electrons into the adjacent electron transporting layer. However, such metallic layers are also highly reflective and absorptive in the visible region of the spectrum.

This means that if a transparent OLED is desired, such as for stacked layers of a full-color SOLED or the single OLED of a monochromatic TOLED, a balance needs to be established between metallic layers that are thick enough to function as a cathode, but not so thick as to cause substantial light transmission or reflection losses. A conventional TOLED, therefore, uses 75-100 Å Mg:Ag capped with a thick layer of sputter-deposited ITO; the Mg:Ag layer serving both to inject electrons into $Alq_3$ and to protect it from the ITO sputtering. Thus, although a device with about 70% transmission may be obtained, there is still significant reflection from the compound cathode. In addition, in SOLED devices in which at least one of the color-producing layers is contained between the metallic cathodes of adjacent color-producing OLEDs, microcavity effects are present which can give rise to color tuning problems. *Z. Shen, P.E. Burrows, V. Bulovic, S.R. Forrest, and M.E. Thompson, Science **276**, 2009 (1997).* Such microcavity effects may also lead to an undesired angular dependence of the emitted light. Furthermore, thin Mg:Ag layers are sensitive to atmospheric degradation and, therefore, require special designs and processing steps to be undertaken so as to preserve their effectiveness in functioning as the cathode of an OLED.

Though solar cells have been disclosed in which a highly transparent ITO layer may have functioned as a cathode under certain circumstances, such ITO cathodes were disclosed as having been prepared by depositing the charge-carrying organic layer onto the ITO layer, *N. Karl*, *A. Bauer*, *J. Holzäofel*, *J. Marktanner*, *M. Möbus*, *and F. Stölzle*, *"Efficient Organic Photovoltaic Cells: The Role of Excitonic Light Collection, Exciton Diffusion to Interfaces*, *Internal Fields for Charge Separation*, *and High Charge Carrier Mobilities*", *Molecular Crystals and Liquid Crystals, Vol. 252, pp* 243-258, *1994* (*Karl et al) and Whitlock, J.B., Panayotatos, P., Sharma, G.D., Cox, M.D., Savers, R.R., and Bird*, *G.R.*; "*Investigations of Materials and Device Structures for Organic Semiconductor Solar Cells,* " *Optical Eng., Vol. 32, No. 8, 1921-1934 (August 1993)*, *(Whitlock et al).* ITO layers on which the organic layer had been deposited would not have been expected to form a low-resistance electrical contact with the adjacent organic layer and, thus, would not have been expected to function, as confirmed hereinafter for OLEDs, as an efficient cathode.

It would be desirable if optoelectronic devices could be made using cathodes that are as highly transparent as the highly transparent ITO anodes. It would be desirable, furthermore, that such highly transparent cathodes still have, for example, in OLEDs. electron-injection characteristics comparable to the thin, semi-transparent, low-work-function metallic layers, such as Mg:Ag, that are typically used as the cathode layer.

Various compounds have been used as HTL materials or ETL materials. HTL materials mostly consist of triaryl amines in various forms which show high hole mobilities ($10^{-3}$ $cm^2/Vs$). There is somewhat more variety in the ETLs used in OLEDs. Aluminum tris(8-hydroxyquinolate) ($Alq_3$) is the most common ETL material, and others include oxidiazol, triazol, and triazine.

For example, a typical single heterostructure device may be made of ITO/TPD/$Alq_3$/Mg-Ag. ITO serves as the anode, N,N'-diphenyl-N,N'-bis(3-methylphenyl)-1, 1'biphenyl-4,4'diamine (TPD) serves as the HTL. $Alq_3$ serves as the ETL, and Mg-Ag serves as the cathode. When a bias is applied across the device, holes are injected from ITO into TPD and migrate to the interface between the TPD and the $Alq_3$, and electrons are injected from the Mg-Ag alloy into $Alq_3$ and move to the same interface. Holes are injected from the TPD into the $Alq_3$, where they combine with electrons to form excitons. The excitons randomly diffuse through the $Alq_3$ layer until they recombine, preferentially via a photoemissive mechanism. The maximum distance of exciton migration in the $Alq_3$ layer of such a device is estimated to be around 300 Å in $Alq_3$.

Most emissive materials used in OLEDs have either low hole mobility or low electron mobility. As a result, exciton formation typically occurs very close to the interface where the charge carrier having the lower mobility is injected into the emissive layer. For example, most ETL materials have very poor hole conducting properties, such that the excitons will be preferentially formed very close to the HTUETL interface in a single heterostructure OLED having an emissive ETL. Because excitons are very short lived, they do not move very far before recombining. As a result, only a small volume of the ETL is used for exciton formation and recombinations. Using only a small volume of the emissive layer for exciton formation and recombination may lead to lower lifetime for the OLED. There is therefore a need for an emissive layer having a high electron mobility and a high hole mobility, such that exciton formation can occur in a reasonable volume of the layer. For example, there is a need for an ETL having a high hole mobility, so that exciton formation and light emission can occur in a reasonable volume of the ETL of a single heterostructure OLED having an emissive ETL.

It was first thought that mixing HTL and ETL materials together would decrease the spatial separation and increase the diffusion of holes into the ETL material. This idea was first demonstrated in a variety of polymer systems where both hole transporting and electron transporting moieties were blended into the polymer matrix. The most commonly used combination is the PVK/PBD system where PVK (polyvinylcarbazole) serves as hole transporter and PBD (2-(4-t-butylphenyl)-5-(4-phenyl-1-phenylene)oxidiazole) as the electron transporter. A number of different dyes have been doped into this system as emitting centers to generate colors from blue to red.

U.S. Patent No. 5,294,870 discloses a series of aluminum(III) complexes with two 8-hydroxyquinaldine ligands and a derivative of phenolate ligand, $Alq'_2(OAr)$. All of these species are blue emitters and are reasonable electron transporters. Devices have been fabricated with $Alq'_2(OAr)$ sandwiched between TPD and $Alq_3$. This configuration is necessary because of the poor electron injection from the Mg-Ag electrode. Nevertheless, these devices show blue-green emissions in their electroluminescent (EL) spectra and are nearly identical to their photo-luminescent (PL) spectra. These compounds have also shown good properties as host materials for doping with other fluorescent dyes, such as perylene to give blue emission. However, the ETL materials disclosed are poor hole transporters.

At the molecular level, a series of new emitting materials have been designed having an oxadiazole group as electron transporter and a triphenylamine as hole transporter. *Tamoto et al, Electroluminescence of 1,3,4-Oxadiazole and Triphenylamine-Containing Molecules* as *an Emitter in Organic Multilayer Light Emitting Diodes, Chem. Mater. 9, 1077-1085 (1997).* Layers formed of these emitting materials tend to form exciplexes with hole transporters with low ionization potentials. When exciplexes are not formed, high external quantum efficiency and energy conversion efficiency are observed However, devices consisting of these materials suffer from low luminescent lifetimes. It was also discovered that these emitting materials have more tendency to transfer electrons than holes.

In addition to emissive materials that are present as the predominant component in the electron transporting layer, and that function both as the electron transporting material as well as the emissive material, the emissive material may itself be present in relatively low concentrations as a dopant in the electron transporting layer.
Whenever a dopant is present, the predominant material in the electron transporting layer may be referred to as a host material. Materials that are present as host and dopant are selected so as to have a high level of energy transfer from the host to the dopant material. In addition, these materials need to be capable of producing acceptable electrical properties for the OLED. Furthermore, such host and dopant materials are preferably capable of being incorporated into the OLED using starting materials that can be readily incorporated into the OLED by using convenient fabrication techniques, in particular, by using vacuum-deposition techniques.

It is desirable for OLEDs to be fabricated using materials that provide electroluminescent emission in a relatively narrow band centered near selected spectral regions, which correspond to one of the three primary colors, red, green and blue so that they may be used as a colored layer in an OLED or SOLED. It would be desirable, in particular, to be able to select these compounds from a class of compounds in which the emission may be varied by selectively varying the substituents or by modifying the structure of a base compound that produces emission from a charge transfer transition. It is furthermore desirable that such compounds also be capable of being readily deposited as a thin layer using vacuum deposition techniques so that it may be readily incorporated into an OLED that is prepared entirely from vacuum-deposited organic materials.

A recent article disclosed a jelly fish (*Aequorea victoria*) which gives very narrow green fluorescence, *R. Heim, A.B. Cubitt, and R. Y. Tsien, Nature (1995) 373. 663-664.* The reported spectrum was centered at roughly 510 nm with a half width of 40 nm and the active chromophore responsible for this emission was shown to be:

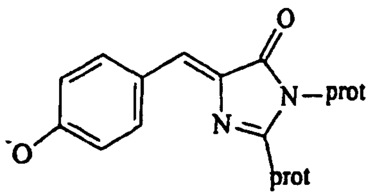

This *p*-hydroxybenzylidene-imidazolidinone chromophore is reported to be generated by cyclization and oxidation of the protein's own Ser-Tyr-Gly sequence and is bound to the protein in two places, labeled as "prot". Mutants of the protein have been reported which produce emission which is significantly blue shifted. It was proposed that the blue shift is due to changes in the protein matrix to which the dye is bound rather than to changes in the dye itself, *R. Heim, D.C. Prasher, and R.Y. Tsien, Proc. Nat. Acad. Sci. (1994) 91*, *12501-12504.* The fluorescence in these dyes is from a donor/acceptor network, involving a phenoxide ion donor and a carbonyl acceptor. The Heim publications describe use of the chromophores for labeling proteins with fluorescent tags to detect their localization or conformational changes both *in vitro* and in intact cells. However, these publications disclose nothing about preparation or use of the isolated chromophore molecule itself.

In the invention a class of azlactone-related compounds may be used as a dopant in the emissive layer of an OLED in which the emission of the dopant may be varied by selectively varying the substituents or by modifying the structure of the base compound that produces the emission. Such compounds are capable of being readily deposited as a thin layer using vacuum deposition techniques so that it may be readily incorporated into an OLED that is prepared entirely from vacuum-deposited organic materials. A review article summarizing azlactones discloses nothing about the fluorescent properties and nothing about a utility for these compounds, *Y.S. Rao and R. Filler, Synthesis (1975) 749-764.*

US 6 048 630 A is directed to OLEDs containing emitting compounds that produce a saturated red emission. The emission layer is comprised of an emitting compound having a chemical structure represented by Formula I:

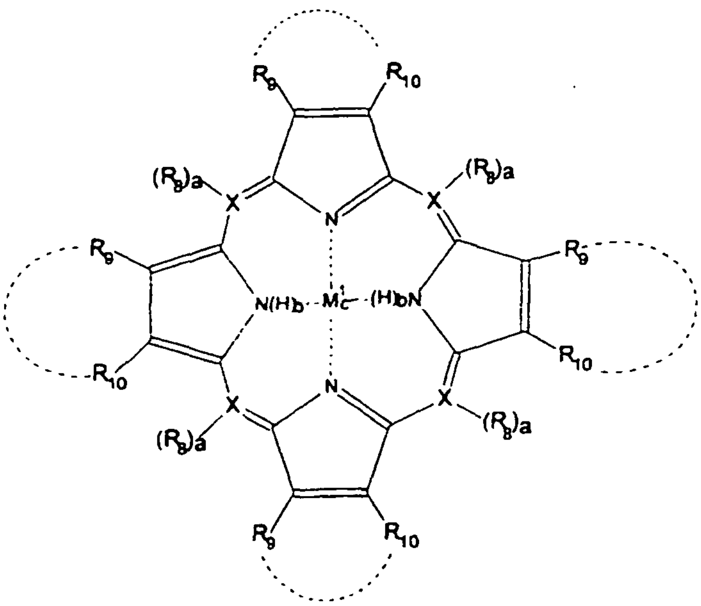

D-I

wherein X is C or N;

$R_8$, $R_9$ and $R_{10}$ are each independently selected from the group consisting of hydrogen, alkyl, substituted alkyl, aryl and substituted aryl; wherein $R_9$ and $R_{10}$ may be combined together to form a fused ring:

M, is a divalent, trivalent or tetravalent metal; and

a, b and c are each 0 or 1;

wherein, when X is C, then a is 1; when X is N, then a is 0;

when c is 1, then b is 0; and when b is 1. c is 0.

The examples disclosed in US 6 048 630 A included an emissive compound of formula I wherein X = C; $R_8$ = phenyl;

$R_9 = R_{10} = H$; c = 0; and b = 1. This compound has the chemical name 5,10,15,20-tetraphenyl-21H, 23H-porphine (TPP). OLEDs comprised of the TPP-containing emissive layer produce an emission spectrum comprised of two narrow bands that are centered at about 650 and about 713 nm, as shown in Fig. 1. The emission from this device involves fluorescence from the TPP dopant. One of the problems with the TPP-doped device is that the narrow band at 713 nm, which comprises about 40% of the emission, is not within a range that is useful for display applications. A second problem is that TPP-doped OLEDs are very unstable, such that the shelf life of such devices is typically very short. It would be desirable if these two aspects of TPP-doped devices could be improved.

Based on spin statistical arguments, it is generally understood that the majority of the excitons that are produced in an OLED are in a non-emissive triplet electronic state. Formation of such triplet states can result in a substantial loss of the excitation energy in the OLED via radiationless transitions to the ground state. It would be desirable if the total OLED quantum efficiency could be enhanced by utilizing this energy transfer pathway through the exciton triplet states, for example, by having the exciton triplet state energy transferred to an emissive material. Unfortunately, though it is known that the energy from an excited triplet state may be efficiently transferred under certain circumstances to the triplet state of a molecule that phosphoresces, the phosphorescent decay rate is typically not expected to be rapid enough to be adequate for use in a display device.

A well documented cause of OLED failure is thermally induced deformation of the organic layers (*e.g.* melting, crystal formation, thermal expansion, etc.). This failure mode can be seen in the studies that have been carried out with hole transporting materials, *K. Naito and A. Miura, J. Phys. Chem. (1993), 97, 6240-6248; S. Tokito, H. Tanaka, A. Okada and Y. Taga. Appl. Phys. Lett. (1996), 69, (7), 878-880; Y. Shirota*, *T Kobata and N, Noma, Chem. Lett. (1989), 1145-1148; T. Noda, I. Imae, N. Noma and Y. Shirota, Adv. Mater. (1997), 9, No. 3; E. Han, L. Do, M. Fujihira, H. Inada and Y. Shirota, J. Appl. Phys.* (1996), *80, (6) 3297-701; T. Noda, H. Ogawa, N. Noma and Y. Shirota. Appl. Phys. Lett. (1997). 70, (6). 699-701; S. Van Slyke, C. Chen and C. Tang, Appl. Phys. Lett. (1996), 69*, *15, 2160-2162; and U. S. Pat. No. 5,061,569.*

Organic materials that are present as a glass, as opposed to a crystalline or polycrystalline form, are desirable for use in the organic layers of an OLED, since glasses are capable of providing higher transparency as well as producing superior overall charge carrier characteristics as compared with the polycrystalline materials that are typically produced when thin films of the crystalline form of the materials are prepared. However, thermally induced deformation of the organic layers may lead to catastrophic and irreversible failure of the OLED if a glassy organic layer is heated above its $T_g$. In addition, thermally induced deformation of a glassy organic layer may occur at temperatures lower than $T_g$, and the rate of such deformation may be dependent on the difference between the temperature at which the deformation occurs and $T_g$. Consequently, the lifetime of an OLED may be dependent on the $T_g$ of the organic layers even if the device is not heated above $T_g$. As a result, there is a need for organic materials having a high $T_g$ that can be used in the organic layers of an OLED.

The most common hole transporting material used in the HTL of OLEDs is a biphenyl bridged diamine, N,N'-diphenyl-N,N'-bis(3-methylphenyl)-1,1-biphenyl-4,4'-diamine (TPD) having the chemical structure:

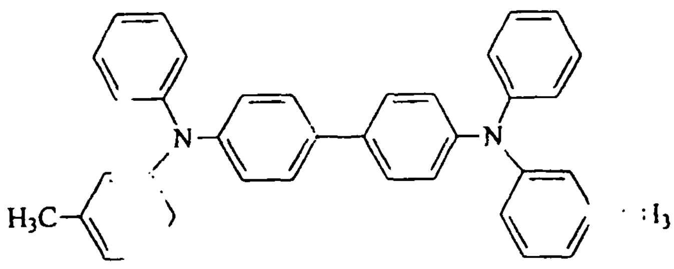

**TPD**

This material has a good hole mobility and efficiently transfers holes to aluminum tris (8-hydroxyquinoline) in a simple single heterostructure OLED. However, TPD has a melting point of 167°C and a glass transition temperature of 65°C. If a device prepared with TPD is heated above 65°C, the glass transition temperature, catastrophic and irreversible failure results. In order to increase the glass transition temperature of the HTL, several groups have explored different modifications to the basic structure of TPD, *Naito et al.; Tokito et al.; Shirota et al.; Noda et al. (Adv. Mater.);*

*Han et al.; Noda et al.(Appl. Phys. Lett.); Van Slyke et al.;* and U.S. Pat. No. 5,061,569. While these studies have led to materials with $T_g$ values as high as 150°C, they have not led to an understanding of why certain structural modifications increase $T_g$, while other modifications may not affect $T_g$ at all or may even lower $T_g$. Still other modifications may produce a material not having a glass transition temperature at all or a material not having the combination of properties that is suitable for use in an HTL. For example, replacing the amine groups of TPD with carbazole groups to produce 4,4'-di(N-carbazolo)diphenyl (CBP), having the chemical structure:

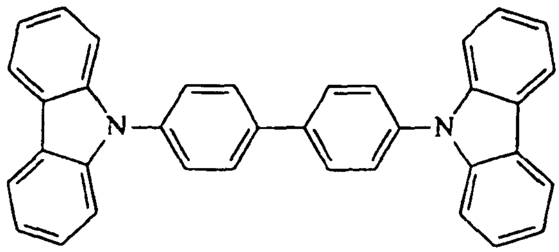

**CBP**

increases the melting point to 285°C. However, the material shows no glass transition. Further changes in the basic structure of TPD can increase the $T_g$ value even higher, but the materials often have poorer hole transporting properties than TPD, *i.e.* OLEDs made with these high temperature materials give poor device properties in OLEDs compared to TPD.

U.S. Patent No. 5,061,569 discloses hole transporting materials comprised of at least two tertiary amine moieties and further including an aromatic moiety containing at least two fused aromatic rings attached to the tertiary amine nitrogen atoms. Out of the large number of compounds encompassed by the broadly disclosed class of compounds recited, U.S. Patent No. 5,061,569 fails to disclose how to select those compounds which have a high glass transition temperature. For example, the naphthyl derivatives do make stable glasses. One such molecule is 4,4'-bis[N-(1-naphthyl)-N-phenyl-amino]biphenyl (α-NPD), having the chemical structure:

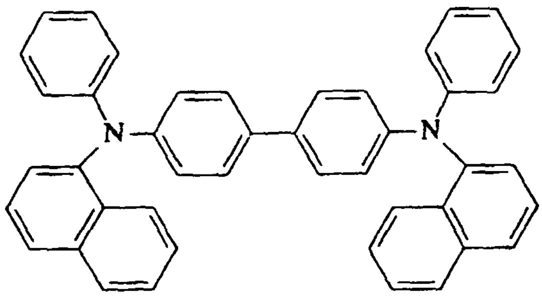

**α-NPD**

The present inventors' measurements show that α-NPD has a $T_g$ of 100-105°C. which is substantially higher than the $T_g$ of 65°C of TPD. This material has excellent hole conduction properties, and the $T_g$ of 100-105°C is higher than the $T_g$ of TPD of about 65°C. OLEDs prepared with NPD have electrical properties very similar to those prepared with TPD. However,

4,4'-bis[N-(2-naphthyl)-N-phenyl-amino]biphenyl (β-NPD), having the structure:

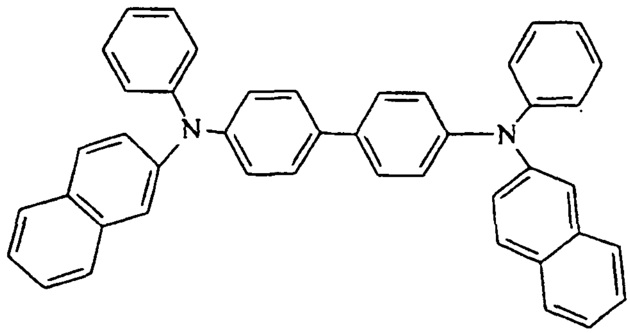

**β-NPD**

has been generally understood to have a $T_g$ which is substantially lower than α-derivative. Apparently because of this purportedly low and anomalous difference between $T_g$ of the α- β-derivatives, there had been no known reports of using the β-derivative as the hole transporting material of an OLED.

It would be desirable if OLED's could be fabricated from glassy charge carrier materials having improved temperature stability, while still providing luminescent characteristics comparable to prior art compounds. As used herein, the term "charge carrier layer" may refer to the hole transporting layer, the electron transporting layer or the separate emissive layer of an OLED having a double heterostructure. In addition, it would be useful to have a method for selecting and preparing such glassy charge carrier materials having improved temperature stability, as characterized in particular, by glassy charge carrier materials having a high glass transition temperature.

In addition, there is a general inverse correlation between the $T_g$ and the hole transporting properties of a material, i.e., materials having a high $T_g$ generally have poor hole transporting properties. Using an HTL with good hole transporting properties leads to an OLED having desirable properties such as higher quantum efficiency, lower resistance across the OLED, higher power quantum efficiency, and higher luminance. There is therefore a need for a HTL having a high hole mobility and a high glass transition temperature.

U.S. Patent No. 5,203,974 discloses a process for producing a thin film comprising electrotreating a dispersion or solution obtained by dispersing or dissolving a hydrophobic substance powder in an aqueous medium with a surfactant having a HLB value of 10.0 to 20.0, under conditions for forming a thin film of the hydrophobic substance on a cathode. The thin film of a hydrophobic substance can be formed on base metals such as aluminum, which can be applied to photosensitive materials and the like.

Bulovic et al in nature, volume 380, 7. March 1996 describes a transparent OLED, in which the electrone injecting contact was made by the depositing through a shadow mask of a thin layer of Mg-Ag alloy and finally depositing a second layer of indium tin oxide sputter deposited onto the Mg-Ag surface to provide a continuous transparent conducting surface.

## ADVANTAGES AND SUMMARY OF THE INVENTION

The present invention is directed to a method of fabricating a cathode comprised of an electrically conductive non-metallic layer in low-resistance contact with a semiconductive organic layer as defined in claim 1.

The present invention is directed toward a method of fabricating a highly transparent non-metallic cathode that may be used in substantially any type of optoelectronic device.

More specifically, the present invention is directed to a method of fabricating a highly transparent non-metallic cathode that may be used, for example, in OLEDs that have electron-injection properties comparable to semi-transparent metallic cathodes but which, instead, have an optical transmission of up to at least about 85% or still higher.

Still more specifically, the present invention is directed to a method of fabricating a highly transparent organic light emitting device (OLED) comprised of a non-metallic cathode as defined in claim 5.

Preferably, the OLED is comprised of a non-metallic cathode which may be in contact with an organic protection layer that is capable of assisting in the injection and transport of electrons from the cathode to the luminescent zone of

the OLED and that is, furthermore, capable of protecting the underlying organic layers from damage during deposition of the cathode layer. This organic protection layer may be in direct contact with the electron transporting layer in the luminescent zone of the device or there may be an additional electron transporting layer between these two layers which further assists in transporting electrons to the luminescent zone of the OLED.

Preferably, the organic light emitting device (OLED) comprises a heterostructure for producing electroluminescence, wherein the heterostructure may have a charge carrier layer that includes a compound having molecules having at least one electron transporting moiety and at least one hole transporting moiety wherein the electron transporting moiety is a 2-methyl-8-quinolinolato ligand coordinated with a Group III metal, such as Al, Ga, or In. The hole transporting moiety may be a hole transporting amine moiety. One example of such a hole transporting amine moity is a triarylamine derivatized phenoxide. The compound may therefore have, for example, two 2-methyl-8-quinolinolato ligands coordinated with A1 as electron transporting moieties and one triarylamine derivatized phenoxide as a hole transporting moiety.

The charge carrier layer that includes a compound having molecules having at least one electron transporting moiety and at least one hole transporting moiety may be an emissive layer, such as the ETL of a single heterostrucutre, or the separate emissive layer of a double heterostructure. This compound may also be used as an injection enhancement layer, such as an electron injection enhancement layer, which is disposed between the ETL and cathode of an OLED, and enhances the injection of electrons from the cathode into the ETL, or a hole injection enhancement layer, which is disposed between the HTL and anode of an OLED, and enhances the injection of holes from the anode into the HTL.

The ETL has a high hole mobility, so that holes can be transported away from the HTL/ETL interface of a single heterostructure to ultimately recombine with electrons throughout a large volume of the ETL. It is believed that this feature enhances the lifetime of OLEDs.

The heterostructure may be comprised of an emissive layer containing an azlactone-related compound.

The emission from the device may be obtained via a phosphorescent decay process wherein the phosphorescent decay rate is rapid enough to meet the requirements of a display device.

The OLED may also be comprised of a material that is capable of receiving the energy from an exciton singlet or triplet state and emitting that energy as phosphorescent radiation.

One of the benefits is that the phosphorescent decay process utilizes exciton triplet state energy that is typically wasted in an OLED via a radiationless energy transfer and relaxation process. The OLED may be comprised of materials capable of producing a highly saturated red emission. More specifically, OLEDs may also be comprised of platinum octaethylporphine (PtOEP), a compound that produces a narrow emission band that peaks near 640 nm when the PtOEP is doped in an electron transporting layer comprised of tris-(8-hydroxyquinoline)-aluminum ($Alq_3$). Such emission is perceived as highly saturated red emission.

Another of the benefits of PtOEP-doped OLEDs is that such OLEDs have a stability, when the device is exposed to ambient environmental conditions for a few days, that is comparable to prior art devices and, in particular, a decidedly greater shelf life stability as compared with TPP-doped devices.

Preferably, the phosphorescent dopant compound produces a highly saturated red emission in a spectral region for which the photopic response function for the human eye is significantly increased as compared with PtOEP-doped OLEDs.

Phosphorescent dopant compounds for use in an OLED can be selected, wherein the phosphorescent compound may be selected to be a platinum-porphine compound having reduced symmetry as compared with the 4-fold symmetry of compounds such as PtOEP, so as to obtain compounds having an emission peak shifted toward the peak of the eye sensitivity curve, while still remaining in a spectral region that is perceived as saturated red.

The organic light emitting devices may comprise a heterostructure for producing electroluminescence having a hole transporting layer with a glass structure. The hole transporting layer may be comprised of a compound having a symmetric molecular structure. The end groups of the symmetric molecule are hole transporting amine moieties having an unsaturated linkage between two arenes.

Further objectives and advantages of the present invention will be apparent to those skilled in the art from the detailed description of the disclosed invention.

## BRIEF DESCRIPTION OF THE DRAWINGS

Fig. A1 shows a schematic illustration of a standard prior art OLED.

Fig. A2a shows a representative OLED of the present invention.

Fig. A2b shows another representative OLED of the present invention.

Fig. A3 shows the light output vs. current of an OLED as shown in Fig. A2a having an ITO cathode layer and a CuPc electron injectin interface layer. The lowest set of values shown in this Fig. was obtained at 180 hours.

Fig. A4 shows the light output vs. current of a standard prior art TOLED device having an Mg:Ag cathode layer. The lower set of values in this Fig. was measured at 180 hours.

Fig. A5 shows the I-V curves for a ZnPc electron injecting interface layer and a CuPc electron injecting interface layer.

Fig. A6 shows the light output vs. current for a ZnPc electron injecting interface layer as compared with a CuPc electron injecting interface layer where the efficiency $\eta$ of the CuPc device was 0.23% and the ZnPc device was 0.15%.

Fig. A7 shows the transmission (T), reflection (R) and absorption (A), as a function of wavelength (nm), of an OLED having an ITO cathode and CuPc electron injecting interface layer.

Fig. A8 shows the I-V characteristics of a standard prior art OLED having a metallic Mg:Ag cathode layer with the higher set of values at 0 hours and the lower set of values at 180 hours.

Fig. A9 shows the I-V characteristics of an OLED having an ITO cathode and a CuPc electron injecting interface layer with the higher set of values at 0 hours and the lower set of values at 60 and 180 hours.

Fig. A10 shows the light output vs. current for devices having CuPc injection layer thicknesses from about 30 Å up to about 120 Å. These devices show a quantum efficiency $\eta$ of about 0.1%.

Fig. A11 shows the I-V characteristics of the devices of Fig. A10.

Fig. A12 shows the current-voltage characteristics of a 0.4 mm diameter non-metallic-cathode-containing TOLED ("MF-TOLED") and a reference TOLED grown in the same vacuum cycle.

Fig. A13 shows a schematic illustration of the non-metallic-cathode-containing TOLED structure.

Fig. A14 shows the summed optical output power from top and bottom surfaces vs. drive current of the non-metallic-cathode-containing TOLED ("MF-TOLED") and reference TOLED in Fig. A12. The luminance at the maximum current density measured corresponds to 2000 $cd/m^2$.

Fig. A15 shows the electroluminescence spectra from both the top and bottom device surfaces with a fit (solid line) to the top emission spectrum using the calculation method described herein.

Fig. A16 shows the proposed simplified energy level diagram of a non-metallic-cathode-containing TOLED. $D_{ss}$ indicates the density of surface states.

Fig. A17 shows digitally reproduced photographs that were taken of the non-metallic-cathode-containing TOLED and the conventional TOLED. The conventional TOLEDs show up as small gray areas whereas the the non-metallic-cathode-containing TOLEDs are not visible in the digitally reproduced photographs.

Fig. B1 shows absorption and fluorescent spectra of Al-pNP, Al-pCb, and Al-mCb in a $CH_2Cl_2$ solution.

Fig. B2 shows electroluminescent (EL) and photo-luminescent (PL) spectra of OLEDs having an Al-pNP layer.

Fig. B3 shows I-V characteristics of OLEDs having an Al-pNP layer.

Fig. B4 shows I-V characteristics of OLEDs having an Al-pCb layer.

Fig. B5 shows EL and PL spectra of OLEDs having an Al-pCb layer.

Fig. B6 shows EL and PL spectra of OLEDs having an Al-pCb/perylene layer.

Fig. B7 shows I-V characteristics of OLEDs having an Al-pCb/perylene layer.

Fig. C1 shows the electroluminescent spectra of a device doped with 0.8% of Compound 3 as dopant and an undoped $Alq_3$ device ("0%"), as compared with the photoluminescent spectrum of the dopant when present in $CH_2Cl_2$.

Fig. C2 shows the I-V characteristic of doped and undoped devices.

Fig. D1 shows the electroluminescent (EL) spectrum of TPP-doped OLEDs.

Fig. D2 shows the EL spectra as a function of wavelength at different voltages (6, 9. 12 and 15V) for OLEDs having an $Alq_3$ layer doped with 0.6 mol % PtOEP as compared with the EL spectrum of a TPP-doped device ("TPP EL").

Fig. D3 shows the EL spectra as a function of wavelength at different voltages for OLEDs doped with about 6 mol % PtOEP.

Fig. D4 shows the photoluminescent (PL) spectra as a function of wavelength for different PtOEP concentrations for $Alq_3$ devices doped with PtOEP.

Fig. D5 shows the PL spectra as a function of wavelength at different excitation wavelengths for a solution of PtOEP as compared with the EL spectrum of an OLED having an $Alq_3$ layer doped with 0.6 mol % PtOEP.

Fig. E1 shows a representative platinum porphyrin and four additional porphyrins that may be used to prepare platinum porphyrins for use as the platinum-substituted phosphorescent dopant compound in an OLED.

Fig. E2 shows the electroluminescent (EL) spectra of a PtDPP-doped polymer OLED as compared with the photoluminescent (PL) spectra of PtDDP in polystyrene and with the EL spectra of PtOEP doped in an $Alq_3$ device.

Fig. E3a shows the EL spectra as a function of applied voltage for PtDPP doped in the $Alq_3$ layer of an OLED comprised of (ITO/NPD/$Alq_3$/MgAg).

Fig. E3b shows the EL spectra of a PtDPP-doped $Alq_3$ OLED as compared with a PtOEP-doped $Alq_3$ OLED (with each device being operated at 9V).

Fig. E4 shows the CIE coordinates and luminance outputs from a PtDPP-doped OLED as compared with other red-emitting OLEDs.

Fig. E5 shows the overlap of the CIE standardized photopic response curve for human eye sensitivity with the normalized emission spectra for compounds that produce a saturated red emission.

Fig. F1 shows a plot of current v. voltage for an example outside of the present invention.

Fig. F2 shows a plot of current v. voltage for an example outside of the present invention having a CuPc hole injection enhancement layer.

## DETAILED DESCRIPTION OF THE PREFERRED EMBODIMENTS

The present invention will now be described in detail for specific preferred embodiments of the invention, it being understood that these embodiments are intended only as illustrative examples and the invention is not to be limited thereto.

The present invention is directed to the method of fabricating a cathode according to claim 1. Such cathodes may be employed in a wide range of electrical devices. In particular, since the cathodes of the present invention may be fabricated from highly transparent materials, such cathodes have particular benefit for use in organic optoelectronic devices such as OLEDs, solar cells, photodetectors, lasers and phototransistors. *S.R. Forrest, Chem. Rev.* ***97,*** *1793 (1997).* In an optoelectronic device having at least one electron transporting layer (ETL) comprised of an electron transporting material and at least one hole transporting layer (HTL) comprised of a hole transporting material, the cathode is identified as the electrode on the ETL side of the device and the anode is identified as the electrode on the HTL side of the device. In an OLED, for example, the cathode may be referred to as the electrode that injects electrons into the ETL and the anode as the electrode that injects holes into the HTL. Injecting holes into the HTL is equivalent to extracting electrons from the HTL.

Each electrode of an OLED may typically be present as a layer that is in direct contact with the adjacent HTL

or ETL, dependent on whether the electrode functions as an anode or cathode, respectively. Alternatively, it was disclosed in US 5 998 803 A that an additional organic layer may be included between the anode and the organic HTL. Such an additional layer, which was referred to as a protection layer or a hole-injection-enhancement layer, was disclosed to function as a protection layer for protecting the underlying organic layers during deposition of the electrode layer and/or as an enhancement layer for enhancing the hole-injection efficiency of the anode. For example, US 5 998 803 A disclosed that a protection layer, for example, of a phthalocyanine compound, such as zinc phthalocyanine (ZnPc) or copper phthalocyanine (CuPc), or PTCDA, could be deposited on top of the organic HTL so as to protect the organic layer during the subsequent sputter deposition of the ITO anode layer. US 5 998 803 A further disclosed that the protection layer could in some cases enhance the hole-injection efficiency of the hole-injecting anode.

Because of the combination of high electrical conductivity and low work function, which is provided by certain metals, practically useful optoelectronic devices such as OLEDs containing a cathode/organic-layer interface have until now been comprised of metallic cathode materials that inherently have a high reflectivity as well as modest absorption of optical radiation, even though the performance of such optoelectronic devices tends to be adversely affected by the high reflectivity of the metallic cathode layers. In optoelectronic devices such as OLEDs for which a high transparency is desired, semi-transparent metallic cathode layers are typically prepared using very thin metallic layers. Nevertheless, the metal's high reflectivity, which is inherently related to the high electrical conductivity of the metallic cathode materials, still causes significant losses in the overall performance of the device, for example, in producing a reduced quantum efficiency for the overall device.

One of the surprising aspects of the present invention is the fact that an electrically conductive non-metallic material has been to be found to be capable of forming a low-resistance electrical contact with an organic layer, wherein the electrically-conductive-non-metallic-layer/semiconductive-organic-layer interface, hereinafter "cathode/organic layer interface", is capable of efficiently injecting electrons form the non-metallic cathode layer, through the semiconductive organic layer of the cathode/organic layer interface, and then into the adjacent ETL of a practically useful organic optoelectronic device. The semiconductive organic layer of the cathode/organic layer interface is, therefore, alternatively referred to herein as the electron injecting interface layer.

For the purpose of specifying what is meant herein by a low-resistance electrical contact that may be used in a practically useful organic optoelectronic device, such a contact is one for which the voltage drop across the cathode/organic layer interface is not greater than the total voltage drop across the remainder of the device, that is, the voltage drop across the cathode/organic layer interface is less than about 50% of the total voltage drop across the overall device. Preferably, the voltage drop across the cathode/organic layer interface is less than about 30% of the total voltage drop.

The electrically conductive non-metallic layer may be selected from a wide range of non-metallic materials, wherein the term "non-metallic" is meant to embrace a wide range of materials provided that the material is free of metal in its chemically uncombined form. When a metal is present in its chemically uncombined form, either alone or in combination with one or more other metals as an alloy, the metal may alternatively be referred to as being present in its metallic form or as being a "free metal". Thus, the non-metallic cathodes of the present invention may sometimes be referred to by one or more of the inventors of the present invention as "metal-free cathodes" wherein the term "metal-free" is expressly meant to embrace a material free of metal in its chemically uncombined form. Such "non-metallic" or "metal-free" materials may also be referred to as "metal substitutes". As used herein, the term "metal substitute" refers to a material that is not a metal within the normal definition, but which has metal-like properties in appropriate contexts. Commonly used metal substitutes for electrodes would include wide band gap semiconductors, for example-., transparent conducting oxides such as indium tin oxide (ITO), tin oxide (TO) and gallium indium tin oxide (GITO). In particular, ITO is a highly doped degenerate n+ semiconductor with an optical band gap of approximately 3.2 eV rendering it transparent to wavelengths greater than approximately 320 nm. Another electrode material is the transparent conductive polymer polyanaline (PANI) and its chemical relatives.

It is to be understood that while the present invention relates to a non-metallic cathode material that is characterized as being free of a metal or metal alloy that is present in its metallic or chemically uncombined form, that is, free of free metal, the cathode material might include low levels of free metal in its metallic form while still falling within the scope of the present invention. The non-metallic cathode materials of the present invention may, thus, be characterized as embracing materials that are effectively free of free metal, wherein a material effectively free of free metal will correspondingly have a conductivity value which decreases as its temperature is lowered toward absolute zero. A concomitant property of non-metallic materials is that there is almost no practically detectable optical reflectivity due to the lack of partially occupied electronic conduction bands which are present in a free metal.

Another surprising aspect of the present invention is the fact that when representative embodiments of the claimed cathodes are used, for example, in an OLED, the cathode can efficiently inject electrons into the adjacent organic layer even though the non-metallic cathode material does not have the Fermi energy level that is typically provided by a low-work-function metal. Such efficient electron injection occurs in spite of the fact that a large barrier to electron injection would be expected at the cathode/organic layer interface between the electrically conductive non-metallic layer and the semiconductive organic layer. The present invention is thus, further directed to a method for effectively reducing

such barriers to electron injection.

More specifically, in the representative embodiment of the present invention as disclosed herein, the method of the present invention for producing a low-resistance electrical contact for electron flow across a cathode/organic-layer interface comprises providing a damage region at or near the surface of the semiconductive organic layer of the cathode/organic interface and, thus, between the non-metallic cathode material and the bulk of the semiconductive organic material. This damage region is prepared in such a manner that a high density of surface states or defect states for transport of electrons is produced. Though these surface states or defect states have not yet been detected directly, and may not in fact be readily observable directly, the presence of these surface states is believed to be necessary to account for the substantially reduced barrier to electron transport as evidenced indirectly by the low-resistance electrical contact across the cathode/organic layer interface.

In particular, it is believed that surface states are present at energy levels intermediate between the energy level of a conducting electron in the non-metallic cathode material and the energy level of a conducting electron in the bulk of the semiconductive organic layer of the cathode/organic interface. Furthermore, it is believed that the distribution of surface states is sufficiently dense in both energy and physical space that efficient electron transport can be effected across the cathode/organic layer interface region in spite of the large barrier to electron flow that would normally exist between a semiconductive organic material and a non-metallic material. Non-metallic cathodes having such an cathode/organic layer interface have been found, thus, to have a high efficiency, for example, for injecting electrons into the adjacent ETL of an OLED.

Furthermore, in the representative embodiment of the present invention in which ITO is used as the electrically conductive non-metallic layer and a phthalocyanine compound such as ZnPc or CuPc is used as the electron injecting interface layer, the low-resistance electrical contact is formed only when the ITO is deposited onto the organic layer and not when the organic layer is deposited onto the ITO layer. It is, thus, a further aspect of the present invention that when the preferred ITO and ZnPc or CuPc compounds are used to form the cathode/organic layer interface, the present invention is specifically directed to cathodes in which the non-metallic ITO layer is deposited onto the phthalocyanine layer using ITO deposition rates and phthalocyanine thicknesses that result in the low-resistance electrical contact. By properly controlling the ITO sputter deposition process so as to control the degree of damage caused at the surface, a cathode/organic layer interface can be produced having the desired low resistance. As described elsewhere herein, it is believed that the reduced barrier to electron transport, as evidenced by the low-resistance electrical contact, is provided by a high density of surface states at or near the surface of the organic layer.

It is believed that this feature of the present invention, that is, the ability to form a highly efficient electron-transporting cathode/organic layer interface, comparable to low-work-function metallic materials, but without the high reflectivity of metallic materials, is a uniquely advantageous combination of properties not possessed by the previously known cathode materials that were used in organic optoelectronic devices. A substantial reduction in the barrier to electron flow is specified herein as a reduction that results in the formation of a low-resistance electrical interface, as defined herein, between the electrically conductive non-metallic layer and the semiconductive organic layer of the cathode/organic interface. Thus, the present invention is directed to a method for fabricating a cathode comprising preparing an interface having an electrically conductive non-metallic material on one side of the interface and a semiconductive organic material on the opposite side of the interface, wherein the preparation step includes the step of forming any intermediate region between the electrically conductive non-metallic material and the semiconductive organic material such that the electrically conductive non-metallic material is capable of functioning as a cathode that forms a low-resistance electrical contact with the semiconductive organic material.

Use of electrically conductive non-metallic cathode materials that do not have the highly reflective properties inherent for metallic materials generally provides the specific benefit of being able to select highly transparent materials for use as the cathode in devices for which high optical transmission is desired, in particular, in optoelectronic devices such as OLEDs. One of the further features of the present invention is, thus, that optoelectronic devices such as OLEDs may be made using highly transparent non-metallic cathodes with electron-injection properties comparable to semi-transparent metallic cathodes. As compared with the thinnest practical semi-transparent metallic cathodes, which may typically provide a maximum transmission in the overall device of about 60-10%, devices having an optical transmission of at least about 85% may be prepared using the non-metallic cathodes of the present invention.

The electrically conductive non-metallic material that may be used to prepare the cathodes of the subject invention may be selected, for example, to be a transparent wide band gap semiconductor, for example, wide band gap semiconductor having a band gap of at least 1 eV and a transmission of at least 50% for incident and admitted radiation. Preferred wide band gap semiconductors include conductive oxides such as ITO, tin oxide, or gallium indium tin oxide (GITO).

The semiconductive organic materials that may be effectively used in combination with the ITO layer to produce the efficient electron injection preferably have the following properties:

1. A chemical and structural stability that is sufficient to permit only limited damage, as described hereinafter, due

to sputtering during deposition of the ITO layer. Large planar molecules such as phthalocyanines, naphthalocyanines and perylenes are representative examples. Derivatives of these compounds with further extended conjugation (e.g., additional fused benzo-, naphtha-, anthra-, phenanthrene-, polyacene, etc., groups) may also be used. Polymeric materials may also be present under certain circumstances.

2. An electron mobility that is sufficient to permit the layer to function as an electron transporting layer; an electron transporting material having a carrier mobility with a value of at least $10^{-6}$ $cm^2$/Vsec is generally believed to be sufficient for a material to function as an electron transporting layer, though substantially higher values are generally preferred; once again, large planar molecules such as the phthalocyanines and certain perylenes are representative examples.

3. The difference between the ionization potential (IP) and the HOMO/LUMO gap energy (the energy gap between the highest occupied molecular orbital and the lowest unoccupied molecular orbital), that is, the "IP-HOMO/LUMO gap energy", of the material used in the electron injecting interface layer is such that it is approximately equal to or preferably less than the IP-HOMO/LUMO gap energy of the film into which electrons are being injected. This guideline is not intended as a constraint that is to be strictly obeyed, but is instead intended to be approximately followed. For example, small deviations from this guideline of about 0.5 eV may be tolerated for certain combinations of materials. Use of this guideline helps to prevent formation of an energy barrier to electron flow into the contacted film (e.g. $Alq_3$).

Still more specifically, the subject method may be comprised of using ITO as the electrically conductive non-metallic layer and a phthalocyanine such as CuPc or ZnPc as the electron injecting interface layer. In this case, the electrically conductive non-metallic ITO layer is sputter deposited onto an organic protection layer comprised of the electron injecting interface layer of CuPc or ZnPc. So as to control the level of damage, the ITO is sputtered onto the organic layer at relatively low initial deposition rates of about 1 to about 10 Å/minute until a thickness of about 100 Å to about 300 Å has been deposited. As described in further detail hereinafter, such an electrically-conductive-non-metallic-layer/semiconductive-organic-layer interface is capable of providing a low-resistance electrical contact for use in an OLED as well as for other types of optoelectronic devices.

The present invention is, thus, more specifically directed to a new class of highly transparent organic light emitting devices (OLEDs) employing a non-metallic cathode. OLEDs that make use of a non-metallic cathode have a very low reflectivity and a high transparency that is close to the theoretical maximum that can be achieved for a multi-layer organic structure. The low-reflectivity of such OLEDs may be particularly beneficial for use in high contrast display applications as well as for use in eliminating microcavity effects in stacked organic light emitting devices (SOLEDs). OLEDs employing these low resistance non-metallic cathodes are expected to be particularly useful in reliable high-resolution full color flat panel displays, "heads-up" displays and organic-based lasers.

As a representative embodiment of the present invention as shown in Fig. A2a, a TOLED is deposited on a glass substrate pre-coated with a film of indium tin oxide (ITO) which serves as the transparent hole injecting anode. The TOLED includes, for example, a non-metallic cathode **1,** an electron injecting interface layer **6,** an electron transporting layer **2,** a hole transporting layer **3,** an anode layer **4** and a substrate **5.** After depositing a hole transporting layer and an electron transporting layer, the electron injecting interface layer is added by depositing, for example, a thin film of copper phthalocyanine (CuPc) which is then capped with a film of low-power, radio-frequency sputtered ITO. This second ITO layer functions as the cathode of this device. In addition to functioning, in some cases, as a protection layer that prevents damage to the underlying organic layers during the ITO sputtering process, the CuPc layer also functions in combination with the ITO layer as the electron injecting region for delivering electrons to the adjacent electron transporting layer. An additional intermediate electron transporting layer of 4,4'-di(N-carbazolo)diphenyl (CBP), for example, may be present between the first electron transporting layer and the CuPc layer, as shown in Fig. A2b. The intermediate electron transporting layer lies between the electron injecting interface layer that is in low-resistance electrical contact with the non-metallic cathode layer and the electron transporting layer that is in contact with the hole transporting layer. In particular, the OLED of Fig. A2b includes a non-metallic layer **1,** an electron injecting interface layer **6,** an intermediate electron transporting layer **7**, an electron transporting layer **2**, a hole transporting layer **3,** an anode layer **4** and a substrate **5.**

Due to the absence of a metallic cathode layer, the representative $Alq_3$-based TOLEDs disclosed herein emit nearly identical light levels in the forward and back scattered directions with a total external quantum efficiency of about 0.3%. These devices are over 80% transmissive in the visible. The reflection and absorption characteristics, current-voltage, luminance-current, and electroluminescence spectra of OLEDs prepared according to the present invention demonstrate performance characteristics that are at least comparable with and in certain respects superior to conventional TOLEDs that employ a more reflective cathode comprised of a thin film of Mg:Ag capped with ITO.

For example, as shown by a comparison of the TOLED results shown in Fig. A3 with Fig. A4, TOLEDs according to the present invention show only about a 2-fold drop in light output at 180 hours, whereas prior art TOLEDs have about a 4-fold drop in light output over the same time interval. The results in Fig. A5 show that the phthalocyanines of both Cu (CuPc) and Zn (ZnPc) may be used as the electron injecting interface layer, though the results in Fig. A6 show that the

CuPc device has a significantly higher quantum efficiency. A comparison of the results in Fig. A8 with the results in Fig. A9 shows that the stability of the I-V characteristics of OLEDs made according to the present invention is comparable to prior art devices. The results shown in Fig. A7 show that the total light transmission of an OLED made according to the present invention is near the theoretical maximum of what can be achieved for an OLED, except for that part of the spectrum which shows the Q-band absorption structure characteristic of CuPc. The reflection spectrum of this device approaches the theoretical minimum as limited by the glass/air and ITO/air interfaces. Anti-reflection layers can further reduce this reflection to a negligible value.

The electron injecting interface layer that is in contact with the ITO layer may have a thickness ranging from about 15-120 Å. For example, Figs. A10 and A11 show that whenever CuPc is used as the electron injecting interface layer, devices having a CuPc injection layer thickness from about 30 Å to about 120 Å produced comparable performance characteristics. The devices that were prepared to collect the data shown in Figs. A10 and A11 also included a CuPc layer with a 50 Å thickness between the ITO anode layer and the hole transporting layer. This CuPc layer, which is in contact with the ITO anode layer, functions as a hole injection enhancement layer, such as disclosed in US 5 998 803 A.

As further examples for representing the scope of the present invention, the current-voltage (I-V) characteristics of another typical transparent organic light emitting device having the non-metallic cathode and a conventional TOLED grown in the same run are shown in Fig. A12. The TOLED that was used to obtain the results shown in Fig. A12 is shown in Fig. A 13, wherein the non-metallic cathode 1 was ITO, the electron injecting interface layer **6** was CuPc, the electron transporting layer **2** was $Alq_3$, the hole transporting layer **3** was $\alpha$-NPD, the anode layer **4** was ITO and the substrate **5.** This device included an additional CuPc layer **8** between the ITO anode and HTL. Two distinct regions of operation are observed, above and below the "turn-on voltage", $V_T$. Below $V_T$, trap-free, space-charge limited transport follows $I \propto V^{m-1}$ . Above $V_T$, the current is trapped-charge-limited following $| \propto V^{m-1}$. *P.E. Burrows., S.R. Forrest, Appl. Phys. Lett.* ***64,*** *2285 (1994)*, *P.E. Burrows, Z. Shen, V. Bulovic, D.M. McCarty, S.R. Forrest, J.A. Cronin, and M.E. Thompson, J. Appl. Phys.* ***79***, *7991 (1996)*. The slightly less efficient injection properties of the ITO/CuPc as compared to those of the ITO/Mg:Ag contact are reflected in the nominal difference in $V_T$ for the TOLED (4.2 V) and non-metallic-cathode-containing TOLED (5.2 V). Similar I-V characteristics are obtained when the CuPc in the cathode is replaced with ZnPc, indicating their equivalence in forming good electron injecting contacts. However, when CuPc is replaced with PTCDA, there is a significant increase in $V_T$ to 20V.

The sum of the output optical power emitted from both top and bottom device surfaces as a function of the current is shown in Fig. A14 for the same devices as in Fig. A12. The total external quantum efficiencies of the devices are similar at $\eta = (0.38 \pm 0.05)\%$. Their luminance at 10 mA/cm$^2$ is -200 cd/m$^2$, increasing to 2000 cd/m$^2$ at 10 mA/cm$^2$, corresponding to the maximum drive current indicated in Fig. A14. The ratio of the power emitted from the top to that emitted from the bottom substrate surface of the non-metallic-cathode-containing TOLED is $r = 1.0 \pm 0.05$.

Due to the relatively large difference between the complex index of refraction of CuPc and the other organic materials in the non-metallic-cathode-containing TOLED, there is some broadening of the output electroluminescence spectrum measured from the top, relative to the bottom device surface, as shown in Fig. A15. Using the known complex indices of refraction for each layer in the device, and assuming that the radiative, dipolar cm6 molecules are uniformly distributed in the emissive layer, r was calculated as a function of wavelength ($\lambda$). A fit to the top surface.spectrum was obtained by multiplying the emission from the substrate by r($\lambda$). The fit closely matches the normalized, measured output spectra (Fig. A14), with the remaining discrepancies between calculation and experiment due to the neglect of dispersion in the CuPc.

While the I-V characteristics of devices employing CuPc or ZnPc cathodes were similar, $\eta$ of the ZnPc device was 30% lower than that of the CuPc device. In sharp contrast to the CuPc and the ZnPc cathodes, however, the devices with PTCDA in the cathode had quantum efficiencies that were only about 1% that of the CuPc devices. This indicated very poor electron injection into the ETL employing PTCDA cathodes, consistent with the I-V data.

The elimination of semi-transparent metal films from the TOLED results in a significant increase in the total optical transmission. This is readily apparent from the digitally reproduced photographs that were taken of the non-metallic-cathode-containing TOLED arrays and the conventional TOLED arrays, as shown in Fig. A17 The arrays were placed on a white background having a grid of black dots for contrast. The devices were illuminated from underneath and are indicated by the arrows at about 0.2-0.4 cm for the conventional TOLED arrays and at about 1.3-1.6 cm for the non-metallic-cathode-containing TOLED arrays, respectively. The photographs in Fig. A17 show the non-metallic cathodes of the present invention are natureless due to the absence of any metal in the electrode whereas conventional TOLEDS have a faint grey appearance due to the metallic cathodes. The non-metallic-cathode-containing TOLED arrays could be detected only if the digitally Measurements of the non-antireflection-coated device optical transparency shown in Fig. A7 indicate a transmission of $0.85 \pm 0.05$, which corresponds to a 35% increase over the conventional OLED. The reflection and absorption of the non-metallic-cathode-containing TOLED are also plotted in Fig. A 7, where the primary source of absorption is due to the CuPc Q bands, *B.H. Schechtman and W.E. Spicer, J. of Mol. Spec.* ***33***, *28 (1970),* which peak at $\lambda$ = 620 nm and 665 nm.

Without being limited to the theory of how the present invention works, it is believed that during the initial stages

of ITO deposition, damage-induced states are produced in what is herein referred to as a damage layer at the cathode/organic film interface. In contrast to the prior art understanding of high efficiency electron injecting electrodes, *C.W. Tang and S.A. VanSlyke, Appl. Phys. Lett.* ***51****, 913 (1987),* which required energy band alignment of the lowest unoccupied molecular orbital (LUMO) of the ETL with the Fermi energy of a low work function metal, it is believed herein that the damage layer is responsible for providing improved electron-injection properties for non-metallic cathodes comprised of materials that do not have the matching Fermi energy level that is typically provided by a low-work-function metal.

The improved electron-injection properties of the highly transparent non-metallic cathodes may be understood in terms of the proposed energy band diagram in Fig. A16. The ionization potentials (IP), which are defined as the vacuum lever to HOMO distance, and optical energy gaps ($E_g$) are taken from *A. Rajagopal, C.I. Wu, and A. Kahn,* 1997 *Fall Mtg. Of Mat. Res. Soc.*, *paper J1.9; and K. Seki, Mol. Cryst. Liq. Cryst. 171, 255 (1989)*. Since the ionization potential (IP) of CuPc lies between the work function of ITO and the IP of α-NPD, CuPc lowers the barrier to hole injection into the HTL. In contrast, there is a large barrier (1.6 eV) to electron injection at the ITO/CuPc interface, in spite of the fact that this electrode is disclosed herein to efficiently inject electrons whenever this electrode is prepared in accord with the present invention. This apparent contradiction is consistent with previous reports, *S. R. Forrest. L.Y. Leu, F.F. So, and W.Y. Yoon. J. 4ppl. Phys.* ***66****, 5908 (1989)*, of efficient hole injection using anodes consisting of PTCDA capped with ITO. In this case, hole injection is achieved although the barrier from ITO into PTCDA is 2.1 eV. Values for the ionization energies (defined as the vacuum level to HOMO distance) are from *A. Rajagopal, C.I. Wu, and A. Kahn*, *J. Appl. Phys. 83, 2649 (1998) and K. Seki, Mol. Cryst. Liq. Cryst. 171, 255 (1989).* While these authors suggest that the vacuum level may not be flat between organic heterojunctions, as shown in Fig. A4, this does not change our conclusions and hence has been omitted for simplicity.

Efficient electron injection in the presence of a large energy barrier suggests that the barrier is effectively reduced due to the electrode deposition/formation process. When the ITO is sputtered onto the CuPc surface, the Cu can form a Cu-O bond via an exothermic reaction, *F.F. So and S.R. Forrest, J. Appl. Phys. 63*, *442* (*1988*), thereby inducing a high density of midgap or surface states, $D_{ss}$, as shown in Fig. A16. These states, whose density decreases away from the ITO interface, provide small energy "steps" which can easily be surmounted by the injected electron. These data and the resulting model are in contrast to previous suggestions that low work function metals are required for efficient electron injection. The residual energy barrier may account, in part, for the small increase in $V_T$ for the non-metallic-cathode-containing TOLED as compared with TOLEDs not having the non-metallic cathodes of the present invention.

Evidence that such damage is effective to produce the non-metallic cathode only when the damage is restricted to the CuPc layer was obtained by reducing the CuPc layer thickness from 60 Å to 30 Å. This reduction in thickness which resulted in a concomitant reduction in the non-metallic-cathode-containing TOLED fabrication yield from about 90% to 40%. The fabrication yield is defined as the ratio of the non-shorted, efficient devices to the total number of devices fabricated and tested. Such yields were based on test populations of 10 to 20 test devices. These results suggests that only the first few monolayers of the CuPc are damaged in low resistance cathode/organic layer interfaces, as was shown in the case of ITO/PTCDA. It is thought that when the CuPc is made too thin, the sputtered ITO can "punch through" to damage the underlying $Alq_3$. Indeed, ITO sputtered directly onto either α-NPD or $Alq_3$, wherein the ITO layer would function as an anode or a cathode, respectively, results in a yield approaching zero.

It is believed herein that the limited damage to CuPc and PTCDA may be due to the extended conjugated electron orbitals in these large planar molecules. When an energetic metal or oxygen atom is incident on one of these surface molecules during sputtering, the energy of impact is efficiently distributed over the numerous bonds in the molecular π-electron systems. In contrast, no comparably large n-electron systems exist in $Alq_3$ or α-NPD. For such molecules, the impact energy is more localized among only a few atomic sites, thereby increasing the probability for breaking a molecular bond. The planar or nearly planar stacking arrangements of crystalline molecular systems such as CuPc and PTCDA may also assist in the dissipation of energy among several neighboring molecules in the lattice.

This hypothesis, based on the role played by energetic atom-induced defects in assisting carrier injection, was tested by fabricating a transparent non-metallic-cathode-containing TOLED such as that shown in Fig. A12, except with the $Alq_3$ and α-NPD layer order reversed. For these devices having the ETL and HTL layers reversed, $V_T$ was observed to increase by a factor of two (-10V) and an external quantum efficiency η of about $10^{-3}$% was measured even though the ITO/CuPc interface is known to be excellent for injecting holes into α-NPD. The low efficiency of this device, therefore, provides clear evidence that an CuPc/ITO interface not having the damage layer is ineffective in injecting electrons into $Alq_3$. It is, therefore, concluded that the low energy deposition of CuPc *on top of* ITO does not generate the interfacial mid-gap states required for electron injection. Thus, since the ITO layers prepared by *Karl et al* and *Whitlock et al* were also produced by deposition of the organic layer onto the ITO layer, rather than by deposition of the ITO layer onto the organic layer so as to produce a damage layer, the ITO layers of *Karl et al* and *Whitlock et al* would be expected also not to produce the enhanced electron-injection characteristics of the present invention, whenever the electrodes of *Karl et al* or *Whitlock et al* functioned as the cathode of a solar cell.

The asymmetry in the device characteristics is also consistent with the very low current and lack of electroluminescence when the non-metallic-cathode-containing TOLED is reverse biased. In this case, the electrons are unable to traverse the 1.6 eV energy barrier from the ITO into the undamaged CuPc at the anode. Once electrons are injected into the CuPc, they are transported into the $Alq_3$ due to the lack of an energy barrier to electron transport in that direction. In contrast, a large energy barrier to electrons of 0.9 eV exists from PTCDA to $Alq_3$. This barrier, coupled with the poor electron mobility of PTCDA, results in the observed high $V_T$ and low η of $Alq_3$ -based devices employing ITO cathodes in contact with PTCDA. This low efficiency does not, however, suggest that PTCDA cannot be used to make a good low resistance cathode/organic interface. In particular, these results only show that a barrier of 0.9 eV is too high between the PTCDA and the $Alq_3$. By properly matching the PTCDA with an appropriate adjacent charge-carrying layer, in accord with the guidelines provided herein for aligning the LUMO/HOMO levels, an efficient ITO/PTCDA interface may be made.

As an example of an OLED, the non-metallic cathode may be used in a double heterostructure wherein a thin luminescent layer is present between the electron transporting layer and the hole transporting layer.

As another example of an OLED in which an electron transporting layer is the emissive layer, the ITO layer may be in contact with the electron injecting interface layer that produces the electroluminescence and that, in addition, is in direct contact with a hole transporting layer. In this case, the IP-HOMO/LUMO gap energy of the material used in the electron injecting interface layer is such that it is approximately equal to or preferably less than the IP-HOMO/LUMO gap energy of the material in the adjacent hole transporting layer and, in addition, the ionization potential of the material in the hole transporting layer is greater than the ionization potential of the material used in the electron injecting interface layer.

As an example in which the hole transporting layer is the emissive layer, the ITO layer may be in contact with an electron injecting interface layer that is in direct contact with a hole transporting layer that produces the electroluminescence. In this case, the IP-HOMO/LUMO gap energy of the material used in the electron injecting interface layer is also such that it is approximately equal to or preferably less than the IP-HOMO/LUMO gap energy of the material in the adjacent hole transporting layer. However, in this case, the ionization potential of the material in the hole transporting layer is less than the ionization potential of the material used in the electron injecting interface layer.

Thus, while the present invention is demonstrated for a single heterostructure in which the ITO layer is in contact with an electron injecting interface layer that is in contact with an electron transporting layer such as $Alq_3$, the present invention is directed, *inter alia,* toward any OLED comprised of a heterostructure for producing electroluminescence wherein the heterostructure includes a non-metallic cathode.

In particular, the OLEDs are comprised of a heterostructure for producing electroluminescence which may be fabricated as a single heterostructure or as a double heterostructure. The materials, methods and apparatus for preparing the organic thin films of a single or double heterostructure are disclosed, for example in U.S. Patent No. 5,554,220. As used herein, the term "heterostructure for producing electroluminescence" refers to a heterostructure that includes, for a single heterostructure, in sequence, a hole injecting anode layer, a hole transporting layer, an electron transporting layer, and a cathode layer. An additional layer or layers may be present between one or more of the sequential pairs of these layers. For example, for a double heterostructure, a separate emissive layer is included between the hole transporting layer and the electron transporting layer. This separate emissive layer may be characterized as being a "thins luminescent layer".
Alternatively, or in addition, a hole injection enhancement layer may be present between the anode layer and the hole transporting layer.

The hole injecting enhancement layer may in some cases be comprised of the same material, CuPc, as is used in the electron injecting interface layer. In each case, the CuPc layer may be in direct contact with an ITO electrode, with the distinction between the two CuPc layers being that in one case the CuPc layer is in contact with an ITO layer that functions as an anode and in the other case the ITO layer functions as a cathode. In each case, the CuPc layer functions as a charge carrier and interface layer. On the one hand when in contact with the ITO anode, the CuPc layer assists in injecting and transporting holes from the anode to a hole transporting layer, and on the other hand when in contact with the ITO cathode, the CuPc layer assists in injecting and transporting electrons from the cathode to an electron transporting layer. The CuPc layer, in each case, may also function as a layer that protects any underlying organic layers, if present, from damage during the ITO deposition process. Whenever the ITO layer is present as the electrode in a SOLED structure, opposite faces of the ITO may function as an anode and cathode, respectively.

Either the anode layer or the cathode layer may be in contact with a substrate and each electrode is connected to electrical contacts which are capable of delivering a voltage across the device causing it to produce electroluminescence from either an electron transporting layer or a hole transporting layer. If the cathode layer is deposited on the substrate, the device may be referred to as having an inverted or IOLED structure. If the heterostructure for producing electroluminescence is included as part of a stacked OLED (SOLED), one or both of the electrodes of an individual heterostucture may be in contact with an electrode of an adjacent heterostructure. Alternatively, dependent on the circuitry used to drive the SOLED, an insulating layer may be provided between adjacent electrodes of two of the OLEDs in the stack.

While the OLEDs comprise non-metallic cathode layers rather than metallic cathode layers, the OLEDs may, under certain circumstances, be used in combination with an OLED that does contain a metallic layer, for example, as

the top or bottom OLED of a SOLED.

In such cases, if the cathode layer is a metal cathode layer of Mg:Ag, a metal protective layer, for example, made of a layer of Ag for protecting the Mg:Ag cathode layer from atmospheric oxidation, may also be present.

The single or double heterostructures as referred to herein are intended solely as examples for showing how an OLED may be fabricated without in any way intending the invention to be limited to the particular materials or sequence for making the layers shown. For example, the heterostructure typically includes a substrate which may be opaque or transparent, rigid or flexible, and/or plastic, metal or glass, in particular, a transparent polymer such as polyester, glass, sapphire or quartz, or substantially any other material that may be used as the substrate of an OLED.

The organic light emitting device (OLED) may comprise a heterostructure for producing electroluminescence wherein, in particular, the heterostructure has a charge carrier layer that includes a compound having molecules having at least one electron transporting moiety and at least one hole transporting moiety wherein the electron transporting moiety is a 2-methyl-8-quinolinolato ligand coordinatd with a Group III metal, such as Al, Ga, or In. The hole transporting moiety may be a hole transporting amine moiety.

As used herein, the term "charge carrier layer" may refer to a "hole transporting layer" (HTL), an "electron transporting layer" (ETL) or, for an OLED having a double heterostructure (DH), a "separate emissive layer". The charge carrier layer that includes a compound having molecules having at least one electron transporting moiety and at least one hole transporting moiety may be an emissive layer, such as the ETL of a single heterostructure (SH), or the separate emissive layer of a double heterostructure. The charge carrier layer that includes a compound having molecules having at least one electron transporting moiety and at least one hole transporting moiety may also be a non-emissive layer, such as an electron injection enhancement layer disposed between the cathode and ETL of an OLED.

The term "hole transporting moiety" as used herein, refers to a group which, when present in a material contained in a layer of an OLED, causes the material to provide electrical conduction through the layer, when a voltage is applied, by conduction of holes. The term "electron transporting moiety" as used herein, refers to a group which, when present in a material contained in a layer of an OLED, causes the material to provide electrical conduction through the layer, when a voltage is applied, by conduction of electrons. The term "hole transporting amine moiety" as used herein, refers to an amine group that is a hole transporting moiety. Such hole transporting amine moieties are typically comprised of nitrogen atoms that are directly bonded to two phenyl groups (in addition to a phenyl group that forms the ligand bond with the Group III metal), wherein the two phenyl groups may be joined so as to form a heterocyclic ring including the nitrogen, for example, a carbazole group, or the two phenyl groups may be unattached to each other. Each phenyl group may itself be fused with still another phenyl group, being bonded to the nitrogen atom, for example, either as a 1-naphthyl group or as a 2-naphthyl group.

A compound having the chemical structure may be used:

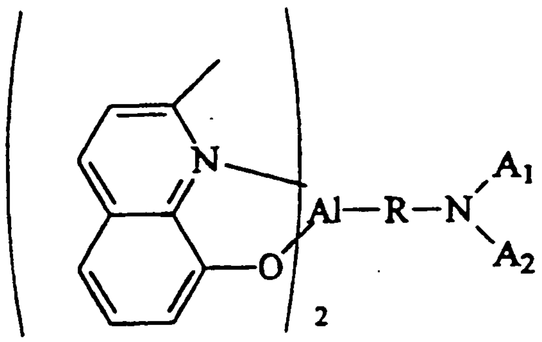

wherein $A_1$ and $A_2$ are each comprised of one or more phenyl groups that produce an overall hole transporting functionality, and -R- is an alkyl or aryl group, preferably an aryl group, that is capable of forming a ligand bond with a Group III metal. The phenyl groups of $A_1$ and $A_2$ may be joined so as to form a heterocyclic ring including the nitrogen, e.g., so as to constitute a carbazole group, or the phenyl groups of $A_1$ and $A_2$ may be unattached to each other. Each phenyl group may itself be fused with still another phenyl group and bonded to the nitrogen atom either, for example, as a 1-naphthyl group or as a 2-naphthyl group. The -R- group may be a hole transporting substituting alkoxide, thiolate, benzoic acid or chelating oxygen or nitrogen group (such as substituted acetylacetonate). Preferably, the R-group is a p-amino-substituted phenoxide group. Another Group III metal, such as Ga or In, may be substituted for the Al.

The 2-methyl-8-quinolinolato ligand coordinated with a Group III metal, such as Al, acts as an electron transporting moiety. The amine group acts as a hole transporting moiety. Because the molecule has both electron and hole transporting moieties, a charge carrier layer made from the molecule will have a high electron mobility and a high hole mobility. Using such a charge carrier layer as the ETL of a single heterostructure, for example, allows holes to move a

substantial distance into the ETL before recombining with an electron to form an exciton. As a result, the luminescent region of such an ETL is larger than the luminescent region of an ETL having a low hole mobility. This larger electroluminescent region may result in a longer electroluminescent lifetime. Similarly, using such a charge carrier layer as a separate emissive layer of a double heterostructure leads to a luminescent region larger than that of a separate emissive layer having a lower hole or electron mobility, and a longer electroluminescent lifetime.

In particular, the compound having molecules having at least one electron transporting moiety and at least one hole transporting moiety may be bis(2-methyl-8-quinolinolato)[*p*-(N-phenyl-2-naphthylamino)phenolato)aluminum(III), which is referred to as Al-pNP, bis(2-methyl-8-quinolinolato)(*p*-carbazolphenolato)aluminum(III), which is referred to as Al-pCb, or bis(2-methyl-8-quinolinolato)(*m*-carbazolphenolato)aluminum(III), which is referred to as Al-mCb:

Al-pNP

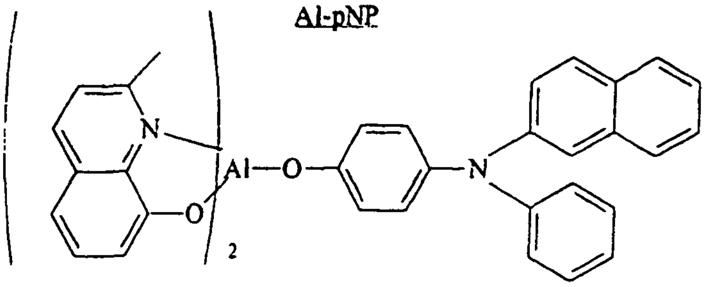

Al-pCb

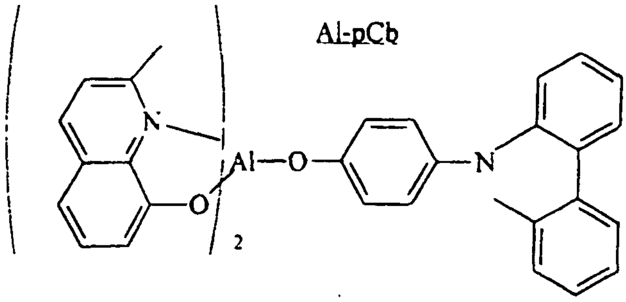

Al-mCb

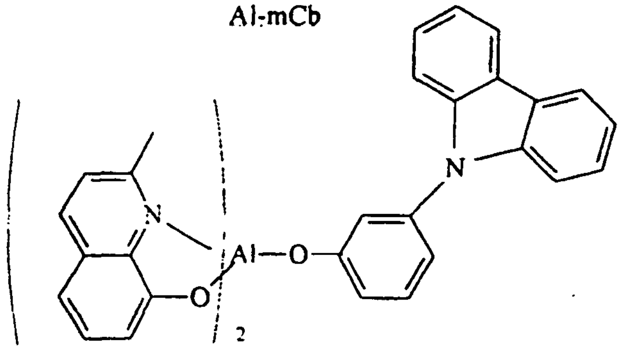

Experimental results show that solid films of Al-pCb have a photo-luminescent emission about 100 times more

efficient than that of Al-pNP. This difference may be explained by the different structure of the two molecules. In particular, Al-pCb has a more rigid conformation due to its carbazol moiety, which may prevent the molecules in the solid state from non-radiative self-quenching, which could account for the hundred times greater efficiency of the photo-luminescent emission of Al-pCb as compared to Al-pNP.

The OLEDs may comprise an emissive layer comprised of a dopant compound selected from the class of azlactone-related compounds having the chemical structure of formula F-1:

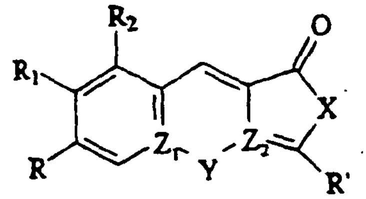

**(F-1)**

where R is hydrogen or a group which is a donor or acceptor group relative to hydrogen;
R' = alkyl or substituted or unsubstituted aryl;
$R_1$ and $R_2$ are hydrogen or are joined to form a fused aryl ring; X is O; $NR_5$, where $R_5$ is hydrogen or substituted or unsubstituted alkyl; alkyl or substituted or unsubstituted aryl;
$Z_1$ and $Z_2$ are, independently of one another, a carbon or nitrogen atom; and
Y is M, a metal atom, whenever $Z_1$ and $Z_2$ are both nitrogen atoms;
Y is O; $NR_6$, where $R_6$ is hydrogen or substituted or unsubstituted alkyl; or S;
whenever either $Z_1$ or $Z_2$ is a carbon atom; or
Y is absent. Representative donor groups include the $\Pi$-electron donor groups such as -OR, -Br, $-NR_3R_4$, where $R_3$ and $R_4$ are, independently of one another, hydrogen or substituted or unsubstituted alkyl. Representative acceptor groups include the $\Pi$-electron acceptor groups such as -CN, $-NO_2$ or a carbonyl-containing group.

More specifically, the OLED comprises an emissive layer comprised of a dopant azlactone-based compound having the chemical structure of formula C-II:

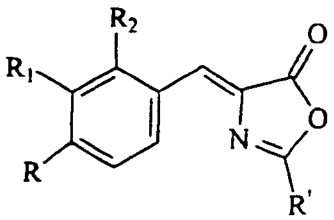

(C-II)

where R, R', $R_1$ and $R_2$ have the same meaning as above.

As a further still more specific representative embodiment, the present invention is directed to an OLED comprising an emissive layer comprised of a dopant azlactone-based compound having the chemical structure of formula C-III:

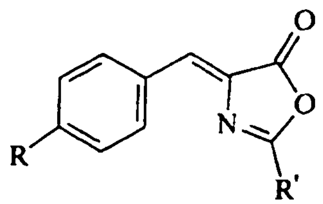

(C-III)

where R and R' have the same meaning as above.

Still more specifically, the dopant azlactone-based compound may have the chemical structure of formula C-III wherein R = H and R' = $C_6H_5$ (Compound 1); R = OOCCH, and R' = $C_6H_5$ (Compound 2); R = $N(CH_3)_2$ and R' = $C_6H_5$ (Compound 3); and R = $C(CH_3)_3$ and R' = $C_6H_5$ (Compound 4).

As still another specific embodiment of the present invention, the dopant azlactone-based compound may have the chemical structure of formula C-IV:

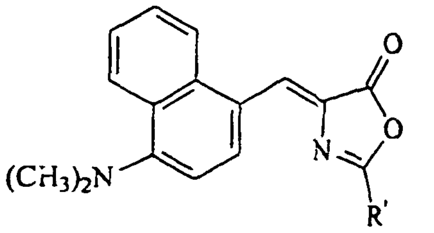

(C-IV)

where R' = $C_6H_5$ (Compound 5).

As yet other specific embodiment of the present invention, the dopant azlactone-based compound may have a chemical structure of formula C-V (Compound 6):

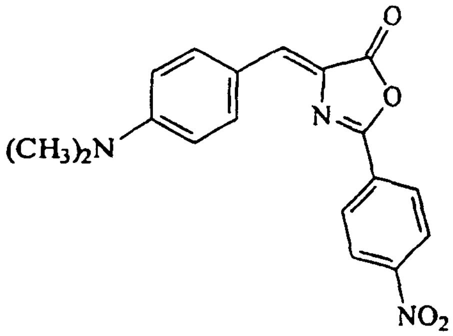

(C-V)

or a chemical structure of formula C-VI (Compound 7):

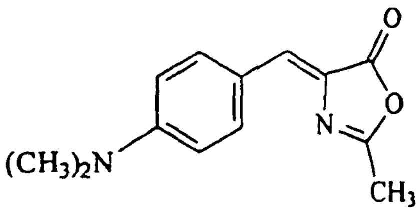

(C-VI)

These azlactone-based compounds may be prepared from para-substituted benzaldehyde and hippuric acid derivatives. By varying the para-substituted donor group, the emission can be tuned from the green into the blue part of the spectrum, as illustrated by the results in Table C1 below.

Table C1: Fluorescence data showing the absorption maxima and the photoluminescence (PL) maxima for compounds 1-7.

| | Abs. Max. (nm) | PL Max. (nm) |
|---|---|---|
| Compound 1 | 364 ($CH_2Cl_2$)<br>367 (DMSO) | 416 ($CH_2Cl_2$) |
| Compound 2 | 368 ($CH_2Cl_2$) | 422 ($CH_2Cl_2$) |
| Compound 3 | 471 ($CH_2Cl_2$)<br>481 (DMSO)<br>464 (hexane) | 522 ($CH_2Cl_2$)<br>552 (DMSO)<br>464 (hexane) |

Table continued

| | Abs. Max. (nm) | PL Max. (nm) |
|---|---|---|
| | 467 (toluene) | 500 (toluene) |
| Compound 4 | 373 ($CH_2Cl_2$)<br>374 (DMSO) | 428 ($CH_2Cl_2$) |
| Compound 5 | 368 ($CH_2Cl_2$)<br>471 (shoulder, $CH_2Cl_2$) | 518 ($CH_2Cl_2$, excit. = 360nm)<br>582 ($CH_2Cl_2$, excit. = 470nm) |
| Compound 6 | 513 ($CH_2Cl_2$)<br><br>518 (DSMO)<br>498 (hexane)<br>499 (toluene) | 734 ($CH_2Cl_2$, excit. = 320nm)<br>752 ($CH_2Cl_2$, excit. = 520nm)<br>406 (DSMO)<br>524 (hexane)<br>602 (toluene) |
| Compound 7 | 438 (DMSO) | very weak emission |

The color of the fluorescence from the materials shown in Table C1 is strongly solvent dependent as is expected for emission from a charge transfer transition.

Still other specific examples of dopant azlactone-based compounds include the chemical structure of formula C-III wherein R = OH and R' = $C_6H_5$ and the compound having the chemical structure of formula C-III wherein R = $C(CH_3)_3$ and R' = $CH_3$.

Compound 6 is an example of a compound having a charge transfer network different from that of the derivatives of Compound 1. Emission in this case comes from the amine donor and nitro group acceptor. This dye can be pumped either in the blue or in the green to get red emission, as shown for Compound 6 in $CH_2Cl_2$. Compound 6 illustrates that these dyes can have different excitation wavelengths leading to red emission. Though the PL efficiency for this dye is very poor, derivatives with other acceptors are expected to be capable of increasing this efficiency.

The line widths observed in the doped OLED for a representative compound of formulas C-II or C-III, Compound 3, are narrower than those seen for $Alq_3$ alone, but are broader than those observed in solution. The reason for this is believed to be that in solution the molecule is free to adopt the planar structure suggested by the scheme shown in Formula C-II, while in the solid state there tends to be distribution of dihedral angles about the bond connecting the phenyl ring bearing the dimethylamino group to the carbonyl moiety. This dispersion gives rise to a range of energies for both absorption and fluorescence and increases the linewidth observed. This dispersion also decreases the overall fluorescence quantum yield.

In order to prevent this solid state broadening, materials may be used which have a locked chemical configuration comprised of a rigid or locked molecule as shown in Formula C-I. In these compounds, the ring groups are held oplanar by the Y group. Thus, while the present invention is demonstrated with respect to the representative embodiments of the azlactone-based compounds of formulas C-II or C-III, the azlactone-related compounds of formula C-I also fall fully within the scope of the present invention. The azlactone-related compounds embraced by formula C-I include, but are not limited to, the azlactone-based compounds of formulas C-II and C-III.

A dopant capable of shifting the emission wavelength of an emissive layer comprised only of a host compound is added to the host compound in an amount effective to shift the wavelength of emission so that the LED device preferably emits light that is perceived by the human eye to be close to one of the primary colors. Although it is recognized that characterization of color perception is a subjective exercise, a quantitative chromaticity scale has been developed by the Commission Internationale de l'Eclairage (International Commission of Illumination), otherwise known as the CIE standard. According to this standard, a saturated color may be represented by a single point, with specific quantitative coordinates according to the defined axes of the chromaticity scale. It will be appreciated by one of skill in the art that such a single point on the CIE scale would represent a standard or a goal that, in practical terms, is difficult, but fortunately, unnecessary, to attain.

In the preferred embodiments of the present invention in which the OLED predominantly produces a primary color, the dopant is incorporated into a host compound so that the OLED emits light that is perceived by the human eye to be close to a saturated primary color. Through the practice of the present invention, it is intended that OLEDs be constructed which can be characterized by an emission that is close to an absolute (or saturated) chromaticity value, as that would be defined by the CIE scale. Furthermore, LED's utilizing the materials of the present invention are also intended to be capable of a display brightness that can be in excess of 100 $cd/m^2$although somewhat lower values, perhaps as low as 10 $cd/m^2$, may be acceptable in certain cases.

The host compounds as defined herein are compounds which can be doped with dopants to emit light with the

desired spectral characteristics. Such compounds include, but are not limited to, both emitting compounds and host compounds as described in US 6 358 631 A. The term "host" is used to refer to the compound in the emissive layer that functions as the component which receives the hole/electron recombination energy and then by an emission/absorption energy transfer process, transfers that excitation energy to the dopant compound, which is typically present in much lower concentrations. The dopant may then relax to an excited state having a slightly lower energy level, which preferentially radiates all of its energy as luminescent emission in a desired spectral region. A dopant that radiates 100% of the dopant's excited state excitation energy is said to have a quantum efficiency of 100%. For host/dopant concentrations which are to be used in a color tunable SOLED, preferably most, if not all, of the host's excitation energy is transferred to the dopant which in turn radiates, perhaps from a lower energy level, but with a high quantum efficiency, to produce visible radiation having a desired chromaticity. In the present invention classes of azlactone-related compounds may serve as dopants which satisfy these demanding energy transfer requirements.

As the term host compound is used herein, it will be appreciated that such compounds can be found in either an electron transporting/emissive layer of a single heterostructure OLED device or in the separate emissive layer of a double heterostructure device. As will be recognized by one of skilled in the art, use of the dopant species such as disclosed herein makes it possible to extend not only the range of colors emitted by the OLED, but also to extend the range of possible candidate species for host and/or dopant compounds. Accordingly, for effective host/dopant systems, although the host compound can have a strong emission in a region of the spectrum where the dopant species strongly absorbs light, the host species preferably does not have an emission band in a region where the dopant also emits strongly. In structures where the host compound also functions as a charge carrier, then additional criteria such as redox potential for the species also becomes a consideration. In general, however, the spectral characteristics of the host and dopant species are the most important criteria.

The amount of dopant that is present is that amount which is sufficient to shift the emission wavelength of the host material as close as possible to a saturated primary color, as that would be defined according to the CIE scale. Typically, the effective amount is from about 0.01 to 10.0 mol %, based on the emitting layer. The preferred amount is from about 0.1 to 1.0 mol %. The primary criterion for determining an appropriate doping level is the level which is effective for achieving an emission with the appropriate spectral characteristics. By way of example, and without limitation, if the amount of dopant species is at too low a level, then emission from the device will also comprise a component of light from the host compound itself, which will be at shorter wavelengths than the desired emission form the dopant species. In contrast, if the level of dopant is too high, emission efficiencies could be adversely affected by self-quenching, a net non-emissive mechanism. Alternatively, too high levels of the dopant species could also adversely affect the hole or electron transporting properties of the host material.

The emission from the OLED may be obtained via a phosphorescent decay process wherein the phosphorescent decay rate is rapid enough to meet the requirements of a display device. As a representative embodiment of the present invention the emission layer is comprised of an emitting compound having a structure represented by Formula D-I:

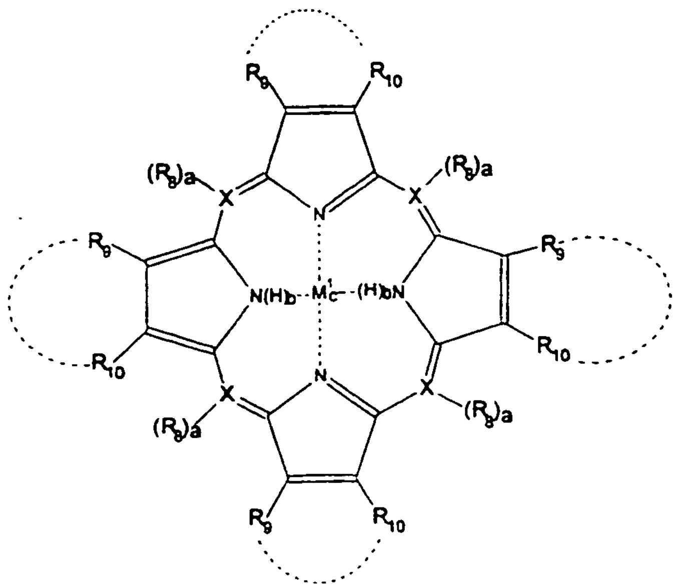

D-I

wherein M = Pt; a = 1 ; b = 0; c = 1; X = C; and $R_8$ = H; and
$R_9$ = $R_{10}$ = Et (ethyl). In particular, this compound, platinum octaethylporphine (PtOEP), has the chemical structure of

formula D-II:

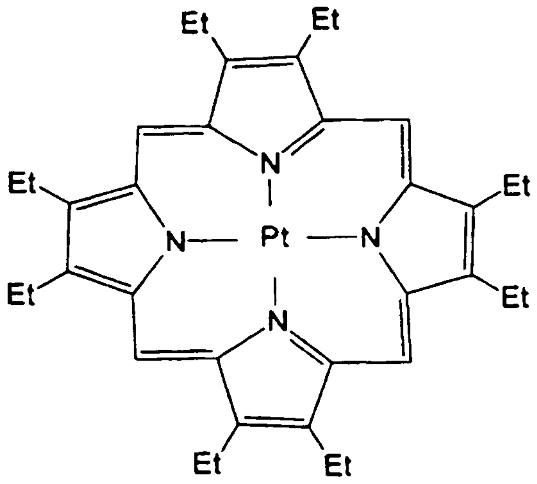

II

The advantage of selecting a dopant compound such as PtOEP as the emissive material of an OLED is based, *inter alia,* on two particular facts. First, the photoluminescent quantum yield for this molecule is significantly greater than TPP.

PtOEP having a photoluminescent quantum yield of greater than 50%, and as high as 90% in the solid state, and TPP having a photoluminescent quantum yield of only about 10%. The improved photoluminescent quantum yield makes it possible to fabricate OLEDs with increased efficiencies. A second advantage that is offered by selecting a phosphorescent compound such as PtOEP is that the emission from such a molecule comes from a triplet state. A molecule that is capable of being excited to a triplet state provides the possibility of having the energy transferred from the non-emissive exciton triplet state to a triplet state that is capable of radiatively emitting this energy as phosphorescent radiation. Though phosphorescence, which refers to radiation that comes from a triplet state, typically occurs at a much slower rate than fluorescence, which refers to radiation from a singlet state, the phosphorescence from a compound such as PtOEP is, nevertheless, sufficiently rapid to satisfy the requirements of certain display devices. In particular, a compound such as PtOEP, which has a lifetime of about 7 μsec when used as the dopant in an $Alq_3$ layer, may be used in passive matrix displays that require a switching time of not faster than about 10 μsec or in an active matrix display for which the switching time only needs to be about 10 msec.

The PtOEP may be doped into the $Alq_3$ layer of an ITO/TPD/$Alq_3$/Mg-Ag OLED. The behavior of such PtOEP-doped OLEDs is very different from OLEDs prepared with TPP dopants. At doping levels greater than 0.5 mol % TPP, the emission from the OLED is exclusively from the TPP. In contrast, at low to moderate doping levels of PtOEP in $Alq_3$, the emission is dominated by PtOEP emission at low voltage, but as the voltage is increased, $Alq_3$ emission appears. At moderately high voltages (e.g., 15V) the majority of the emission comes from $Alq_3$. The EL spectra for a 0.6 mol % PtOEP doped OLED are given in Fig. D2. The spectra for 1.3 mol % PtOEP have about the same shape as those shown for the 0.6 mol % device. The shape of the spectra of an OLED prepared with 6 mol % PtOEP are shown in Fig. D3. As the voltage is increased, the intensity of the red emission increases significantly, but a contribution from $Alq_3$ emission is not observed, even at a high voltage.

It is believed that the explanation for the increase in $Alq_3$ emission as the voltage is increased is related to the different lifetimes for photoluminescence for $Alq_3$ and PtOEP. The PL lifetime for $Alq_3$ is about 13 nsec (nanoseconds) in both the solid state and in solution, whereas the PL lifetime of PtOEP varies from about 10 to about 100 μsec (microseconds) depending on the medium. If the voltage applied to the PtOEP-doped device is kept low, the number of excitons transferred to PtOEP is small enough such that the excited PtOEP molecules can relax at a sufficient rate relative to the $Alq_3$ exciton creation rate, with the result that there are always enough dopant molecules for energy transfer from the $Alq_3$. As the voltage is increased, the available PtOEP dopant molecules become saturated and cannot relax fast enough to keep up with the rate at which the excitons are being created in the $Alq_3$. In this higher voltage regime, some of the $Alq_3$ excitons relax by radiative emission before the excitation energy can be transferred to the PtOEP molecules. At 6 mol % PtOEP, enough dopant is present to trap all of the excitons, but the higher doping levels lead to decreased overall efficiency.

This explanation is further supported by the results shown in Fig. D4, which show the PL spectra as a function of wavelength at different doping levels for PtOEP-doped $Alq_3$ devices. At the lowest doping levels of 0.6 mol %, a large

emission band characteristic of $Alq_3$ is observed, whereas for the high 6 mol % PtOEP-doping level, there appears to be sufficient PtOEP present to capture all the exciton energy from the $Alq_3$.

The emission from the PtOEP-based OLEDs is very narrow and centered at 645 nm. This narrow band, which corresponds to saturated red emission, has a full width at half maximum of about 30 nm. A comparison of the PL spectra as a function of wavelength at different excitation wavelengths for PtOEP in solution, as shown in Fig. D5, with the EL spectrum of a PtOEP-doped $Alq_3$ OLED, shows that a PtOEP-doped OLED selectively produces the narrow band of emission from PtOEP that is centered with a peak at about 645 nm. This narrow, highly saturated red emission is produced, with almost the total absence of the other PtOEP peaks, even though a comparison of the PL excitation spectrum of PtOEP with the broad emission band from the $Alq_3$ might lead one to expect additional bands centered at about 620 and about 685 nm, as is observed for the PL spectra of PtOEP in solution.

The net result is that the emission from a PtOEP-doped device is significantly better, with respect to the saturated red emission, than that of a TPP-doped device since there is no long wavelength tail or peak above 700 nm. The quantum efficiencies for these devices, which are external quantum yields, are listed in Table D1. In each case, the efficiency is listed along with that of a reference device (ITO/TPD/$Alq_3$/Mg-Ag) prepared in parallel with the doped device. At low drive voltages, the efficiencies of the doped devices are superior, while at higher voltages the undoped device has a higher efficiency. These results show that PtOEP-doped devices are capable of performing with efficiencies comparable to prior art $Alq_3$-doped devices.

The shelf lives of PtOEP devices that were exposed to ambient environmental conditions for a few days were observed to be comparable to undoped $Alq_3$ devices and decidedly superior to devices prepared with TPP as the dopant.

Table D1. External quantum efficiencies for PtOEP-doped $Alq_3$ OLEDs as compared with an undoped $Alq_3$ OLED reference.

| Conc. | Voltage | n(doped) | n(ref.) |
|---|---|---|---|
| 0.6 mol % | low volt(6-7V) | 0.2% | 0.15% |
| | high volt(11-12V) | 0.07% | 0.18% |
| 1.3 mol % | low volt(6-7V) | 0.2% | 0.11% |
| | high volt(11-12V) | 0.11% | 0.24% |
| 6 mol % | low volt (6-7V) | 0.14% | 0.17% |
| | high volt(11-12V) | 0.07% | 0.2% |

Such OLEDs may be used, for example, in passive matrix flat panel displays having a switching time not faster than about 10 μsec, in active matrix displays for which the switching time only needs to be about 10 msec, or in low resolution display applications. The phosphorescent compounds may be generally selected from those phosphorescent compounds which have the chemical structure of formula D-I:

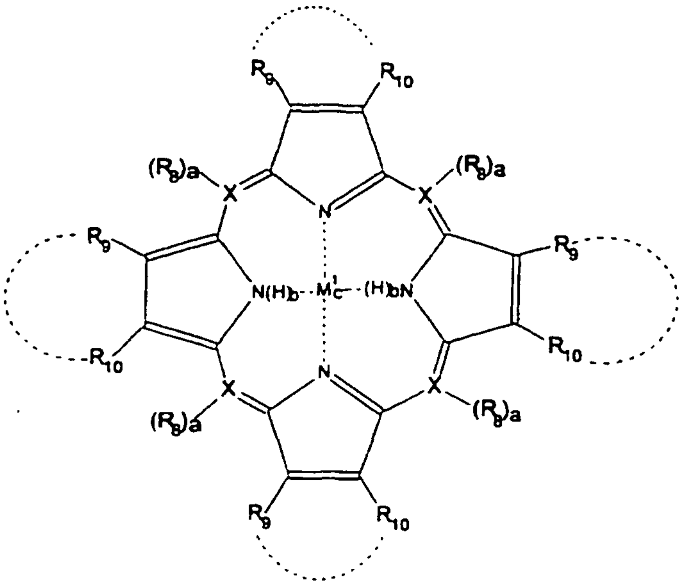

D-I

wherein X is C or N;
$R_8$, $R_9$ and $R_{10}$ are each independently selected from the group consisting of hydrogen, alkyl, substituted alkyl, aryl and substituted aryl;
$R_9$ and $R_{10}$ may be combined together to form a fused ring;
M, is a divalent, trivalent or tetravalent metal; and
a, b and c are each 0 or 1;
wherein, when X is C, then a is 1; when X is N, then a is 0;
when c is 1, then b is 0; and when b is 1, c is 0.

The phosphorescent compounds may also be selected, as another example, from phosphorescent porphyrin compounds, which may be partly or fully hydrogenated.

In addition to selecting phosphorescent compounds according to their phosphorescent lifetimes, which for certain applications may mean selecting compounds having a phosphorescent lifetime not longer than about 10 μsec, the phosphorescent compounds may be selected according to their ability to effectively capture the exciton triplet energy from a charge carrier material and then to emit that excitation energy as phosphorescence in a narrow emission band corresponding to a highly saturated color, such as demonstrated by PtOEP in an $Alq_3$-based OLED

Though PtOEP may itself yet prove to have the most desirable combination of properties for useas the phosphorescent compound in an OLED, such a compound has the disadvantage of producing the saturated red emission near the edge of the eye sensitivity curve, for which the standardized CIE photopic response function of the human eye is centered at bout 550 nm. In particular, at the blue and red ends of the spectrum, there is a steep reduction in the eye sensitivity as a function of wavelength.

It would be desirable if phosphorescent compounds could be found which produce what is perceived to be a saturated red emission with a high external quantum efficiency, but with narrow peaks at somewhat shorter wavelengths for which there is a substantially higher eye sensitivity. For example, if the emission bandwidth could be kept substantially constant and the emission peak shifted about 20 nm toward shorter wavelengths, for an OLED producing the same number of photons, for example, an OLED with the same current and quantum yield, the perceived brightness of the device could be increased by a factor of about two. That is, though the number of photons coming from the two devices would be the same, the standard observer would perceive a factor of two increase in brightness for the device having the peak at the shorter wavelength. The emission would, however, be in a region that would still be perceived as saturated red and, thus, still be useful in an OLED.

The emission from the OLED may be obtained via a phosphorescent decay process wherein the phosphorescent decay rate is rapid enough to meet the requirements of a display device, and wherein, in particular, the phosphorescent dopant compound is comprised of a platinum porphyrin having reduced symmetry as compared, for example, with the 4-fold symmetry of PtOEP.

The advantage of selecting a phosphorescent dopant compound such as a platinum porphyrin compound as the emissive material of an OLED is based, *inter alia*, on two particular facts. First, the photoluminescent quantum yield for such compounds may be significantly greater than prior art compounds such as TPP. For example, PtOEP has a photoluminescent quantum yield of greater than 50%, and as high as 90% in the solid state, and TPP has a photoluminescent quantum yield of only about 10%.

An improved photoluminescent quantum yield offers the possibility of fabricating OLEDs with increased efficiencies.

A second advantage that is offered by selecting a phosphorescent compound such as a platinum porphyrin compound is that the emission from such a compound typically comes from a triplet state. A molecule that is capable of being excited to a triplet state provides the possibility of having the energy transferred from the non-emissive exciton triplet state to a triplet state that is capable of radiatively emitting this energy as phosphorescent radiation. Though phosphorescence, which refers to radiation that comes from a triplet state, typically occurs at a much slower rate than fluorescence, which refers to radiation from a singlet state, the phosphorescence from a compound such as a platinum porphyrin compound may be sufficiently rapid to satisfy the requirements of certain display devices. In particular, a compound such as PtOEP, which has a lifetime of about 7 μsec when used as the dopant in an $Alq_3$ layer, may be used in passive matrix displays that require a switching time of not faster than about 10 μsec or in an active matrix display for which the switching time only needs to be about 10 msec.

A specific advantage is that phosphorescent platinum porphyrin compounds are selected so as to have an emission peak shifted toward the peak of the eye sensitivity curve, while still remaining in a spectral region that is perceived as saturated red. In particular, by chemically modifying compounds such as PtOEP by breaking the 4-fold symmetry of the porphyrin ligand, it has been found that the emission peaks can be shifted about 15-30 nm toward shorter wavelengths, as compared with PtOEP.

The OLED may contain a phosphorescent dopant compound having the structure with the formula E-II:

(E-II)

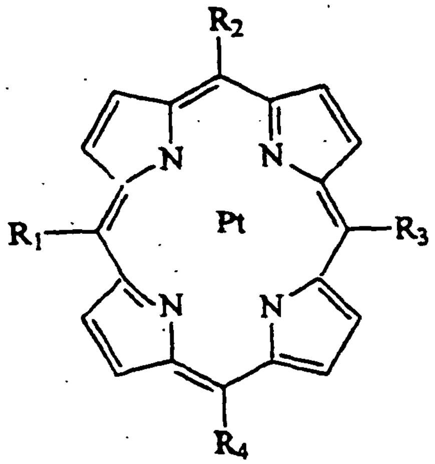

where the R-groups $R_1$, $R_2$, $R_3$ and $R_4$ are, independently of one another, alkyl, aryl or hydrogen, with the proviso that at least one of the R-groups is different from at least one other R-group.

Still more specifically, the OLEDs are comprised of phosphorescent compounds having the structure with the formula E-III:

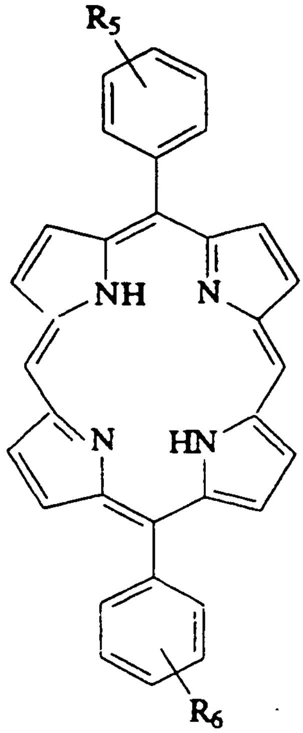

(E-III)

wherein $R_5$ and $R_6$ may be an electron donor or electron acceptor group, for example. -F, -CN or $-OCH_3$, and $R_5$ and $R_6$ may be the same or different.

As representative compounds to be used in OLEDS, the phosphorescent platinum compound may be selected by selectively substituting a phenyl-group at the 5.15 positions of the porphyrin ring so as to arrive at the platinum (II) 5.15-memo-diphenylporphyrin compound (PtDPP) as shown in Fig. E1. Alternatively, a platinum compound may be prepared from any one of the remaining 5,15-substituted porphyrin compounds that are also shown in Fig. E1. In particular, as representative embodiments of the present invention, the phosphorescent platinum porphyrin compound may be prepared from 5,15-memo-diphenylporphyrin ($H_2$DPP); 5,15-*memo*-bis(pentafluorophenyl)porphyrin ($H_2$BPFPP); 5,15-*memo*-bis(4-cyanophenyl)porphyrin ($H_2$BCPP); or 5,15-*memo*-bis(4-anisyl)porphyrin ($H_2$BAP). Such compounds may be prepared using methods such as described hereinafter.

OLEDs were fabricated in which PtDPP was doped as the phosphorescent platinum porphyrin compound. As shown in Fig. E2, the electroluminescent spectra of an OLED having PtDPP doped into a polymer mixture of polyvinyl carbazole (which is referred to as "PtDPP doped polymer OLED") produced an emission peak near the photoluminescence peak of PtDPP in polystyrene. These peaks were shifted about 20 nm towards lower wavelengths as compared with the electroluminescent spectra of an OLED having a PtDPP-doped $Alq_3$ layer.

As shown in Fig. E3a, for OLEDs comprised of layers of ITO/NPD/$Alq_3$-PtDPP/MgAg, (PtDPP being doped in the $Alq_3$ layer), an electroluminescent spectra is produced which has a narrow band of emission peaking at about 630 nm for OLEDs operated at 9V, 15V or 24V. The $Alq_3$-based device produced a small blue shift as the voltage was increased, apparently due to the increase in the $Alq_3$ emission in the spectra region from 500 to 550 nm. As shown in Fig. E3b, for an OLED having the $Alq_3$ layer doped with PtDPP, the emission peak was shifted about 15 nm towards shorter wavelengths as compared with the emission peak produced by an OLED having the $Alq_3$ layer doped with PtOEP. These results show that for OLEDs having equal quantum yields, an OLED having a PtDPP-doped $Alq_3$ layer would have 1.4 times the brightness of an OLED having a PtOEP-doped $Alq_3$ layer.

The practical significance of fabricating OLEDs having a 15-20 nm blue shift in the emission peak can be illustrated by the data as shown in the right hand corner of the CIE chromaticity diagram, which is shown in Fig. E4. Shown in this figure is the CIE chromaticity curve for which the (x, y) coordinates of saturated monochromatic lines at 600, 650 and 700 nm, respectively, are included. The (x,y) coordinates of the emission produced by an OLED having a PtDPP-doped $Alq_3$ layer is compared with OLEDs containing other red-emitting dopants, such as DCM2, indigo and PtOEP.

By using the CIE standardized photopic response curve for human eye sensitivity, which is shown as the broken line in Fig. E5, the relative brightness at a given photopic output level can be determined by calculating the overlap of the normalized emission spectra with the photopic response curve, as shown by the following equation,

$$I_{lumens} \propto \int (\text{photopic response})(\text{EL spectrum}) d\lambda,$$

where $I_{lumens}$ is the perceived intensity in lumens. This function may be referred to as the luminous overlap integral. As illustrated by the representative data shown in Fig. E5, the luminous overlap integral of a PtDPP-emitting OLED, 0.17 (relative units), is significantly greater than that of a PtOEP-emitting OLED, 0.13. The net result is that for a PtDPP-emitting OLED, the CIE coordinates remain in the saturated red region of the chromaticity diagram, while the perceived brightness may be up to 40% or more greater than for a PtOEP-emitting OLED.

Though the luminous overlap integral of a DCM2-emitting OLED would be significantly higher than either PtDPP or PtOEP (0.19 vs. 0.17 or 0.13, respectively), that is, if the same photopic output levels could be realized, a DCM2-emitting OLED is not in fact nearly as bright, since the quantum efficiency of a DCM2-emitting OLED is substantially less than can be realized for OLEDs fabricated with the phosphorescent Pt-containing compounds.

DCM2, which has a structure represented by the formula:

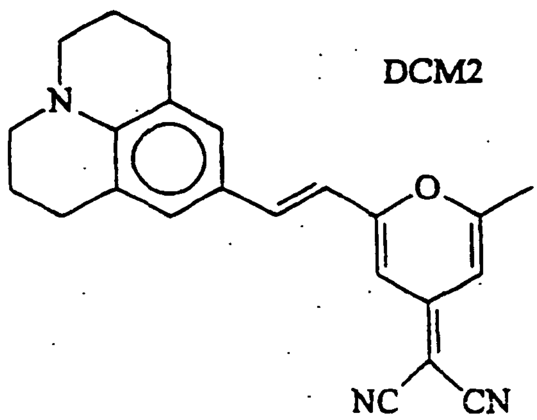

DCM2

has been described as a red emitting chromophore useful in OLED applications. *C. W. Tang et al., Electroluminescence of doped organic thin films, J. Appl. Phys. 65, 3610 (1989).*

The organic light emitting devices may comprise a heterostructure for producing electroluminescence having a hole transporting layer with a glass structure. The hole transporting layer comprises a compound having a symmetric molecular structure. The end groups of the symmetric molecule are hole transporting amine moieties having an unsaturated linkage between two arenes.

The term "unsaturated linkage" as used herein refers to a linkage in which there is at lest one double bond. The term "arene" as used herein refers to a hydrocarbon containing at least one aromatic. The term "symmetric" as used herein refers to a molecule having a point about which the molecule is symmetric. As used herein, the term "charge carrier layer" may refer to a "hole transporting layer" (HTL), an "electron transporting layer" (ETL) or, for an OLED having a double heterostructure (DH), a "separate emissive layer".

The term "hole transporting moiety" as used herein, refers to a group which, when present in a material contained in a layer of an OLED, causes the material to provide electrical conduction through the layer, when a voltage is applied, predominantly by the conduction of holes. The term "electron transporting moiety" as used herein, refers to a group which, when present in a material contained in a layer of an OLED, causes the material to provide electrical conduction through the layer, when a voltage is applied, predominantly by the conduction of electrons. The term "hole transporting amine moiety" as used herein, refers to an amine group that is a hole transporting moiety. Such hole transporting amine moieties are typically comprised of nitrogen atoms that are directly bonded to at least two phenyl groups, wherein the two phenyl groups may be joined so as to form a heterocyclic ring including the nitrogen, for example, a carbazole group, or the two phenyl groups may be unattached to each other. Each phenyl group may itself be fused with still another phenyl group, being bonded to the nitrogen atom, for example, either as a 1-naphthyl group or as a 2-naphthyl group.

While not intending to be limited by any particular theory or mechanism for explaining exactly how or why such materials have good hole conducting properties, the inventors' examination of electronic structure by semi-empirical theoretical methods shows that a molecule having end groups that are hole transporting moieties having an unsaturated linkage between two arenes has holes delocalized onto the end groups. One such molecule is 4,4'-(N,N'-bisiminostilbene)biphenyl (ISB). By way of contrast, the normal situation in amines such as TPD and NPD is for the nitrogen lone pair to be conjugated through the biphenyl group, such that the hole is delocalized predominantly onto the biphenyl

group. The importance of the unsaturated linkage can be shown by examining 4,4'-(N,N'-iminodibenzyl)biphenyl (IDB), which has the same structure as ISB, except that the IDB has a saturated linkage between the amino phenyl groups, while that of ISB is unsaturated. The inventors analysis shows that IDB has a nitrogen lone pair conjugated through the biphenyl group, whereas ISB has the nitrogen lone pair coupled to the stilbene group, not the biphenyl. To the extent that the hole is delocalized, it is spread onto the stilbene group. This delocalization helps keep the hole on the exterior of the ISB molecule and not on the biphenyl, where it would be shielded from adjacent molecules. Keeping the hole on the exterior of the molecule gives it more contact with adjacent molecules and increases the rate of hole transfer to adjacent molecules, which results in good hole conducting properties.

Moreover, the inventors' analysis shows that substitutions may be made to molecules having end groups that have an unsaturated linkage between two arenes, and the holes would still be delocalized on the ends or periphery of the molecule rather than the center.

In particular, compounds useful in the present invention include the symmetric compounds having a biphenyl bridge, as represented by formula F-I:

(F-I)

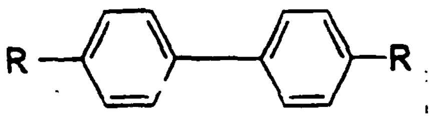

where R is a hole transporting amine moiety having an unsaturated linkage between two arenes. The molecule represented by formula F-I is symmetric because there is a point in the center of the biphenyl bridge between the two R-groups about which the molecule is symmetric. The term "symmetric" as used herein requires that atoms are on either side of the point of symmetry have the same sequence of bonding between the atoms, i.e., the R-groups must have identical atoms bonded in an identical sequence, but allows for differences in the positions of atoms due to the twisting of bonds.

Further useful compounds include symmetric compounds have a phenyl bridge, as represented by formula F-II:

(II)

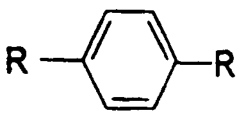

wherein R has the same meaning as above.

An example of an R-group that is a hole transporting amine moiety having an unsaturated linkage between two arenes is represented by the formula F-III:

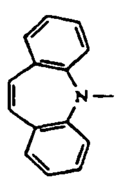

(F-III)

where the two phenyl groups are the arenes, and the ethenyl group is the unsaturated linkage between the two arenes.

Using the R-group of formula F-III in the molecule of formula F-I, the present invention therefore includes ISB, represented by the formula F-IV:

(F-IV)

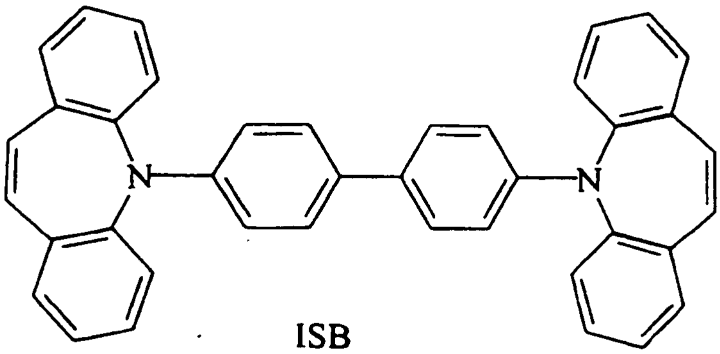

ISB

The ethenyl groups of ISB may be substituted while still maintaining the unsaturated linkage between the two arenes. For example, the unsaturated linkage may, in fact, be provided by a phenylene group, resulting in a molecule having a structure as represented by formula F-V:

(F-V)

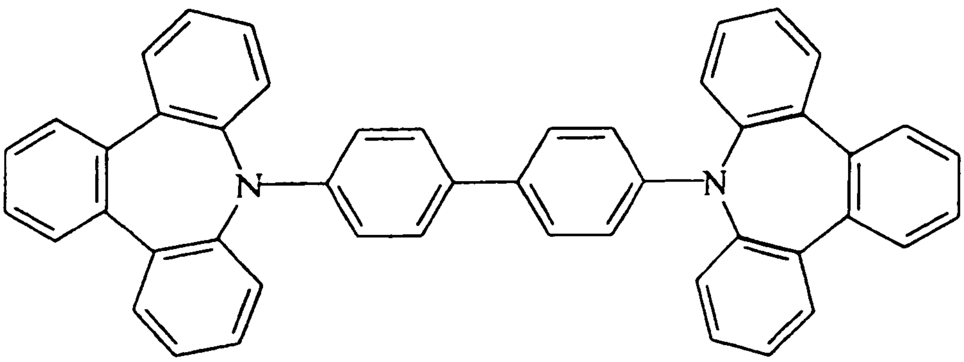

Alternatively, the ethenyl groups of ISB may be substituted so as to result in a molecule having a structure as represented by formula F-VI:

(F-VI)

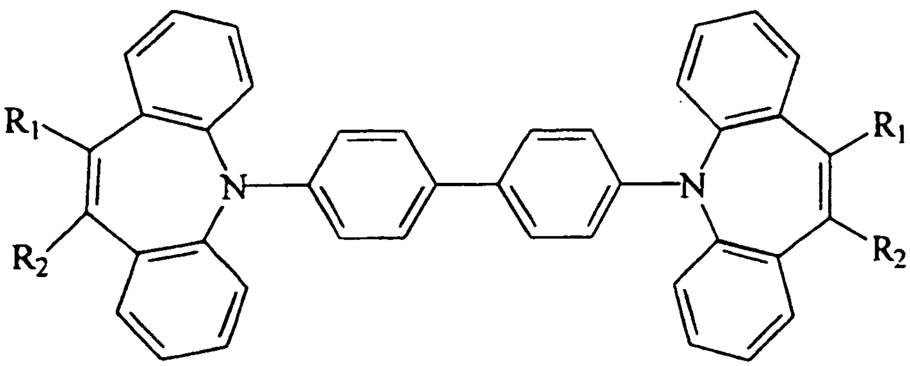

where $R_1$ and $R_2$ are selected from the group consisting of: alkyl, phenyl, substituted alkyl, and substituted phenyl groups. $R_1$ may be the same as $R_2$, or may be different The substitutions leading to the molecules of formulae F-V and F-VI are expected to assist in shifting the hole delocalization to the ends of the molecule. In addition, the substitutions increase

the molecular weight of the molecule and may lead to a higher $T_g$.

In a conventional single heterostructure OLED, the emissive material is the ETL, and the HTL must have an absorption energy higher than that of the ETL. As a result, it is preferable that substitutions made to ISB do not lead to significant shifts in the electronic spectrum if the resultant molecule is to be used in a single heterostructure OLED having an emissive ETL.

To provide a contrast to ISB, a molecule having an R-group that is a hole transporting amine moiety having a saturated linkage between two arenes may be used. Such an R-group is represented by the formula F-VII:

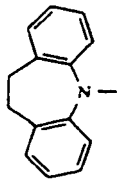

(F-VII)

Using the R-group of formula F-VII in the molecule of formula F-I results in 4,4'-(N,N'-aminodibenryl)biphenyl (IDB), as represented by the formula F-VIII:

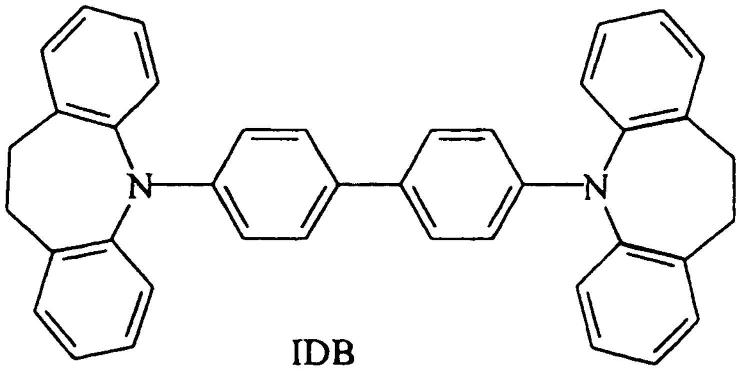

(F-VIII)

IDB

The thermal and other physical properties of ISB and IDB, as well as those of their phenylene bridged analogs, are given in Table 1:

TABLE F1: Physical data for ISB, IDB and their phenyl bridged analogs.

| compound | melting point (°C) | $T_g$(°C) | $\lambda_{max}$ abs. (nm) | $\lambda_{max}$ PL (nm) |
|---|---|---|---|---|
| ISB | 317 | 110 | 300, 340 | 530 |
| IDB | -- | 117 | 320 | 402 |
| 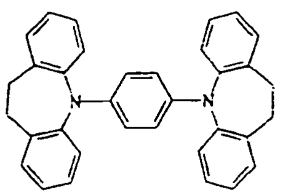 | -- | 73 | 315 | 368 |
| 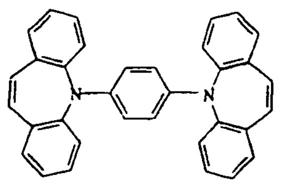 | 310 | 110 | 290, 340 | 444, 488 |

The $T_g$ of both ISB (110°C) and IDB (117°C) are significantly higher than that of HTL materials conventionally used in OLEDs, such as TPD (65°C) and NPD (105°C), which are materials conventionally used in OLEDs. As a result, OLEDs using ISB or IDB as an HTL may be operated at a higher temperature than OLEDs using TPD or NPD, and are expected to have a longer lifetime when operated at the same temperature.

As discussed in greater detail below, two different types of OLEDS were fabricated using ISB and IDB as HTL materials. Similar OLEDs were also fabricated using TPD and NPD as HTL materials. Both types of OLED start with an ITO coated substrate as an anode and use a Mg-Ag cathode. The simplest OLED structure examined was ITO/HTUAl$q_3$/Mg-Ag. A slightly more complicated structure uses a copper phthalocyanine, CuPc, hole injector, *i.e.* ITO/CuPc/HTUAl$q_3$/Mg-Ag. The use of a CuPc hole injector, such as disclosed in US 5 998 803 A can provide improved quantum yields. As illustrated in Table F2, it was observed for both types of OLED that OLEDS using ISB as the HTL have superior performance to those using IDB, and that OLEDs using ISB have performance comparable to that of OLEDs using NPD:

TABLE F2

| | Quantum Efficiency with CuPc layer | Quantum Efficiency without CuPc layer | V @ 0.1mA with CuPc layer | V @ 0.1mA without CuPc layer | Power Q.E. without CuPc layer at 200 cd/m$^2$ (W/W) | Power Q.E. without CuPc layer at 200 cd/m$^2$ (W/W) | Luminance @ 5mA for 1 mm dot with CuPc layer | Luminance @ 5mA for mm dot without CuPc layer |
|---|---|---|---|---|---|---|---|---|
| **ISB** | 0.58% | 0.62% | 7.5 V | 9.0 V | 0.174 | 0.156 | 8460 | 8930 |
| **IDB** | 0.30% | 0.15% | 9.5 V | 12.9 V | 0.071 | 0.025 | 1510 @ 1mA | 470 @ 1mA |
| **α-NPD** | 0.85% | 0.88% | 7.3 V | 8.65 V | 0.285 | 0.251 | 12550 | 12925 |
| **TPD** | 0.78% | 0.93% | 8.25 V | 9.20 V | 0.230 | 0.250 | 11280 | 13865 |

The quantum yields, turn-on voltages and power efficiencies of the ISB based devices are very good and the higher $T_g$ suggests that the ISB based OLEDs would have significantly improved lifetime, and can be operated at higher temperatures, than NPD and TPD based OLEDs. The similarity of the TPD, NPD and ISB OLED device properties can also be seen in Figs. B1 and B2. The current-voltage plots of OLEDs made TPD, NPD and ISB are nearly indistinguishable, while IDB is poorer.

Table 2 also shows that OLEDs using ISB as the HTL have significantly better properties than OLEDs using IDB as the HTL in several respects. The ISB based OLEDs have a higher quantum efficiency, a require a lower voltage to achieve the same current, and have a higher luminance at the same current. ISB and IDB are both symmetric molecules having a high $T_g$. The only structural difference between ISB and IDB is that ISB has an unsaturated linkage between the amino phenyl groups, while that of IDB is saturated. This difference in the properties of OLEDs using ISB as opposed to IDB is consistent with ISB having better hole conducting properties than IDB. While the $T_g$ of IDB is slightly higher than that of ISB, this higher $T_g$ alone is not expected to alter the OLED properties by the amount observed here.

Representative materials that may be used as the hole-injecting anode layer in a representative embodiment of the present invention include, in particular, ITO, Zn-In-$SnO_2$ or $SbO_2$, or substantially any other material that may be used as the hole-injecting anode layer of an OLED.

Representative materials that are present as a glass are desirable for use in the HTL of an OLED, rather than as a crystalline or polycrystalline material, since glasses are capable of providing higher transparency as well as producing superior overall charge carrier characteristics as compared with the polycrystalline materials that are typically produced when thin films of the crystalline form of the materials are prepared. Representative materials that may be used in the hole transporting layer in a representative embodiment of the present invention include, in particular, N,N'-diphenyl-N,N'-bis(3-methylpheny)1-1'bipheny)-4,4'diamine (TPD).
4,4'-bis[N-(1-naphthyl)-N-phenyl-amino]biphenyl ($\alpha$-NPD) or
4,4'-bis[N-(2-naphthyl)-N-phenyl-amino]biphenyl ($\beta$-NPD).

Representative materials that may be used as the electron transporting layer include, in particular, tris-(8-hydroxyquinoline)-aluminum ($Alq_3$) or 4,4'-di(N-carbazolo)diphenyl (CBP).

Representative materials that may be used as the separate emissive layer, if present, include, in particular, dye-doped $Alq_3$, or substantially any other material that may be used as the separate emissive layer of an OLED.

In those cases wherein the OLEDs of the present invention are used in combination with another OLED to form a SOLED structure that contains a metallic cathode layer, the materials that may be used as the electron-injecting, metallic cathode layer may include, in particular, Mg-Ag, Li-Ag or Ca, or substantially any other material that may be used as the metallic cathode layer of an OLED.

The insulating layer, if present, may be comprised of an insulating material such as $SiO_2$, $SiN_x$ or $AlO_2$, or substantially any other material that may be used as the insulating material of an OLED, which may be deposited by a variety of processes such as plasma enhanced chemical vapor deposition (PECVD), electron beam, etc.

The OLEDs of the present invention have the advantage that they can be fabricated entirely from vacuum-deposited molecular organic materials. A vacuum-deposited material is one which can be deposited in a vacuum typically having a background pressure less than one atmosphere, preferably about $10^{-5}$ to about $10^{-11}$ torr for vacuum deposition, or about 50 torr to about $10^{-5}$ torr for vapor deposition.

Although not limited to the thickness ranges recited herein, the substrate may be as thin as 10 $\mu$, if present as a flexible plastic or metal foil substrate, such as aluminum foil, or substantially thicker if present as a rigid, transparent or opaque, substrate or if the substrate is comprised of a silicon-based display driver; the ITO anode layer may be from about 500 Å (1 Å = $10^{-8}$ cm) to greater than about 4000 Å thick; the hole transporting layer from about 50 Å to greater than about 1000 Å thick; the separate emissive layer of a double heterostructure, if present, from about 50 Å to about 200 Å thick; the electron transporting layer from about 50 Å to about 1000 Å thick; and the non-metallic cathode layer from about 400 Å to greater than about 1500 Å thick with about 400-1000 Å being preferred, and about 500 Å still more preferred.

Thus, while there may be substantial variation in the type, number, thickness and order of the layers that are present, dependent on whether the device includes a single heterostructure or a double heterostructure, whether the device is a SOLED or a single OLED, whether the device is a TOLED or an IOLED, whether the OLED is intended to produce emission in a preferred spectral region, or whether still other design variations are used, the present invention is directed to those devices in which the OLED is comprised of a heterostructure for producing electroluminescence wherein the heterostructure includes a cathode comprising an electrically conductive non-metallic layer in low-resistance electrical contact with a semiconductive organic layer.

The various embodiments of the present invention may be incorporated into an optoelectronic device that is included in a vehicle, a computer, a television, a printer, a large area wall, theater or stadium screen, a billboard or a sign.

In addition, while the method according to claim 1 is illustrated with embodiments in which the highly transparent non-metallic cathode is incorporated into an OLED, substantially any type of optoelectronic device having an anode and a cathode may include a highly transparent non-metallic cathode fabricated in accord with the present invention. In

particular, the non-metallic cathodes may be included in an OLED, a solar cell, a phototransistor, a laser or a photodetector.

This invention will now be described in detail with respect to showing how certain specific representative embodiments thereof can be made, the materials, apparatus and process steps being understood as examples that are intended to be illustrative only. In particular, the invention is not intended to be limited to the methods, materials, conditions, process parameters, apparatus and the like specifically recited herein.

## EXAMPLES OF THE INVENTION

### A Examples of OLEDs containing a non-metallic cathode

#### Example A1

OLEDs were prepared using known procedures except that the OLEDs included a non-metallic ITO cathode layer rather than a metallic cathode layer. In addition, an electron injecting interface layer was present between the ITO cathode and an $Alq_3$ electron transporting layer. The ITO/Borosilicate substrates that were obtained commercially had an ITO thickness of about 1500 Å. The organic layers were thermally deposited in a standard bell-jar evaporator at pressures of $1x10^{-6}$ torr. The alpha-NPD layer was deposited at a thickness of about 350 Å, the $Alq_3$ electron transporting layer was deposited at a thickness of about 450 Å and the copper phthalocyanine (CuPc) or zinc phthalocyanine (ZnPc) were deposited at a thickness of about 60 Å. The top ITO cathode layer was RF sputter-deposited at low powers and had a thickness of about 650 Å. OLEDs were also prepared containing a CBP layer between the CuPc layer and the $Alq_3$ layer. Such OLEDs showed performance characteristics comparable to the OLEDs in which no CBP layer was present.

The devices were characterized by measuring the current-voltage, luminance-current, electroluminescence spectra and the transmission, reflection and absorption spectra. Representative data are shown in Figs. A3-A11.

The results were compared with a standard OLED, for example, as shown in Fig. A1. which includes a metallic Mg:Ag cathode layer **1**, an electron transporting layer **2**, a hole transporting layer **3**, an anode layer 4 and a substrate 5. The alpha-NPD hole transporting layer had a thickness of about 350 Å, the $Alq_3$ electron transporting layer had a thickness of about 450 Å and the Mg:Ag cathode layer had a thickness of about 1500 Å.

#### Example A2

An example of a non-metallic-cathode-containing TOLED of the present invention is shown schematically in Fig. A13. The device was grown, for example, as shown in *G. Gu, V. Bulovic, P.E Burrows, S.R. Forrest, and M.E. Thompson, Appl. Phys. Lett.* ***68****, 2606 (1996)*, in a vacuum system with a base pressure $<10^{-7}$ Torr on a precleaned glass substrate coated with ITO with a sheet resistance of 20Ω/□. A 30 Å to 60 Å thick film of copper phthalocyanine (CuPc) was deposited on the ITO to improve hole injection, followed by a 350 Å to 400 Å thick film of the HTL:4.4'-bis[N-(1-napthl)-N-phenyl-amino]biphenyl(α-NPD). Next, a 400 Å to 500 Å thick film of the emissive ETL, tris-(8-hydroxyquinoline) aluminum ($Alq_3$), was grown, followed by a second 30 Å to 60 Å thick film of CuPc, *S.A. Van Slyke, C.H. Chen, and C.W. Tang, Appl. Phys. Lett.* ***69***, *2160 (1996)*. The first 200 Å of $Alq_3$ was doped to 1% (by mass) with coumarin 6 (cm6). The substrate was then transferred to the ITO sputtering chamber where 400 Å to 600 Å of ITO was radio-frequency sputtered on top of the CuPc in an Ar (200 sccm) and $O_2$(0.10 sccm) ambient at a pressure of 5 mTorr and a power of 5 W. For comparison, a conventional TOLED not having the highly transparent non-metallic cathode was fabricated along with the test device, in particular, this conventional TOLED had a similar structure with the difference that the ITO/CuPc top electrode was replaced with a 100 Å thick, semi-transparent film of Mg:Ag (30:1, mass ratio) deposited onto the $Alq_3$ surface, capped by sputtered ITO. A second series of non-metallic-cathode-containing TOLEDs was fabricated using zinc phthalocyanine (ZnPc) in place of CuPc under the top ITO layer. Finally, in a third series of devices, the electron injecting CuPc layer was replaced by a 60 Å thick layer of 3,4,9,10-perlyenetetracarboxylic dianhydride (PTCDA), which has previously been shown to be effective in protecting an underlying organic film from the damage that may otherwise be incurred during sputtering. *V. Bulovic, P. Tian, P.E. Burrows. M.R. Gokhale, and S. R. Forrest, Appl. Phys. Lett.* ***70****, 2954 (1997)*.

### B. Examples of OLEDs containing a charge carrier layer containing a compound having molecules that have at least one electron transporting moiety and at least one hole transporting moiety (outside of the invention)

#### Experimental:

UV-Visible spectra were measured on a Aviv Spectrophotometer, Model 14DS. H NMR spectra were recorded on a Bruker 250 spectrometer. Photo- and electroluminescent spectra were measured with a Photon Technology International fluorimeter. Current-Voltage characteristics measurements were recorded on an EG&G instruments Potentiostat, model 283. Melting point was measured on a Mel-Temp II device without calibration. Elemental analysis was performed by the Atlantic Microlab, Inc. Mass spectroscopic analysis was performed on a solid state probe of a Hewlett-Packard GC/MS spectrometer with a 5973 Mass Selective Detector. Gradient sublimation was conducted on a Lindberg three-zone oven with a base vacuum of $10^{-3}$ torr.

Chemicals:

Compounds that include a series of phenol derivatives bearing a triarylamine moiety which is known to have good hole transporting properties were further reacted with $Al(OPr)_3$ in the presence of two equivalents of 8-hydroxyquinaldine to create compounds bearing both electron transporting moiety and hole transporting moiety. The compounds so created are referred to as Al-pNP, Al-pCb, and Al-mCb.

TPD and $Alq_3$ were synthesized according to literature procedure, and both were sublimed before use. The ligands 4-(N-phenyl-2-naphthylamino)phenol, 3-carbazolphenol, 4-carbazolphenol, and 4-iminostilbenephenol were synthesized using the Ullman coupling of the iodoanisoles with the corresponding amines followed by deprotection of the methoxy group with $BBr_3$ as described in "Oleds Containing Thermally Stable Asymmetric Charge Carrier Materials", Serial No. 08/929,029, filed September 8, 1997, which is incorporated herein by reference. 8-hydroxyquinaldine was purchased from Aldrich and used as received.

Bis(2-methyl-8-quinolinolato)[*p*-(*N*-phenyl-2-naphthylamino)phenolato]aluminum(III), Al-pNP: To the mixture of $Al(OPr)_3$ (0.63 g, 3.1 mmol) and 8-hydroxyquinaldine (0.51 g, 3.2 mmol) were added 40 mL EtOH under argon. The mixture was refluxed for 3 hours to give a green-yellow solution. An orange-yellow solution of 8-hydroxyquinaldine (0.51 g, 3.2 mmol) and p-(*N*-phenyl-2-naphthylamino)phenol (1.45 g, 4.68 mmol) in 40 mL EtOH was then added in the air. The mixture was refluxed overnight. Solvent was then removed from the yellow solution and the yellow glassy material was then sublimed at over 300°C. The latest sublimed yellow crystalline material was collected and resublimed. An analysis of the resultant material gave the following results:
Yield: 60%
Melting Point: 136-142°C

| | | | |
|---|---|---|---|
| Elemental Composition (calculated): | C, 77.2; | H, 4.93; | N, 6.43 |
| Elemental Composition (measured): | C, 77.0; | H, 5.00; | N, 6.31 |

Mass Spectroscopy: 653 (p), 342 (p - *p*-(*N*-phenyl-2-napthylamino)phenol), 259 (*p*-(*N*-phenyl-2-napthylamino)phenol), 159 (8-hydroxyquinaldine).

Bis(2-methyl-8-quinolinolato)(*p*-carbazolphenolato)aluminum(III), Al-pCb: To the mixture of $Al(OPr)_3$ (0.63 g, 3.1 mmol) and 8-hydroxyquinaldine (0.51 g, 3.2 mmol) were added 40 mL EtOH under argon. The mixture was refluxed for 3 hours to give a green-yellow solution. An orange-yellow solution of 8-hydroxyquinaldine (0.51 g, 3.2 mmol) and p-carbazolphenol (1.2 g, 4.6 mmol) in 40 mL EtOH was then added in the air. The mixture was refluxed overnight. Solvent was then removed from the yellow solution and the yellow glassy material was then sublimed at over 300°C. The latest sublimed yellow crystalline material was collected and resublimed. An analysis of the resultant material gave the following results:
Yield: 63%
Melting Point: 283°C

| | | | |
|---|---|---|---|
| Elemental Composition (calculated): | C. 75.9; | H, 4.69; | N, 6.98 |
| Elemental Composition (measured): | C. 75.6: | H. 4.79; | N, 6.96 |

Mass Spectroscopy: 601 (p), 343 (p - *p*-carbazolphenolato), 259 (*p*-carbazolphenol), 159 (8-hydroxyquinaldine).

Bis(2-methyl-8-quinolinolato)(*m*-carbazolphenolato)aluminum(III). Al-mCb: $Al(OPr)_3$ (0.28 g. 1.37 mmol) was mixed with 8-hydroxyquinaldine (0.22 g, 1.38 mmol) in ethanol (65 mL) under argon. The mixture was refluxed under Ar for 2 hours, and the resulting yellow-green solution was filtered in the air through celite to give a clear solution. An ethanol solution (30 mL) of 2-methyl-8-quinoline (0.22 g, 1.38 mmol) and meta-carbazolphenol (0.54 g, 2.1 mmol) was added and the combined yellow-green solution was refluxed for 3 hours. Volatile materials were then removed under reduced vacuum and the glassy material was sublimed twice at 280°C through a 3-zone sublimator to give a light yellow microcrystalline solid. An analysis of the resultant material gave the following results:
Yield: 0.29 g (35%)
Melting Point: 280°C

| | | | |
|---|---|---|---|
| Elemental Composition (calculated): | C, 75.9; | H, 4.69; | N, 6.98 |
| Elemental Composition (measured): | $C_3$ 75.1; | H, 4.72; | N. 6.90 |

Mass Spectroscopy: 601 (p), 343 (p - m-carbazolphenolato), 259 (m-carbazolphenol), 159 (8-hydroxyquinaldine).

Device Fabrication and Characterization:

Borosilicate substrates coated with ITO (100Ω/□) were cleaned by sonicating with detergent for five minutes followed by rinsing with deionized water. They were treated twice in boiling 1,1,1-trichloroethane for two minutes. The substrates were then sonicated twice with acetone for two minutes, twice with methanol for two minutes, and dried under $N_2$ flow.

The background pressure in the deposition system prior to device fabrication was normally $7x10^{-7}$ torr or lower and the pressure during film deposition was between $5x10^{-7}$ and $2x10^{-6}$ torr. The compounds used for construction of the OLEDs were evaporated from restively heated tantalum boats onto the substrate held close to room temperature. TPD was first deposited at a rate from 1 to 4 Å/s to give a 300 Å film followed by a layer of the electron transporting layer ($Alq_3$, $Al{>}_3$) deposited at a rate of 1 to 40 Å/s to a thickness of 450 Å. For dye doped OLEDs the electron transporting material and dye were co-deposited at the desired ratio The chamber was vented to air and shadow masks were put directly onto the substrates. Magnesium and silver were then co-deposited at a rate normally of 3 Å/s. The ratio of Mg: Ag varied from 7 : 1 to 12 : 1. The thickness of this layer was typically 500 Å. Finally, the devices were capped with 1000 Å Ag at a deposition rate between 1 to 4 Å/s.

Three types of OLEDs were fabricated having molecules having at least one electron transporting moiety and at least one hole transporting moiety:

Type SH-A: ITO \ TPD (300 Å) \ HET \ Mg-Ag (500 Å) \ Ag (1000 Å)
Type SH-B ITO \ HET \ $Alq_3$ (450 Å) \ Mg-Ag (500 Å) 1 Ag (1000 Å)
Type DH: ITO \ TPD (300 Å) \ HET \ $Alq_3$\ Mg-Ag (500 Å) \ Ag (1000 Å)

where HET represents a charge carrier layer that includes a compound having molecules having at least one electron transporting moiety and at least one hole transporting moiety, such as Al-pNP or Al-pCb. In addition, a TPD/$Alq_3$ OLED was typically fabricated at the same time as the OLEDs of type SH-A, SH-B and DH, for use as a reference. The devices were characterized in air within five hours of fabrication.

Experimental Results:

Each of the OLEDs fabricated had a heterostructure which had a charge carrier layer that included a compound having molecules having at least one electron transporting moiety and at least one hole transporting moiety, which was also an emissive material.

The absorption and photo-luminescent (PL) spectra of Al-pNP, Al-pCb, and Al-mCb in solution in $CH_2Cl_2$ are shown in Fig. B1. All three compounds show absorption at between 280 and 380 nm. The carbazole derivatives Al-pCb and Al-mCb show strong blue emission at around 360 nm, and Al-pCb has a lower energy emission at 480 nm. Al-pNP shows a single strong blue emission at around 440 nm which indicates the potential application of this material as a blue emitter.

In order to probe the properties of these new materials, different types of OLEDs were fabricated with the Al-pNP and Al-pCb. As described above, three types of OLEDs were fabricated for each material, type SH-A, SH-B and DH. Films of TPD and Al-pCb were also deposited onto a substrate. These films were pumped using ultraviolet radiation, and the absorption and PL emission were measured.

Fig. B2 shows the electroluminescent (EL) spectra of OLEDs of type SH-A, SH-B, and DH fabricated with Al-pNP, and the PL spectra of an OLED of type SH-A, Al-pNP in $CH_2Cl_2$, and a TPD film. Fig. B3 shows the I-V characteristics of OLEDs of type SH-A, SH-B and DH fabricated with Al-pNP, as well as a reference OLED having a TPD HTL and an $Alq_3$ ETL. Table B1 summarizes the yield, PL, and EL data for the OLEDs fabricated with Al-pNP. In Figs. B2 and B3 and Table B1, "SH-A" refers to a single heterostructure OLED having a TPD HTL and an Al-pNP ETL (300Å TPD/1000Å Al-pNP). "SH-B" refers to a single heterostructure OLED having an Al-pNP HTL and an $Alq_3$ ETL (300Å Al-pNP/450Å $Alq_3$). "DH" refers to a double heterostructure OLED having a TPD HTL, an Al-pNP seperate emissive layer, and an $Alq_3$ ETL (300Å TPD/200Å Al-pNP/450Å $Alq_3$). The "reference yield" row of Table B1 refers to the yield of a reference SH OLED with a TPD HTL and an $Alq_3$ ETL (300Å TPD/450Å $Alq_3$) fabricated at the same time as the OLED at the top of the column. The maximums reported in Table B1 are maximums in the visible range, and may be broad

maximums that do not correspond to the highest peak of Fig. B2.

As shown in Table B1, the SH-A OLED with Al-pNP is inefficient, having a low yield of 0.0005%, although the low yield may be explained to some extent by contamination, which is indicated by the low reference yield of the reference OLED The SH-A OLED with Al-pNP emits white light. The SH-B OLED with Al-pNP has a better yield of 0.02%. The DH OLED with Al-pNP has an even better yield of 0.05% and improved I-V characteristics, as illustrated in Fig. B3. These results indicate that Al-pNP has some hole-transporting properties but is a poor emitter in the solid.

TABLE 1

| Structure | SH-A | SH-B | DH |
|---|---|---|---|
| Yield (%) | 0.0005 | 0.02 | 0.05 |
| Yield (%) (reference) | 0.02 | 0.1 | 0.1 |
| PL (nm) | 565 | 519 | 518 |
| EL (nm) | 525 | 514 | 520 |

Fig. B4 shows the I-V characteristics of OLEDs of type SH-A, SH-B and DH(2) fabricated with Al-pCb, as well as a reference OLED having a TPD HTL and an $Alq_3$ ETL. Fig. B5 shows the EL spectra of an OLED of type DH(2) and a reference OLED, and the PL spectra of an OLED of type DH(2) and a film of Al-pCb. Table 82 summarizes yield, PL, and EL data for OLEDs fabricated with Al-pCb. In Figs. B4 and B5 and Table B2, "SH-A" refers to a single heterostructure OLED having a TPD HTL and an Al-pCb ETL (300Å TPD/500Å Al-pCb). "SH-B" refers to a single heterostructure OLED having an Al-pCb HTL and an $Alq_3$ ETL (300Å Al-pCb/450Å $Alq_3$). "DH(1)" refers to a double heterostructure OLED having a TPD HTL, an Al-pCb seperate emissive layer, and an $Alq_3$ ETL (300Å TPD/450Å Al-pCb/450Å $Alq_3$). "DH(2)" refers to a double heterostructure OLED having the same materials as DH(1), but deposited to different thicknesses (300Å TPD/410Å Al-pCb/40Å $Alq_3$). The "reference yield" row of Table B2 refers to the yield of a reference SH OLED with a TPD HTL and an $Alq_3$ ETL (300Å TPD/450Å $Alq_3$) fabricated at the same time as the OLED at the top of the column.

The application of Al-pCb in OLEDs of types SH-A, SH-B and DH led to results better than those for Al-pNP both in terms of color and efficiency. The PL spectra of the Al-pCb film, as shown in Fig. B5, is similar to that of Al-pCb in a $CH_2Cl_2$ solution, as shown in Fig. B1, having the same blue emission, and had an intensity comparable to that of $Alq_3$. As shown in Fig. B5 and Table B2, the DH(2) OLED with Al-pCb has a blue emission with a peak at about 500 nm.

TABLE B2

| Structure | SH-A | SH-B | OH (1) | DH (2) |
|---|---|---|---|---|
| Yield (%) | 0.03 | 0.001 | 0.11 | 0.02 |
| Yield (%) (reference) | 0.14 | 0.14 | 0.14 | 0.14 |
| PL (nm) | 480 | 509 | 495 | 480 |
| EL (nm) | 500 | 518 | 518 | 500 |

The efficiency and color purity of the DH(2) OLED with Al-pCb was further improved by the dye-doping technique. Fig. B6 shows the PL spectra of perylene in a $CH_2Cl_2$ solution and a DH(2) OLED with Al-pCb, uniformly doped with 1% perylene, and the EL spectrum of the same OLED. Fig. 87 shows the I-V characteristics of a DH(2) OLED with Al-pCb, uniformly doped with 1% perylene, and a TPD/$AIq_3$ OLED where the $Alq_3$ is doped with 1 % perylene. Table B3 shows the yield, PL and EL data for OLEDs fabricated with Al-pCb. In Figs. B6 and B7 and Table B3, "DH(2)" refers to a double heterostructure OLED having a TPD HTL, a layer of Al-pCb uniformly doped with 1% perylene as a seperate emissive layer, and a $Alq_3$ ETL (300Å TPD/400Å 1% perylene-Al-pCb/50Å $Alq_3$). The "reference yield" row of Table B3 refers to the yield of a reference SH OLED with a TPD HTL and an $Alq_3$ ETL (300Å TPD/450Å $Alq_3$) fabricated at the same time as the OLED at the top of the column.

TABLE B3

| Structure | DH(2) |
|---|---|
| Yield (%) | 0.05 |
| Yield (%) (reference) | 0.12 |

Table continued

| Structure | DH(2) |
|---|---|
| PL (nm) | 445 |
| EL (nm) | 455 |

When the Al-pCb layer was uniformly doped with 1 wt% perylene, the OLED had a more intense blue hue in its EL spectrum than when the Al-pCb layer was not doped. The $\lambda_{max}$ (the wavelength having the greatest intensity) of the EL spectra of the doped device was characteristic of the $\lambda_{max}$ of the PL spectra of perylene in the solution, as shown in Fig. B6.

The quantum efficiency of the doped device was 2.5 times higher than that of the undoped one (0.05%). The EL spectrum of the doped device still had some tail to the green part of a spectrum due to the incomplete quenching of the host emission. This is indicative of some inefficiency in the energy transfer processes from the host to the dopant molecules.

The present invention may also be used with other blue dopants, such as coumarine 1, or dopants that emit other colors, such as green (i.e. coumarine 6) or red (i.e., 1,3-bis(4-(dimethylamino)-2-hydroxyphenylj-2,4-dihydroxycyclobutenediylium dihydroxide).

C. Examples of OLEDs containing an emissive layer containing an azlactone-related dopant (outside of the invention)

The procedures that were used for fabrication of Organic Light-Emitting Devices (OLEDs) were as follows:

The hole transporting material TPD and the electron transporting material $Alq_3$ were synthesized according to known procedures, and were sublimed before use.

Compound 1: Hippuric acid (40.0 g, 0.22 mol), benzaldehyde (25.0 g, 0.24 mol), sodium acetate (16.0 g, 0.24 mol) and anhydrous acetic acid (120 ml, 1.2 mol) were mixed and well stirred for 3 days. The resulting yellow mixture was added to cold deionized water (1 L) and the yellow solid was collected by filtration. Yellow needles (48 g) were collected after crystallization from acetone (2 L). Yield: 88%. M.p.: 162-163°C. Anal. Cald.: C, 77.1; H, 4.45, N, 5.62. Found: 76.2, H, 4.47; N, 5.55. M.S.: 249 (P), 105 (PhCO), 77(Ph). NMR (25°C, CDCl3): 8.15 - 8.30 (m), 7.47 - 7.65 (m).

Compound 2: Hippuric acid (5.0 g, 27.9 mmol), p-hydroxybenzaldehyde (3.70 g,30.3 mmol), sodium acetate (2.0 g, 30 mmol) and anhydrous acetic acid (20 ml, 212 mmol) were mixed and well stirred for a day. The resulting yellow mixture was added to cold deionized water (0.2 L) and the yellow solid was collected by filtration. Yellow crystals were collected after crystallization from acetone. M.S.: 307 (P), 265 (P-CH3COO+H), 105 (PhCO), 77(Ph). NMR (25°C, CDCl3): 8.25 (d, 8.8 Hz).8.18 (d, 8.0 Hz), 7.50 - 7.66 (m), 7.20 - 7.26 (m), 2.34 (s, Me).

Compound 3: Hippuric acid (5.00 g, 27.9 mmol), p-dimethylaminobenzaldehyde (4.38 g, 29.4 mmol), sodium acetate (2.0 g, 30 mmol) and anhydrous acetic acid (20 ml, 212 mmol) were mixed and well stirred for a day. The resulting deep red mixture was added to cold deionized water (200 mL) and the red solid was collected by filtration. Red crystals (1.0 g) were collected after crystallization from acetone and sublimation (200°C). Yield: 12%. M.p.: 211-213°C. Anal. Cald.: C, 74.0; H, 5.52, N, 9.58. Found: 73.9, H, 5.51; N, 9.54. M.S.: 292 (P), 59 (P-PhCOCO), 105 (PhCO), 77(Ph). NMR (25°C, CDCl3): 8.15 (dd, 8.3 Hz, 1.8 Hz), 7.46 - 7.56 (m), 7.22 (s), 6.82 (d, 8.75 Hz), 3.12 (s, Me).

Compound 4: Hippuric acid (8.57 g, 47.8 mmol), p-tert-butylbenzaldehyde (8.0 mL, 47.8 mmol), sodium acetate (3.2 g,48.5 mmol) and anhydrous acetic acid (35 ml, 317 mmol) were mixed and well stirred for two days. The resulting yellow mixture was added to cold deionized water (150 ml) and the yellow solid was collected by filtration and purified by crystallization from acetone and sublimation (160°C). Yield: 88%. M.p.: 142°C. Anal. Cald.: C, 78.7; H, 6.27 N, 4.58 Found: 78.7, H, 6.25; N, 4.58. M.S.: 305 (P), 105 (PhCO), 77(Ph). NMR (25°C, $CDCl_3$): 8.13 - 8.20 (m). 7.50 - 7.64 (m),1.36 (s, Me).

Compound 5: Hippuric acid (0.45 g, 2.5 mmol), 4-dimethylamino-1-naphthaldehyde (0.50 g. 2.5 mmol), sodium acetate (0.26 g. 3.9 mmol) and anhydrous acetic acid (10 ml, 106 mmol) were mixed and well stirred for two days. The resulting red mixture was added to cold deionized water (100 ml) and a red oil was formed. Diethylether (30 ml) was used to extract out the red product. Solvent was then removed and the red liquid was run through a silica gel column with toluene. The deep red elusion was collected and a red liquid was obtain after the solvent was removed (0.52 g).

Compound 6: 4-nitrohippuric acid (2.25 g, 10.0 mmol),
p-dimethylaminobenzaldehyde (1.53 ml, 10.2 mmol), sodium acetate (0.75 g, 11.4 mmol) and anhydrous acetic acid (40 ml, 424 mmol) were mixed and well stirred for two days. The resulting deep red mixture was added to cold deionized water (150 ml) and the black solid was collected by filtration and purified by crystallization from acetone

and sublimation (190 C). M.S.: 337 (P), 159 (P-$NO_2$PhCOCO), NMR (25°C, CDCl3): 8.29 (q, 8.5 Hz), 8.12 (d, 8.0 Hz), 7.26(d, 11 Hz), 6.74 (d, 8.5 Hz), 3.12 (s, Me).

Compound 7: N-Acetylglycine (2.02 g, 17.3 mmol), p-dimethylaminobenzaldehyde (2.49 g, 16.7 mmol), sodium acetate (0.91 g,13.8 mmol) and anhydrous acetic acid (10 ml, 106 mmol) were mixed and well stirred for two days. The resulting deep red mixture was added to cold deionized water (150 ml) and the reddish solid was collected by filtration and purified by crystallization from acetone.

OLEDs were prepared using the following procedures:

The ITO/Borosilicate substrates (100Ω/square) were cleaned by sonicating with detergent for five minutes followed by rinsing with deionized water. They were then treated twice in boiling 1,1,1-trichloroethane for two minutes. The substrates were then sonicated twice with acetone for two minutes and twice with methanol for two minutes.

The background pressure prior to deposition was normally $7x10^{-7}$ torr or lower and the pressure during the deposition was around $5x10^{-7}$ to $1.1x10^{-6}$ torr.

All the chemicals were resistively heated in various tantalum boats. TPD was first deposited at a rate from one to four Å/s. The thickness was typically controlled at 300 Å.

The electron transporting layer $Alq_3$ was doped with Compound 3. Typically, the dopant was first vaporized with the substrates covered. After the rate of the dopant was stabilized, the host material was vaporized to the certain rate. The cover over the substrates was then opened and the host and guest were deposited at the desired concentration. The rate of dopant was normally 0.1 - 0.2 Å/s. The total thickness of this layer was controlled at 450 Å.

The substrates were then released to air and masks were put directly on the substrates. The masks were made of stainless steel sheet and contain holes with diameters of 0.25, 0.5, 0.75, and 1.0 mm. The substrates were then put back into vacuum for further coating.

Magnesium and silver were co-deposited at a rate normally of 2.6 Å/s. The ratio of Mg:Ag varied from 7:1 to 12:1. The thickness of this layer was typically 500 Å. Finally, 1000 Å Ag was deposited at the rate between one to four Å/s.

The devices were characterized within five hours of fabrication. Typically electroluminescent spectra, I-V curves, and quantum yields were measured from direct front.

The spectra of the doped device containing Compound 3 as the dopant and of an undoped $Alq_3$ device are shown in Fig. C1. The emission from this device comes from the Compound 3 dopant.

The I-V characteristics of the device shown in Fig. C2 show not only that the compound can be used in an OLED but also that such compounds are capable of provided enhanced current levels for a given voltage.

<u>D. Examples of OLEDs containing an emissive layer comprised of a phosphorescent dopant compound</u> (outside of the invention)

The procedures that were used for fabrication of Organic Light-Emitting Devices (OLEDs) were as follows:

The hole transporting material TPD and the electron transporting material $Alq_3$ were synthesized according to literature procedures, and were sublimed before use. The dopant PtOEP was purchased from Porphyrin Products, Inc., Logan, UT, and was used as received.

OLEDs were prepared using the following procedures:

The ITO/Borosilicate substrates (1000/square) were cleaned by sonicating with detergent for five minutes followed by rinsing with deionized water. They were then treated twice in boiling 1,1,1-trichloroethane for two minutes. The substrates were then sonicated twice with acetone for two minutes and twice with methanol for two minutes.

The background pressure prior to deposition was normally $7x10^{-7}$ torr or lower and the pressure during the deposition was around $5x10^{-7}$ to $1.1x10^{-6}$ torr.

All the chemicals were resistively heated in various tantalum boats. TPD was first deposited at a rate from one to four Å/s. The thickness was typically controlled at 300 Å.

The electron transporting layer $Alq_3$ was doped with PtOEP. Typically, the dopant was first vaporized with the substrates covered. After the rate of the dopant was stabilized, the host material was vaporized to the certain rate. The cover over the substrates was then opened and the host and guest were deposited at the desired concentration. The rate of dopant was normally 0.1 - 0.2 Å/s. The total thickness of this layer was controlled at about 450 Å.

The substrates were removed from the deposition system and masks were put directly on the substrates. The masks were made of stainless steel sheet and contain holes with diameters of 0.25, 0.5, 0.75 and 1.0 mm. The substrates were then put back into vacuum for further coating.

Magnesium and silver were co-deposited at a rate normally of 2.6 Å/s. The ratio of Mg:Ag varied from 7:1 to 12:1. The thickness of this layer was typically 500 Å. Finally, 1000 Å Ag was deposited at the rate between one to four Å/s.

The devices were characterized within five hours of fabrication. Typically electroluminescent spectra, I-V curves, and quantum yields were measured from direct front.

E. Examples of OLEDs containing an emissive layer comprised of a phosphorescent dopant compound with reduced symmetry (outside of the invention)

The hole transporting material TPD and the electron transporting material $Alq_3$ were synthesized according to literature procedures, and were sublimed before use. The dopant PtDPP compound and additional porphyrin compounds that may also be platinated were synthesized as described below.

OLEDs were prepared using the following procedures:

The ITO/Borosilicate substrates (100Ω/square) were cleaned by sonicating with detergent for five minutes followed by rinsing with deionized water. They were then treated twice in boiling 1,1,1-trichloroethane for two minutes. The substrates were then sonicated twice with acetone for two minutes and twice with methanol for two minutes.

The background pressure prior to deposition was normally $7x10^{-7}$ torr or lower and the pressure during the deposition was around $5x10^{-7}$ to $1.1x10^{-6}$ torr.

All the chemicals were resistively heated in various tantalum boats. TPD was first deposited at a rate from one to four A/s. The thickness was typically controlled at 300 Å.

The electron transporting layer $Alq_3$ was doped with the dopant compound, for example, PtDPP. Typically, the dopant was first vaporized with the substrates covered. After the rate of the dopant was stabilized, the host material was vaporized to at a certain rate. The cover over the substrates was then opened and the host and guest were deposited at the desired concentration. The rate of dopant was normally 0.1 - 0.2 Å/s. The total thickness of this layer was controlled at about 450 Å.

The substrates were removed from the deposition system and masks were put directly on the substrates. The masks were made of stainless steel sheet and contain holes with diameters of 0.25, 0.5, 0.75 and 1.0 mm. The substrates were then put back into vacuum for further coating.

Magnesium and silver were co-deposited at a rate normally of 2.6 Å/s. The ratio of Mg:Ag varied from 7:1 to 12:1. The thickness of this layer was typically 500 Å. Finally, 1000 Å Ag was deposited at the rate between one to four Å/s.

The devices were characterized within five hours of fabrication. Typically electroluminescent spectra, I-V curves, and quantum yields were measured from direct front.

Syntheses of Compounds and their characterization

2,2'-Dipyrrylmethane was prepared from a modified literature procedure (*Aust. J. Chem.,* 1969. 22, 229-249):

2,2'-Dipyrrylthione. To a vigorously stirred solution of 7.0 mL (90 mmol) of thiophosgene in 150 mL of dry THF at 0°C was added dropwise 12.5 g (186 mmol) of pyrrole. After 30 minutes, methanol (20 mL) was added and the mixture was further stirred for 30 minutes at room temperature. The resulting mixture was then evaporated to dryness. This crude material was used for the next step without further purification.

2,2'-Dipyrrylketone. To the aforementioned crude thione in 200 mL of 95% EtOH containing 10 g of KOH was added 17 mL of $H_2O_2$ (30%) at 0°C slowly. The mixture was stirred at 0°C for 2 hours and then at 60°C for 30 minutes after which was concentrated to 1/5 of its original volume. 100 mL of $H_2O$ was added and the precipitate was filtered, washed with cold EtOH and dried to give the product (5.6 g, 39% from thiophosgene) which was sufficiently pure for the next step.

2,2'-Dipyrrylmethane. To a solution of 3.3 g (20.1 mmol) of 2,2'-dipyrrylketone in 200 mL of 95% ethanol containing 3.3 mL of morpholine under $N_2$ at reflux was added $NaBH_4$ (1.7 g x 6) in several portions. 3 mL of $H_2O$ was added 10 minutes after each addition. The mixture was further refluxed for 2 hours after the last addition. 300 mL of $H_2O$ was then added and the mixture was extracted several times with $Et_2O$. The combined extract was dried with $MgSO_4$ and concentrated to give a thick oil which was extracted with hexane until the extract showed no appreciable amount of the product by TLC. The combined hexane portions were concentrated to give 2.3 g (78%) of pale yellow crystals as the product.

General synthesis of 5,15-*meso*-diphenylporphyrins:

2,2'-Dipyrrylmethane (0.5 g, 3.42 mmol) and an equimolar amount of the aldehyde were dissolved in 500 mL of dry $CH_2Cl_2$. The solution was purged with $N_2$ for 15 minutes. Trifluoroacetic acid (154 µL. 2.6 mmol) was then added via syringe and the mixture was stirred for 3 hours under $N_2$ in the absence of light. 2,3-Dichloro-5,6-dicyanoquinone (1.04 g, 4.6 mmol) was added and stirring was continued for 30 minutes. The resulting dark solution was concentrated to 1/3 of its original volume and poured onto a column of silica gel packed with hexane. Elution with $CH_2Cl_2$ afforded a purple band which was concentrated to give purple solids that were filtered and washed with ethanol followed by heaxane. The solids were pure enough for photoluminescence measurements.

5,15-*memo*-diphenylporphyrin ($H_2DPP$): yield: 0.63 g., 80%, MS (EI) m/z (relative intensity) 462 ($M^+$, 100), 386 (50), 368 (30), 313 (25), 231 (50). 5,15-*memo*-bis(pentafluorophenyl)porphyrin ($H_2BPFPP$): yield: 0.05 g, 5%, MS (EI),

m/z (relative intensity) 642 ($M^+$, 100), 623 (8), 602 (8), 368 (45), 321 (35), 236 (40). 5,15-memo-bis(4-cyanophenyl)porphyrin ($H_2$BCPP): yield: 0.09 g, 10%, MS (EI), m/z (relative intensity) 512 ($M^+$, 100), 411 (50), 368 (35), 355 (40), 294 (45), 281 (50). 5,15-memo-bis(4-anisyl)porphyrin ($H_2$BAP): yield: 0.09 g, 10%, MS (EI) m/z (relative intensity) 522 ($M^+$, 8), 416 (100), 401 (20), 372 (23).

Platinum (II) 5,15-*memo*-diphenylporphyrin (PtDPP). A mixture of $H_2$DPP (0.05 g, 0.11 mmol) and $Pt(PhCN)_2Cl_2$ (0.1 g, 0.22 mmol) in 10 mL of dry toluene was refluxed for 24 hours under $N_2$. The resulting solution was dried completely under vacuum to remove traces of PhCN. The dark solid was dissolved in $CH_2Cl_2$ and chromatographed using hexane : $CH_2Cl_2$ (1:1, v/v) as the eluent to give red solids as the product (0.04 g, 56%). $^1$H NMR: δ 10.15 (s,2H), 9.207 (dd, 4H, $J_1$ = 12 Hz, $J_2$ = 7.5 Hz), 8.93 (dd, 4H, $J_1$ = 12 Hz, $J_2$ =7.5 Hz), 8.18 (m, 4H), 7.77 (m, 6H). %, MS (EI) m/z (relative intensity) 655 ($M^+$, 100), 577 (30), 326 (50), 288 (35).

F. Examples of OLEDs comprised of a hole transporting layer containing a glassy organic hole transporting material comprised of a compound having a symmetric molecular structure (outside of the invention)

Procedures for Fabrication of Organic Light-Emitting Devices (OLEDs): Chemicals:

ISB was prepared according to the following procedure: A round bottom flask was charged with Na-*t*-butoxide (4.25g), $Pd_2dba_3$ (0.22g), DPPF (diphenylphosphinoferrocene 0.33g), and 50ml anhydrous toluene. The reaction mixture was stirred under argon at 90°C for 15 minutes. Then dibromobiphenyl (3.12g) and iminostilbene (4.25g) were added, and the reaction was stirred for 8 hours until the amine could not be detected by mass spectroscopy. The solvent was then stripped from the reaction mixture and the crude residue was dried under vacuum. The dried residue was then subjected to a gradient sublimation under reduced pressure ($10^{-4}$ torr). The sublimation yielded 2.06g of pure material. 36% of the theoretical reaction yield.

IDB was prepared according to the following procedure: 51 mmol (10.000 g) iminodibenzyl was reacted with 17 mmol (6.94 g) 4,4'-diiodobiphenyl. The reaction product was added to a round bottom flask fitted with a condenser, along with 34 mmol (2.16 g) copper powder, 68 mmol (9.398 g) potassium carbonate, 2 mmol (0.530 g) 18-crown-6 ether, and 20 ml o-dichlorobenzene. The flask was heated to 185° C and then refluxed under argon for 24 hours. The reaction mixture was filtered hot and the filtrate was put under vacuum to remove the solvent. The residue was then passed through a short column of silica gel in toluene. The solvent was then removed from the column filtrate and the solid left behind was sublimed at 220°C under a vacuum of 0.01 Torr. for purification.

The electron transporting material $Alq_3$, as well as the TPD and NPD, were synthesized according to literature procedure. All organic materials were sublimed before use.

Procedures:

ITO/Borosilicate substrates (100Ω/square) were cleaned by sonicating with detergent for five minutes followed by rinsing with deionized water. They were then treated twice in boiling 1,1,1-trichloroethane for two minutes. The substrates were then sonicated twice with acetone for two minutes and twice with methanol for two minutes.

The background pressure prior to deposition was $8x10^{-7}$ torr and the pressure during the deposition was around $5x10^{-7}$ to $2x10^{-6}$ torr.

The chemicals were sublimed from resistively heated tantalum boats, and then deposited at a rate from 1 to 3.6 Å/s. The thickness was controlled at 300 Å.

The electron transporting layer ($Alq_3$) was deposited at a rate between 1 to 3.3 Å/s. The total thickness of this layer was controlled at 450 Å.

The substrates were then released to air and masks were put directly on the substrates. The masks are made of stainless steel sheet and contain holes with diameters of 0.25, 0.5, 0.75, and 1.0 mm. The substrates were then put back into vacuum for further coating.

Magnesium and silver were co-deposited at a rate of 2 Å/s The ratio of Mg : Ag was 9:1. The thickness of this layer was 500 Å. Finally, 1000 Å Ag was deposited at the rate of 2.7 Å/s.

Characteristics of the Devices:

The devices were characterized within one day of fabrication. I-V curves, quantum yields, and luminance were measured. OLED data derived from these measured quantities are tabulated in Table F2. The current-voltage (I-V) characteristics for the devices are shown in Figs. F1 and F2.

Fig. F1 shows the current-voltage characteristics of OLEDs having a single heterostructure comprising: an ITO anode, an HTL, an $Alq_3$ ETL, and an Mg-Ag cathode, deposited sequentially on a substrate, as discussed above. Four different plots are shown for four different HTL materials: TPD, NPD, ISB and IDB. The current-voltage plots for TPD,

NPD and ISB are very similar, showing that the current-voltage characteristics of the OLED do not significantly change, regardless of whether the HTL is TPD, NPD or ISB. The current -voltage plot for IDB shows a lower current than the plots for TPD, NPD and ISB, indicating that IDB may be a less desirable HTL from the perspective of current-voltage characteristics.

Fig. F2 shows the current-voltage characteristics of OLEDs having a single heterostructure with a hole injection enhancement layer, comprising: an ITO anode, a CuPc hole injection enhancement layer, an HTL, an $Alq_3$ ETL, and an Mg-Ag cathode, deposited sequentially on a substrate, as discussed above. Four different plots are shown for four different HTL materials: TPD, NPD, ISB and IDB. The current-voltage plots for TDP, NPD and ISB are very similar, showing that the current-voltage characteristics of the OLED do not significantly change, regardless of whether the HTL is TPD, NPD or ISB. The current -voltage plot for IDB shows a lower current than the plots for TPD, NPD and ISB, indicating that IDB may be a less desirable HTL from the perspective of current-voltage characteristics.

Figs. F1 and F2 show that OLEDs using ISB as the HTL can have current-voltage characteristics similar to those of OLEDs using TPD or NPD as the HTL. for two different OLED configurations. This similarity, in conjunction with the higher $T_g$ of ISB and the longer expected lifetimes of OLEDs using ISB, indicate that ISB is a superior HTL.

## Claims

1. A method of fabricating a cathode of an optoelectronic device, comprising sputter depositing an electrically conductive oxide layer on a semi-conductive organic layer, wherein the semi-conductive organic layer is comprised of a polyacene compound, of a phthalocyanine, of a copper phthalocyanine or of a zinc phthalocyanine, whereby an interface region is formed at the surface of the semi-conductive organic layer that lowers the voltage drop across the two layers.

2. The method according to claim 1, wherein the electrically conductive oxide layer is comprised of a wide band gap semiconductor having a band gap of at least 1 eV.

3. The method according to claim 1 or 2, wherein the wide band gap semiconductor has a transmission of at least 50% for incident and admitted radiation.

4. The method according to any of claims 1 to 3, wherein the electrically conductive oxide layer is an indium-tin-oxide layer.

5. A method of fabricating an organic light emitting device comprising:

   preparing a heterostructure for producing electroluminescence and **characterised in that** the cathode is fabricated according to the method of claims 1-4.

## Patentansprüche

1. Verfahren zur Herstellung einer Kathode einer optoelektronischen Vorrichtung, das die Sputterabscheidung einer elektrisch leitfähigen Oxidschicht auf eine organische Halbleiterschicht umfasst, wobei die organische Halbleiterschicht aus einer Polyacenverbindung, einem Phthalocyanin, einem Kupferphthalocyanin oder einem Zinkphthalocyanin besteht, wodurch an der Oberfläche der organischen Halbleiterschicht ein Grenzflächenbereich ausgebildet wird, der den Spannungsabfall über die beiden Schichten verringert.

2. Verfahren nach Anspruch 1, wobei die elektrisch leitfähige Oxidschicht aus einem Halbleiter mit großer Bandlücke besteht, der eine Bandlücke von mindestens 1 eV aufweist.

3. Verfahren nach Anspruch 1 oder 2, wobei der Halbleiter mit großer Bandlücke eine Transmission von mindestens 50 % für auftreffende und durchgelassene Strahlung aufweist.

4. Verfahren nach einem der Ansprüche 1 bis 3, wobei die elektrisch leitfähige Oxidschicht eine Indiumzinnoxid-Schicht ist.

5. Verfahren zur Herstellung einer organischen Licht-emittierenden Vorrichtung, das die Herstellung einer Heterostruktur für die Erzeugung von Elektrolumineszenz umfasst und das **dadurch gekennzeichnet ist, dass** die Kathode nach dem Verfahren nach einem der Ansprüche 1-4 hergestellt wird.

## Revendications

1. Une méthode de fabrication d'une cathode pour un mécanisme optoélectronique, comprenant une pulvérisation déposant une couche d'oxyde électriquement conductrice sur une couche organique semi-conductrice, dans laquelle la couche organique semi-conductrice est constituée de polyacène, de phtalocyanine, de phtalocyanine de cuivre ou de phtalocyanine de zinc, au moyen de laquelle une zone d'interface est formée sur la surface de la couche organique semi-conductrice qui réduit la chute de tension à travers les deux couches.

2. La méthode selon la revendication 1, dans laquelle la couche d'oxyde électriquement conductrice est constituée d'un semiconducteur à large bande interdite possédant une bande interdite d'au moins 1 eV.

3. La méthode selon la revendication 1 ou 2, dans laquelle le semiconducteur à large bande interdite possède une transmission d'au moins 50 % en ce qui concerne le rayonnement incident et le rayonnement admis.

4. La méthode selon l'une des revendications 1 à 3, dans laquelle la couche d'oxyde électriquement conductrice est une couche d'oxyde d'étain et d'indium.

5. Une méthode de fabrication d'un mécanisme électroluminescent organique comprenant :

   la préparation d'une hétérostructure permettant de produire de l'éléctroluminescence et **caractérisée en ce que** la cathode est fabriquée selon la méthode des revendications 1-4.

Figure A1

Prior Art Standard OLED

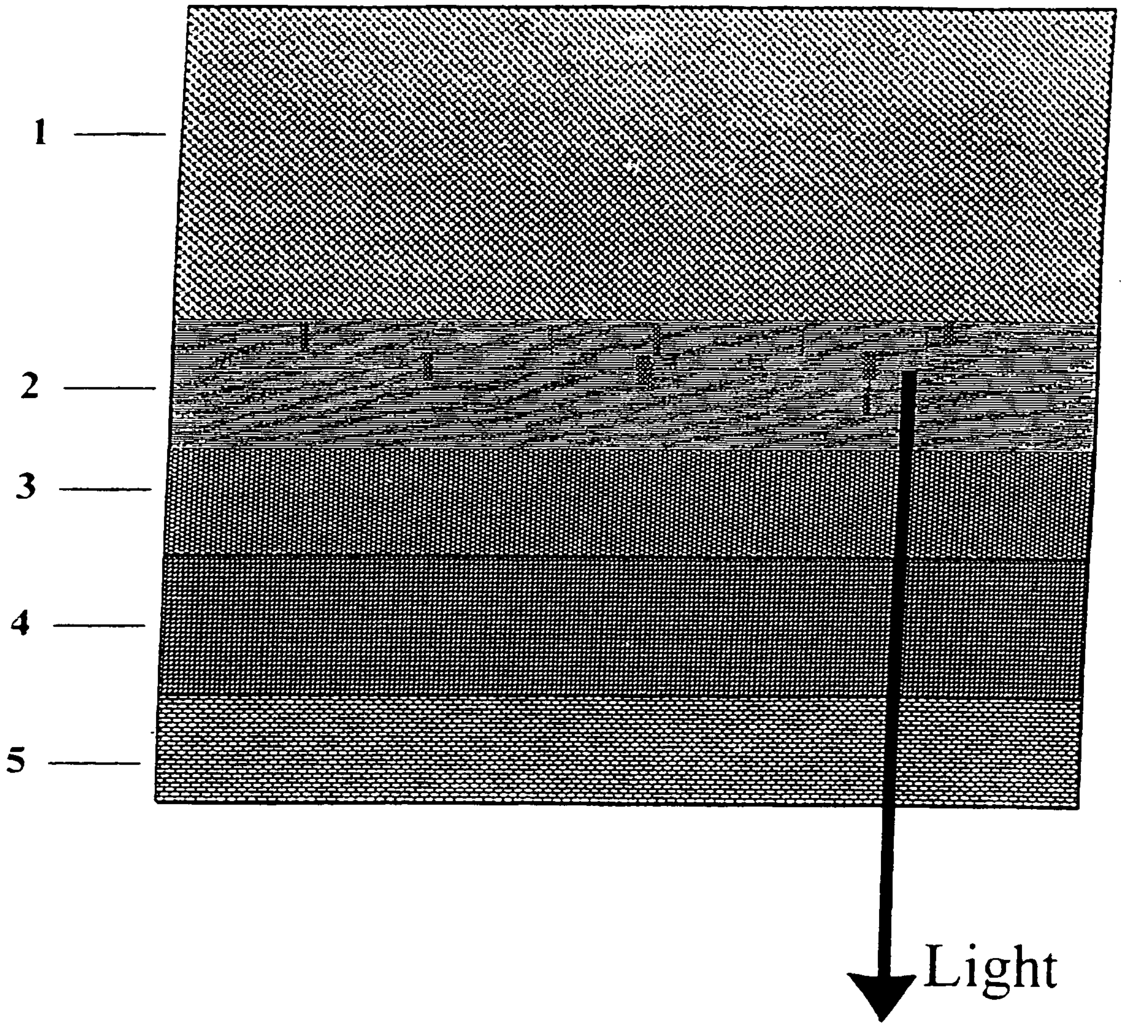

Figure A2a

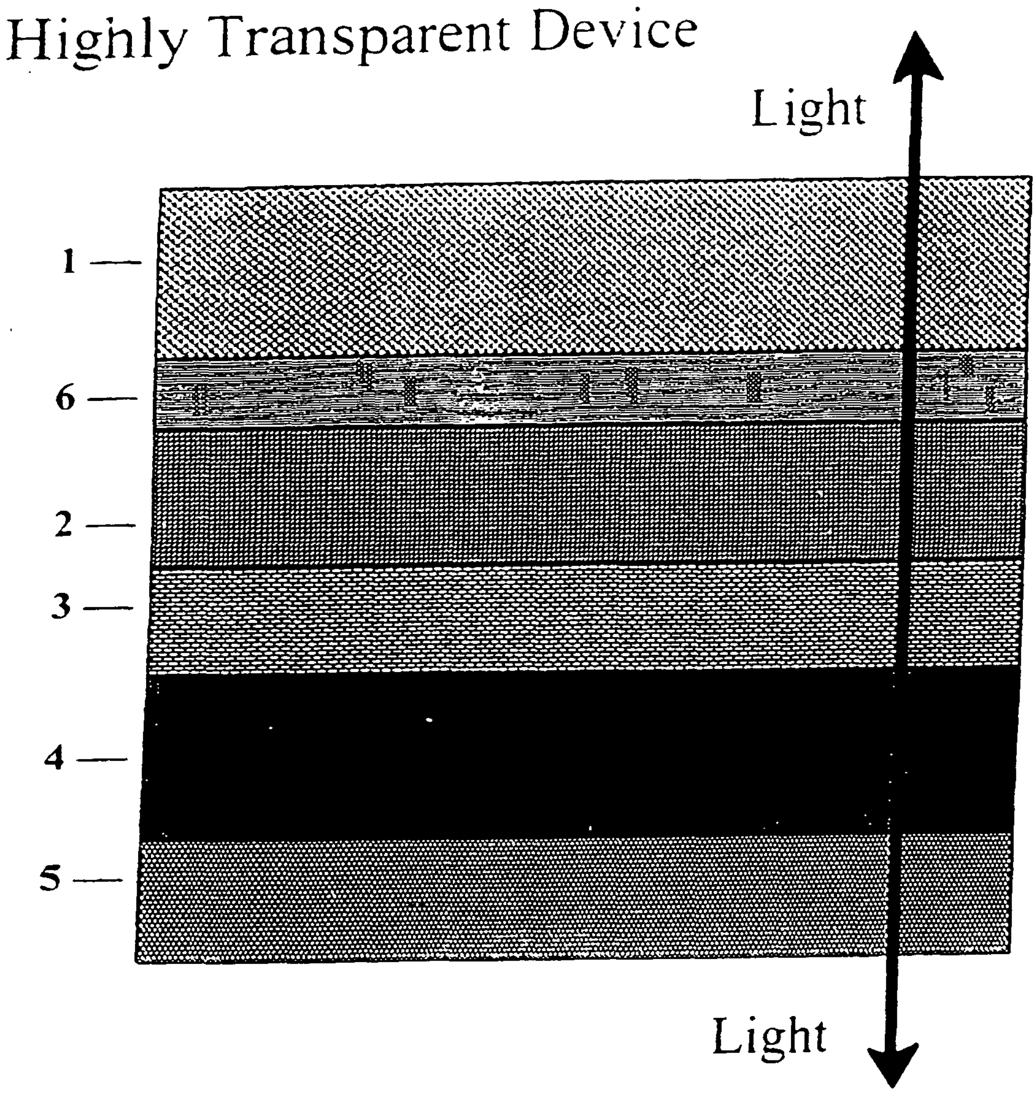

Figure A2b

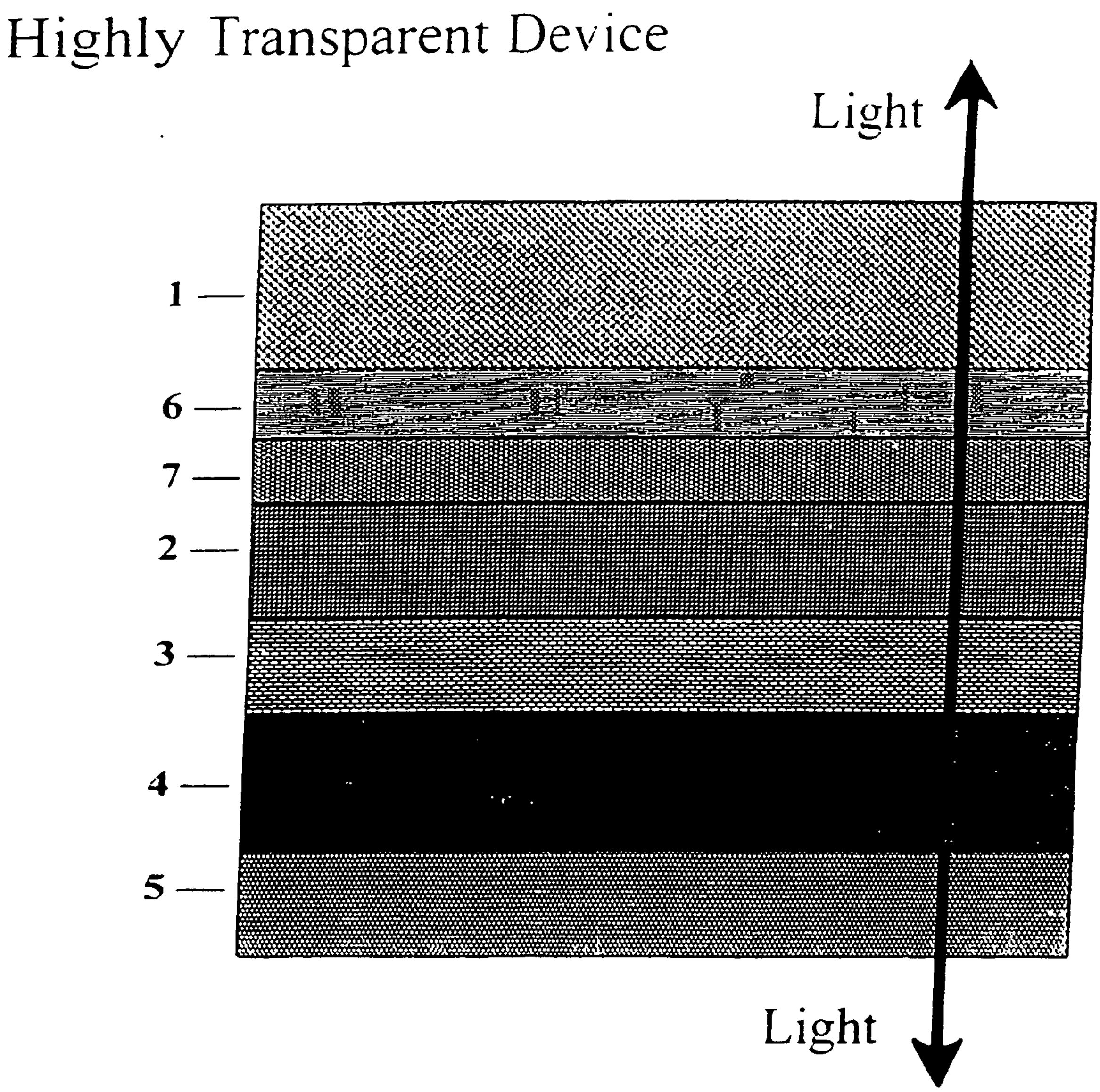

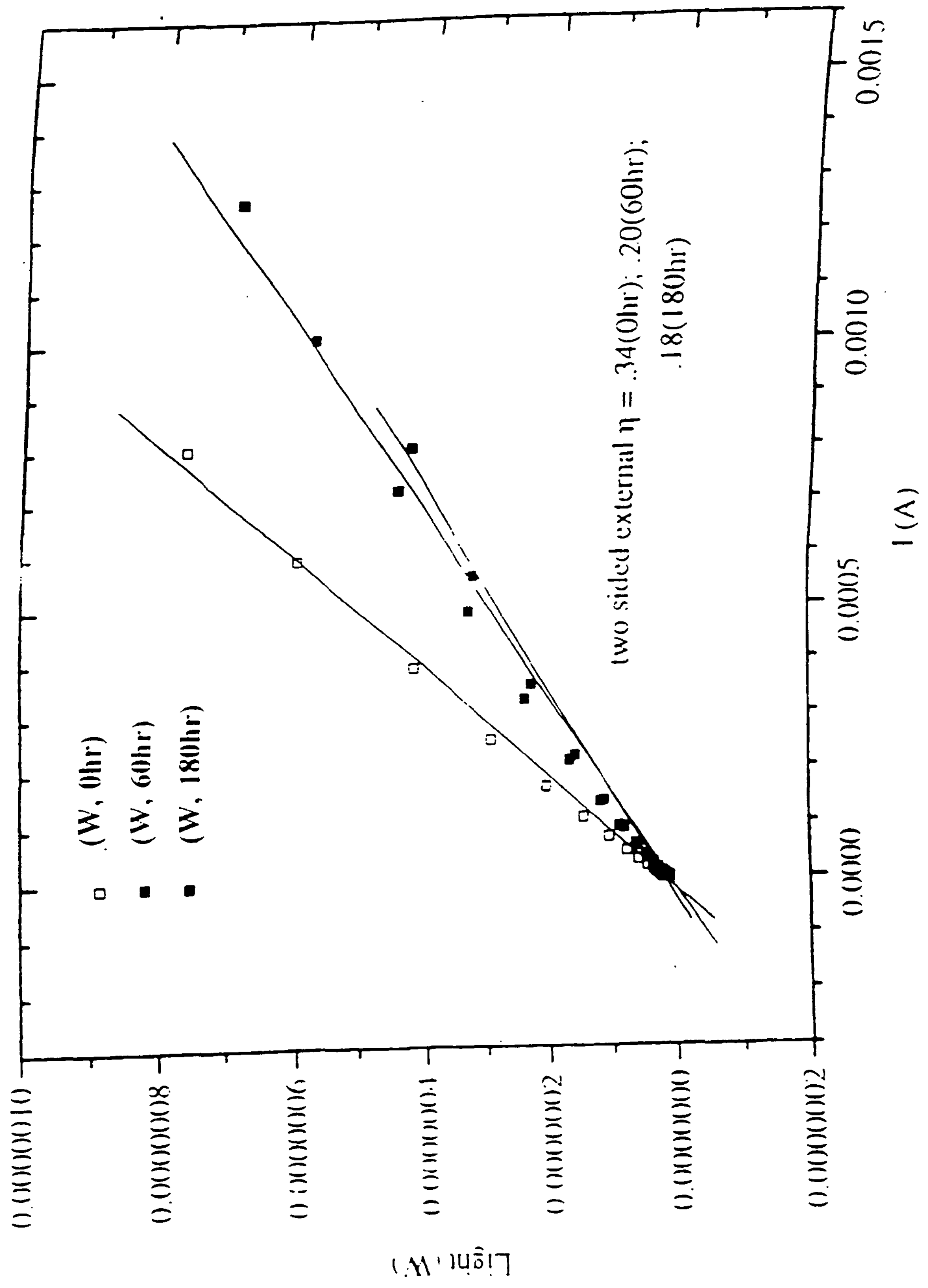
(W, 0hr)
(W, 60hr)
(W, 180hr)
two sided external η = .34(0hr); .20(60hr); .18(180hr)
I (A)
Light (W)
0.0000
0.0005
0.0010
0.0015
0.0000010
0.0000008
0.0000006
0.0000004
0.0000002
0.0000000
0.0000002

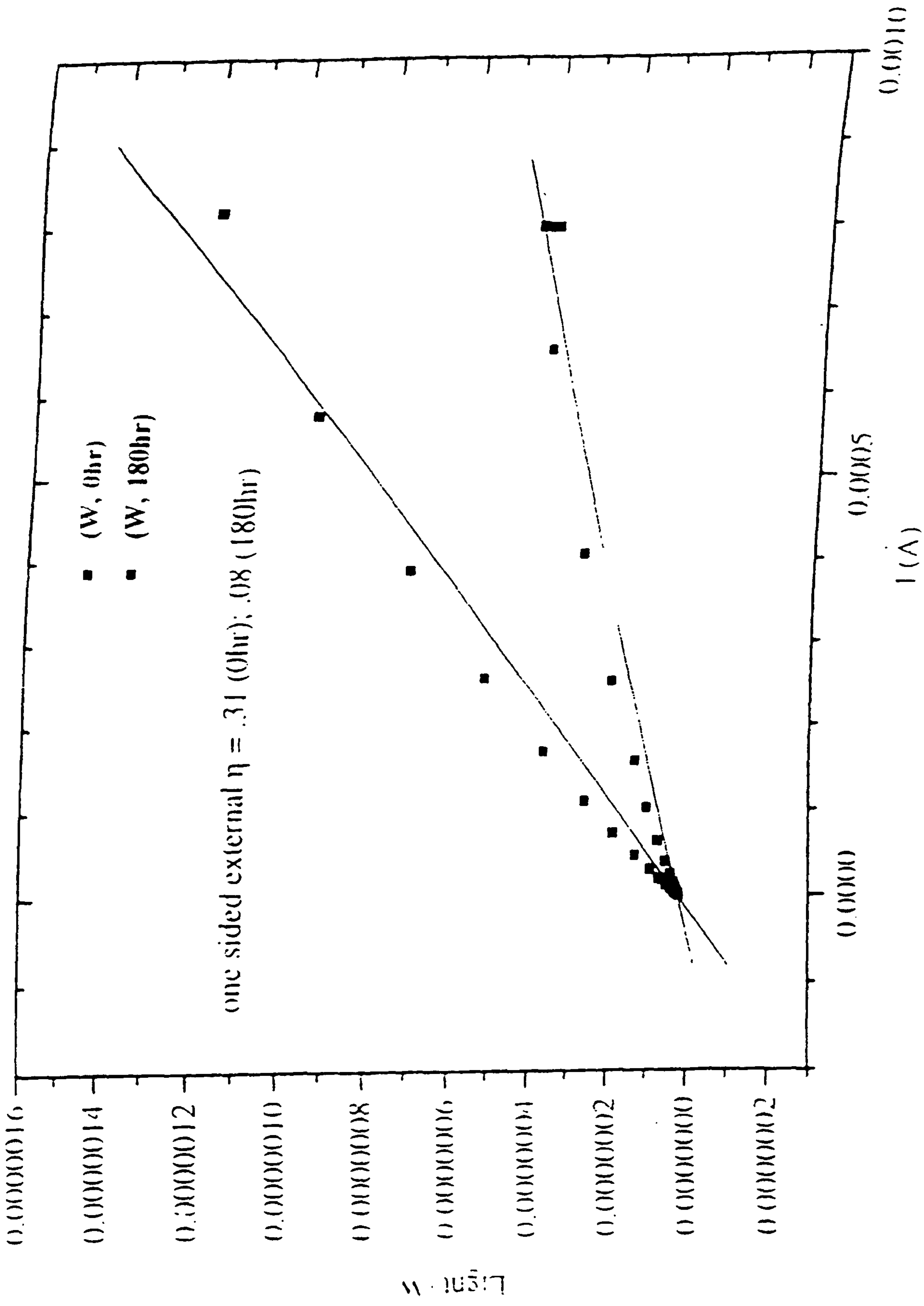
(W, 0hr)
(W, 180hr)
one sided external η = .31 (0hr); .08 (180hr)
I (A)
Light W
0.0000
0.0005
0.0010
0.0000016
0.0000014
0.0000012
0.0000010
0.0000008
0.0000006
0.0000004
0.0000002
0.0000000
-0.0000002

Figure A5

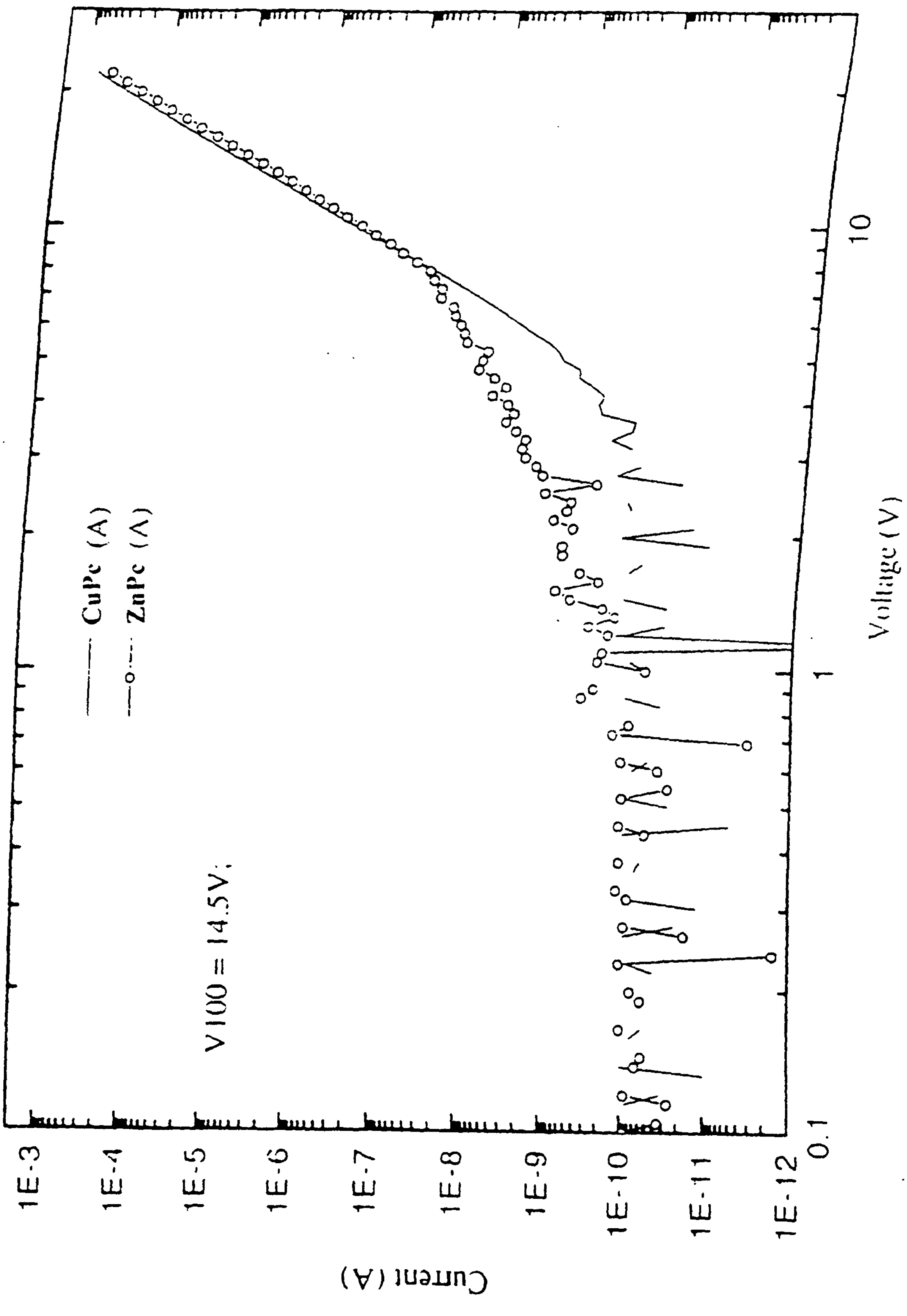

CuPc (A)
ZnPc (A)
V100 = 14.5V
Voltage (V)
Current (A)
0.1
1
10
1E-3
1E-4
1E-5
1E-6
1E-7
1E-8
1E-9
1E-10
1E-11
1E-12

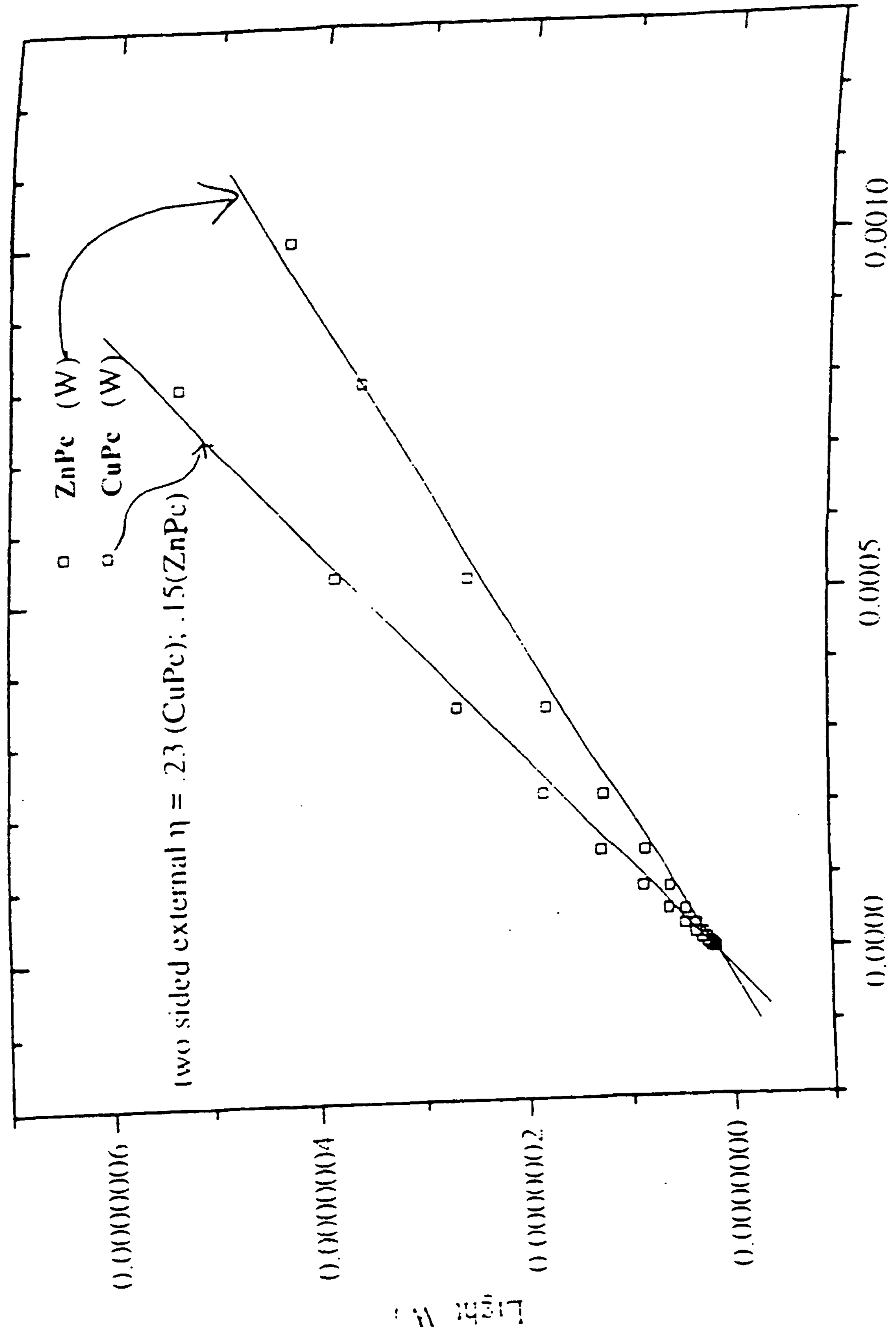

ZnPc (W)
CuPc (W)
two sided external η = .23 (CuPc); .15(ZnPc)
I (A)
0.0000
0.0005
0.0010
Light (W)
0.0000000
0.0000002
0.0000004
0.0000006

Figure A7

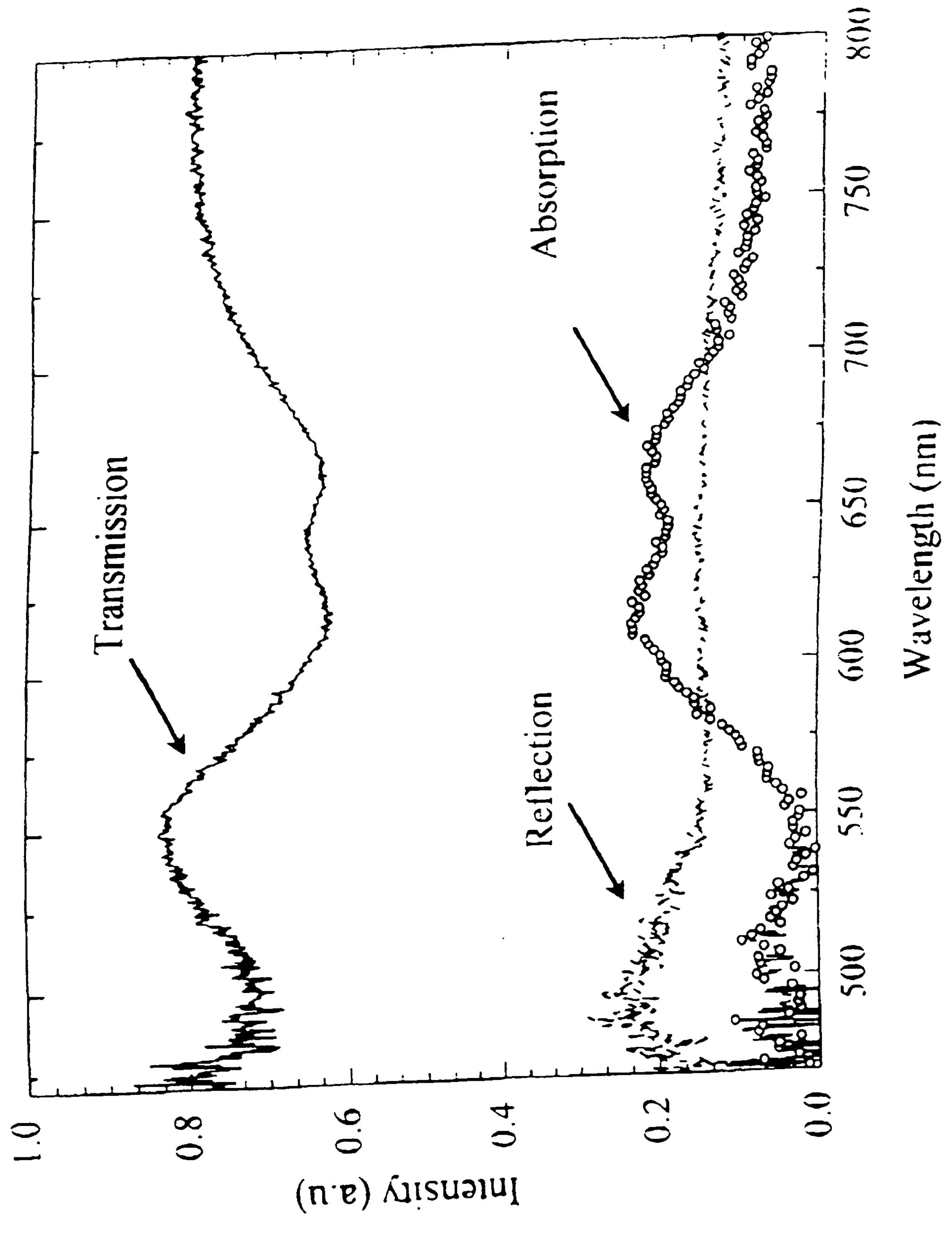
Transmission
Reflection
Absorption
Wavelength (nm)
Intensity (a.u)
1.0
0.8
0.6
0.4
0.2
0.0
500
550
600
650
700
750
800

Figure A8

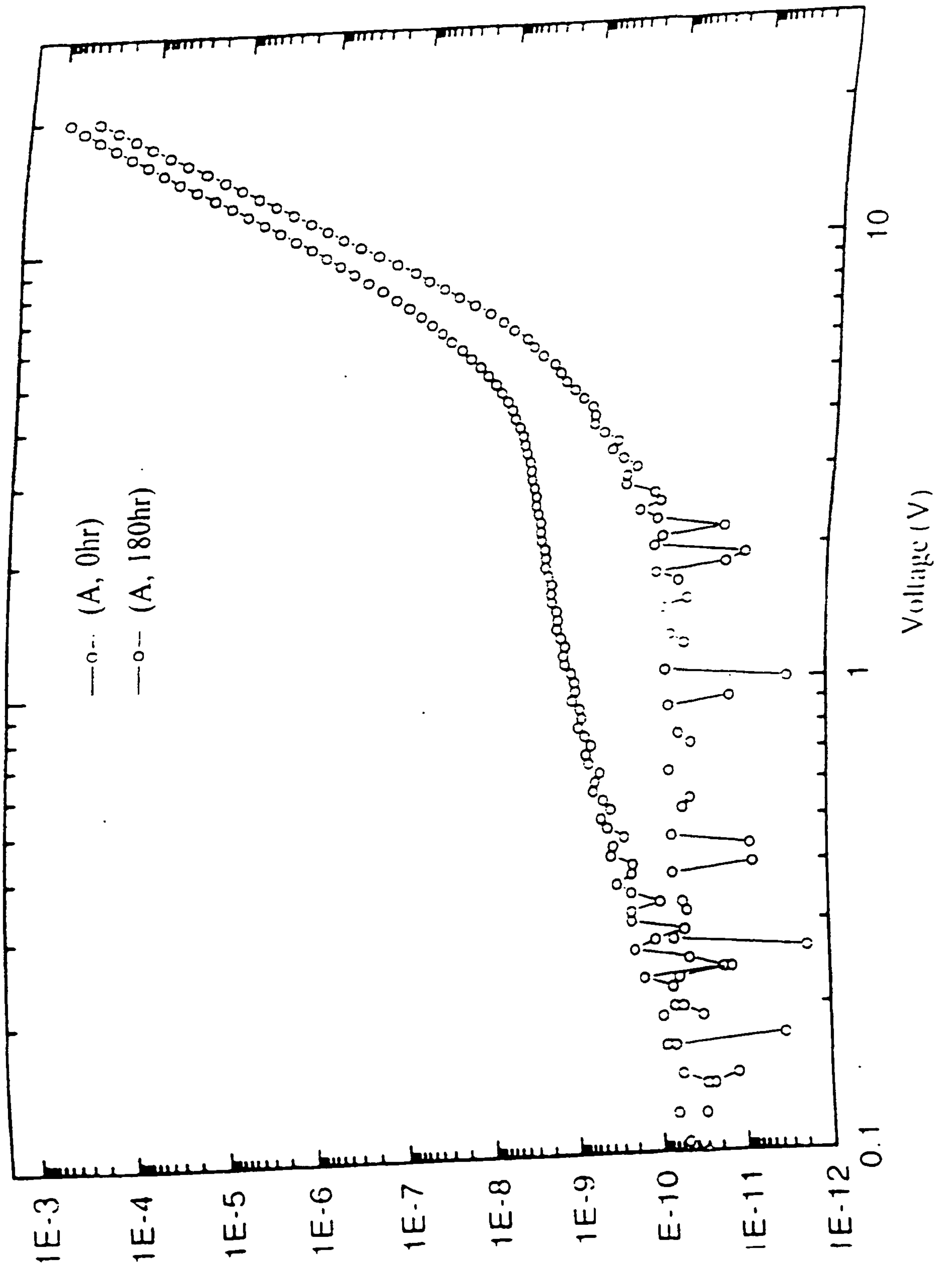
(A, 0hr)
(A, 180hr)
Voltage (V)
0.1
1
10
1E-3
1E-4
1E-5
1E-6
1E-7
1E-8
1E-9
E-10
1E-11
1E-12

Figure A9

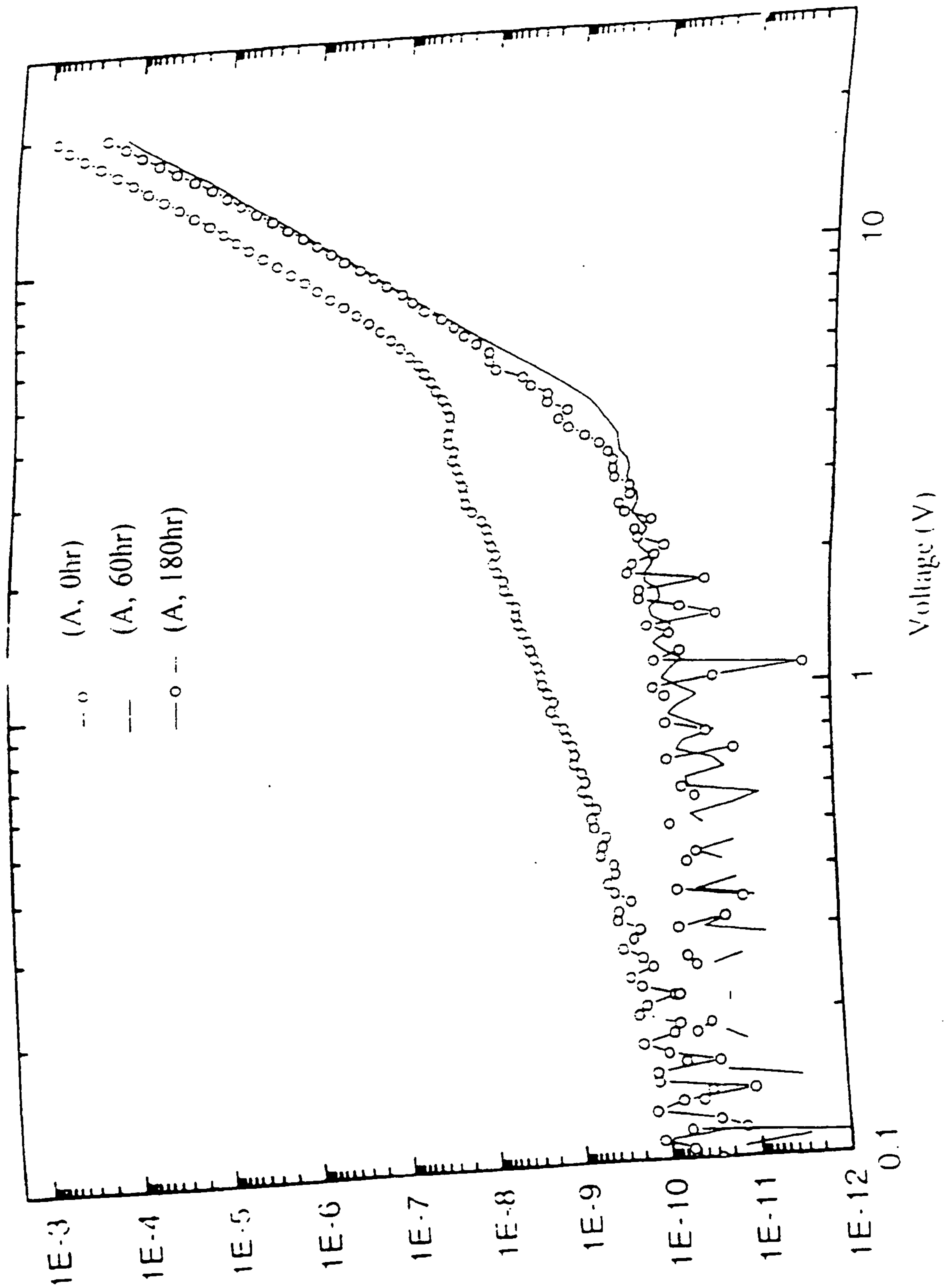
(A, 0hr)
(A, 60hr)
(A, 180hr)
Voltage (V)
0.1
1
10
1E-3
1E-4
1E-5
1E-6
1E-7
1E-8
1E-9
1E-10
1E-11
1E-12

Figure A10

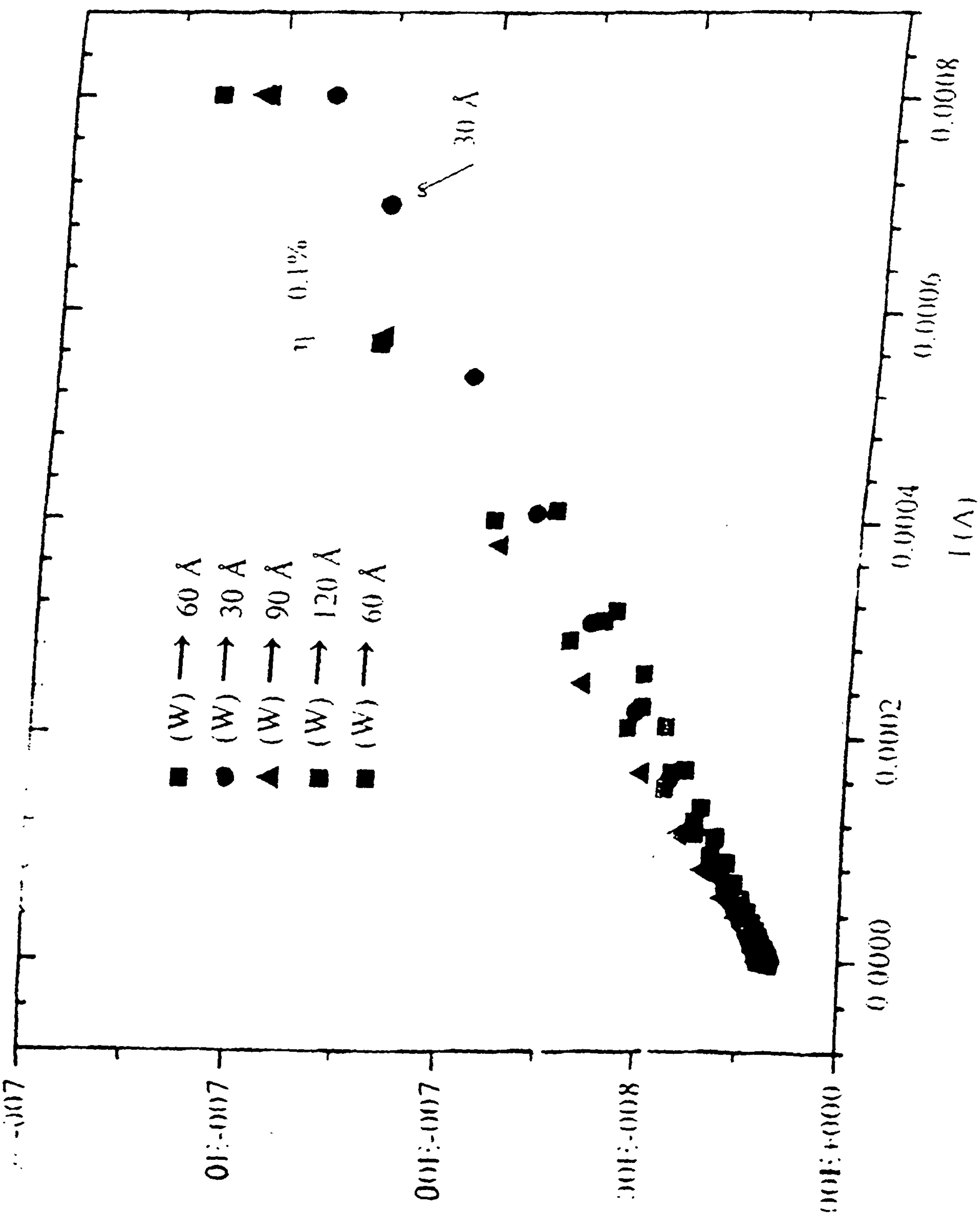
(W) → 60 Å
(W) → 30 Å
(W) → 90 Å
(W) → 120 Å
(W) → 60 Å
η 0.1%
30 Å
0.0000
0.0002
0.0004
0.0006
0.0008
I (A)

Figure A11

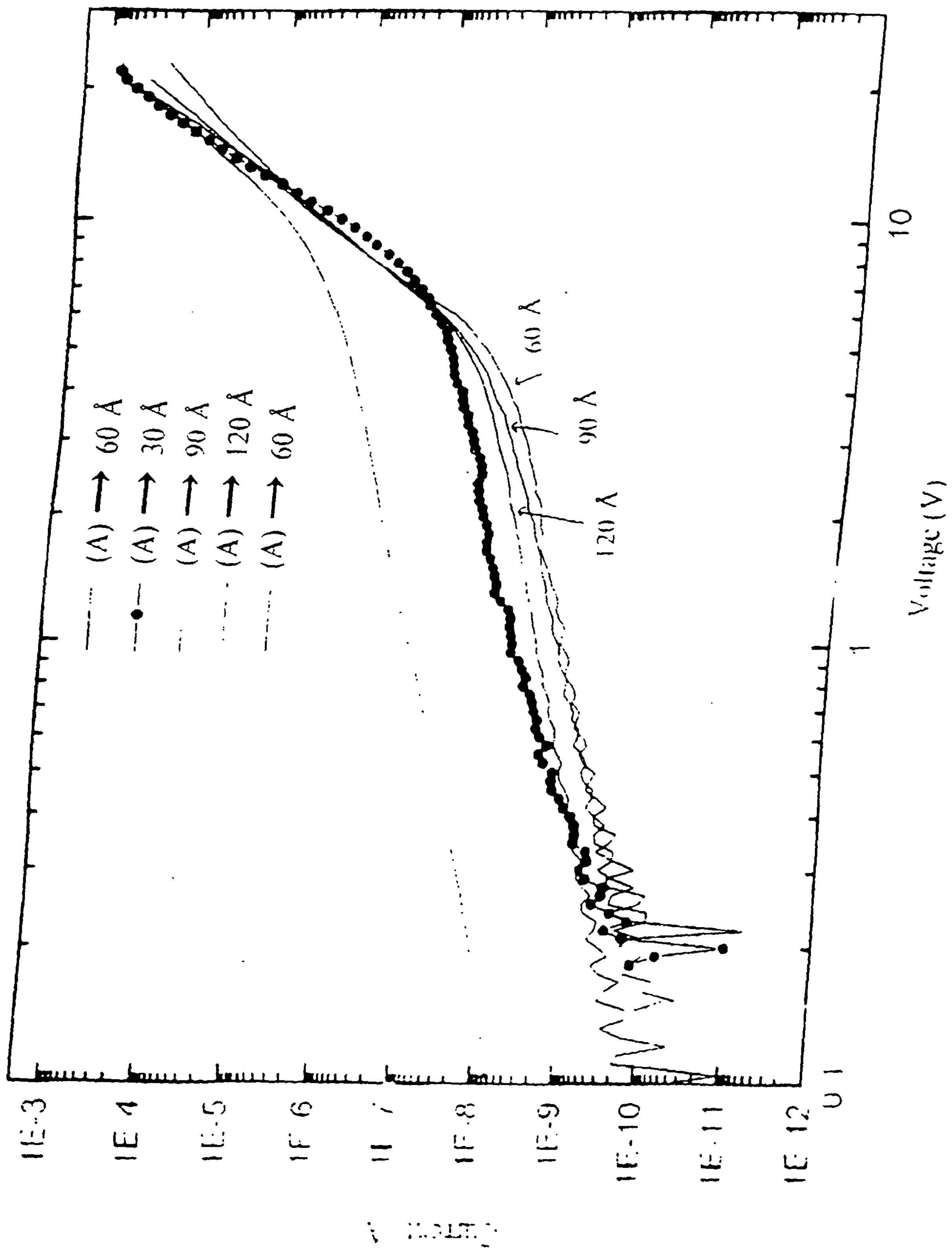
(A) → 60 Å
(A) → 30 Å
(A) → 90 Å
(A) → 120 Å
(A) → 60 Å
60 Å
90 Å
120 Å
Voltage (V)
0.1
1
10
1E-3
1E-4
1E-5
1E-6
1E-7
1E-8
1E-9
1E-10
1E-11
1E-12

Figure A12

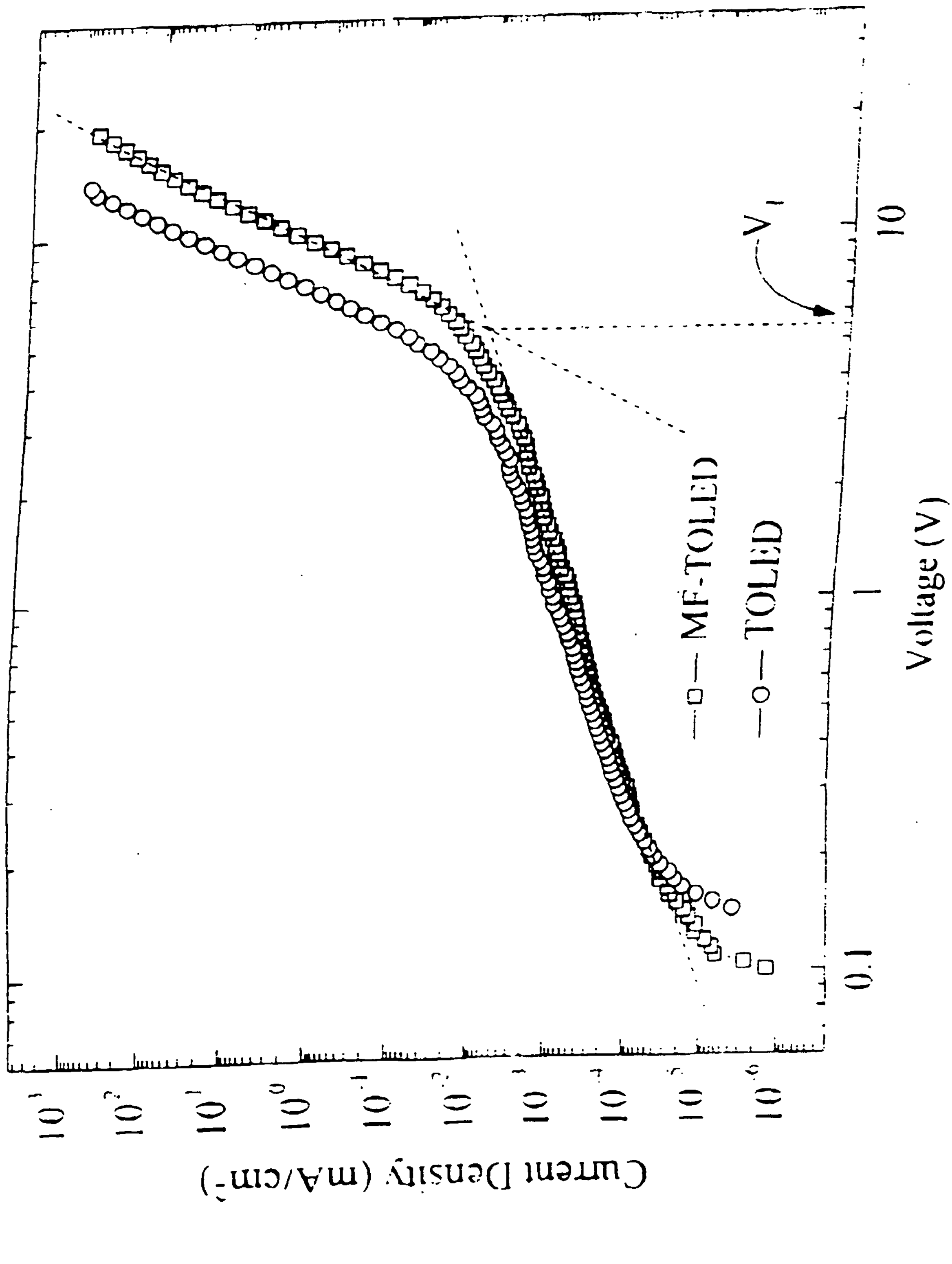

Figure A13

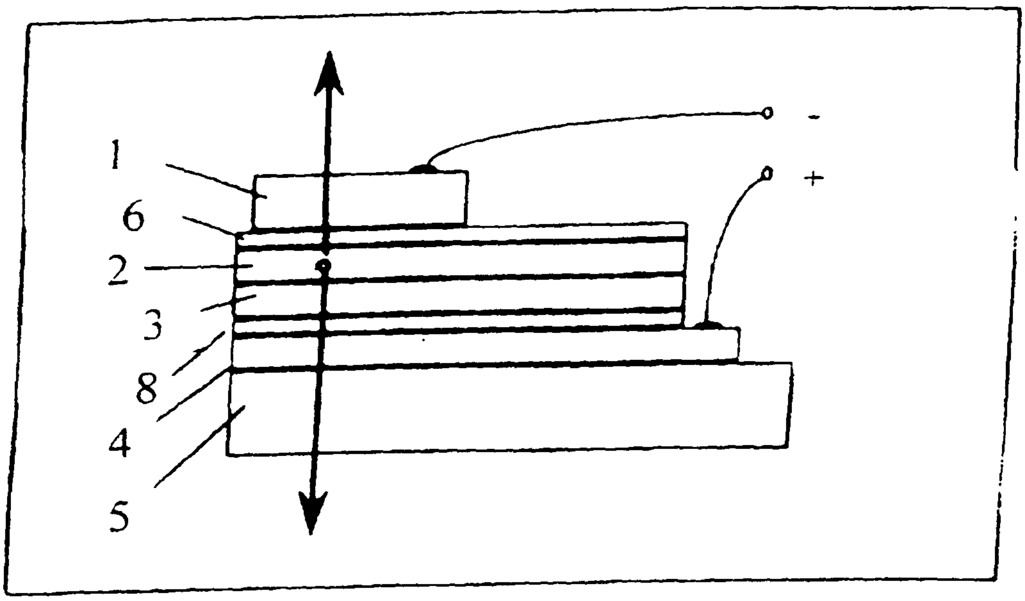

Figure A14

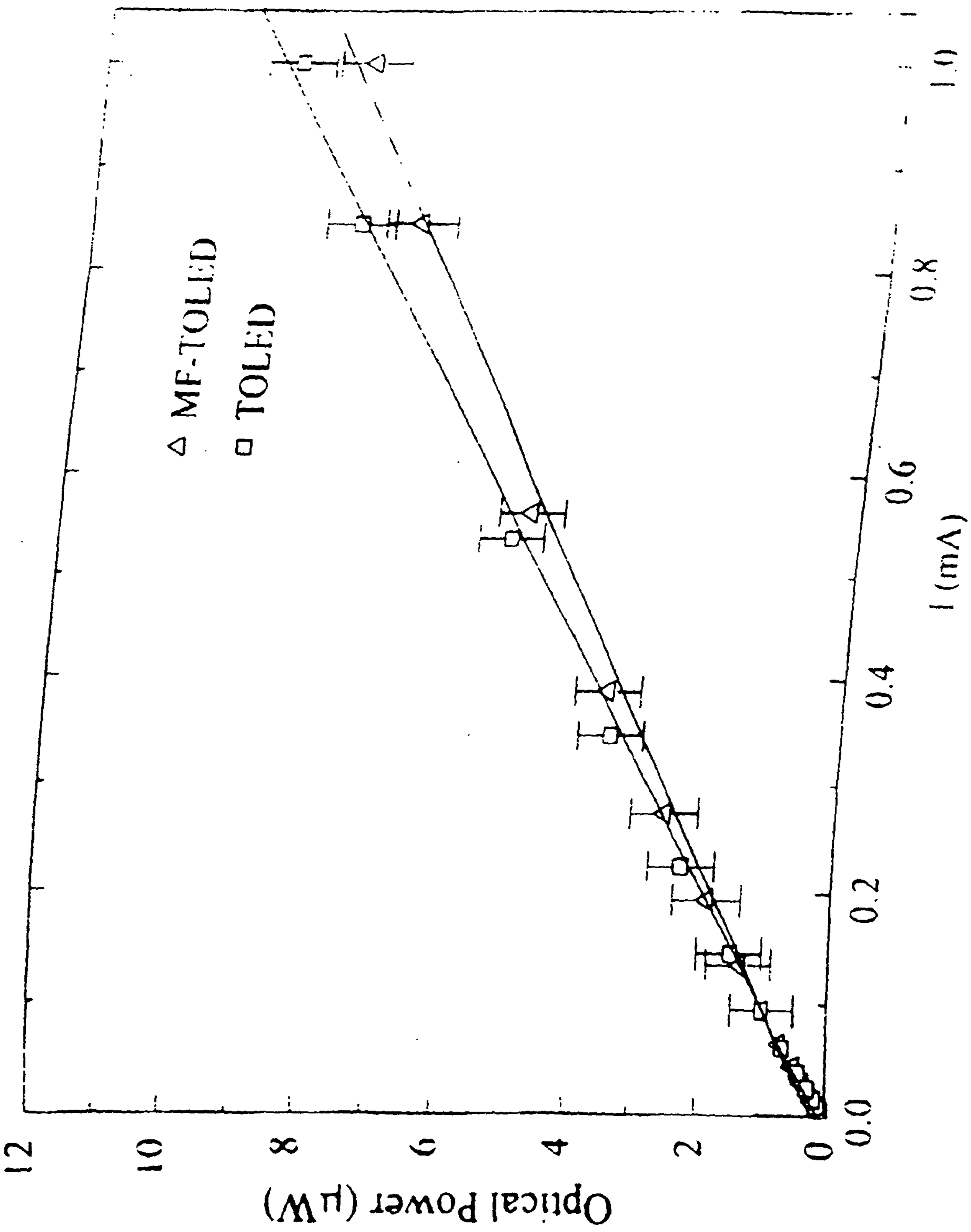

Optical Power (μW)
I (mA)
MF-TOLED
TOLED
0
2
4
6
8
10
12
0.0
0.2
0.4
0.6
0.8
1.0

Figure A15

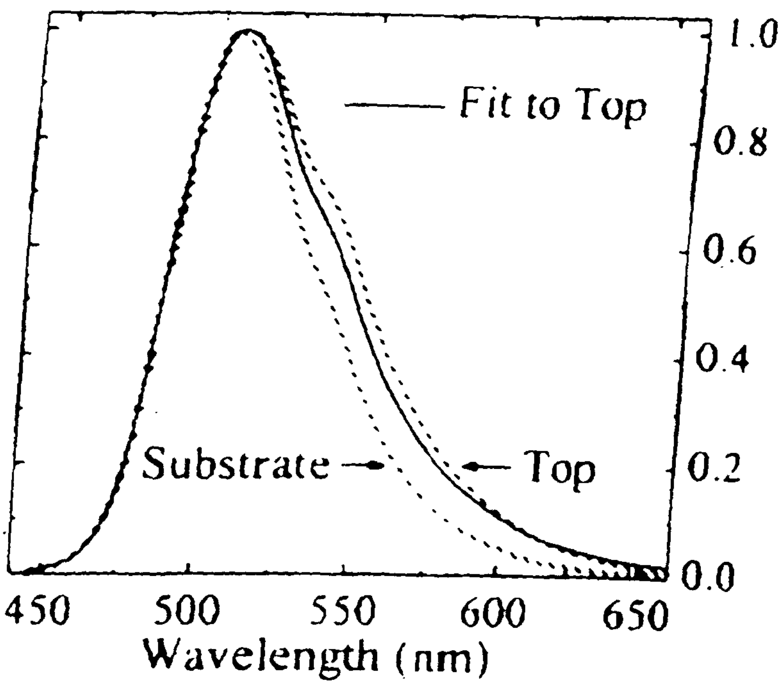
Fit to Top
Substrate
Top
1.0
0.8
0.6
0.4
0.2
0.0
450
500
550
600
650
Wavelength (nm)

Figure A16

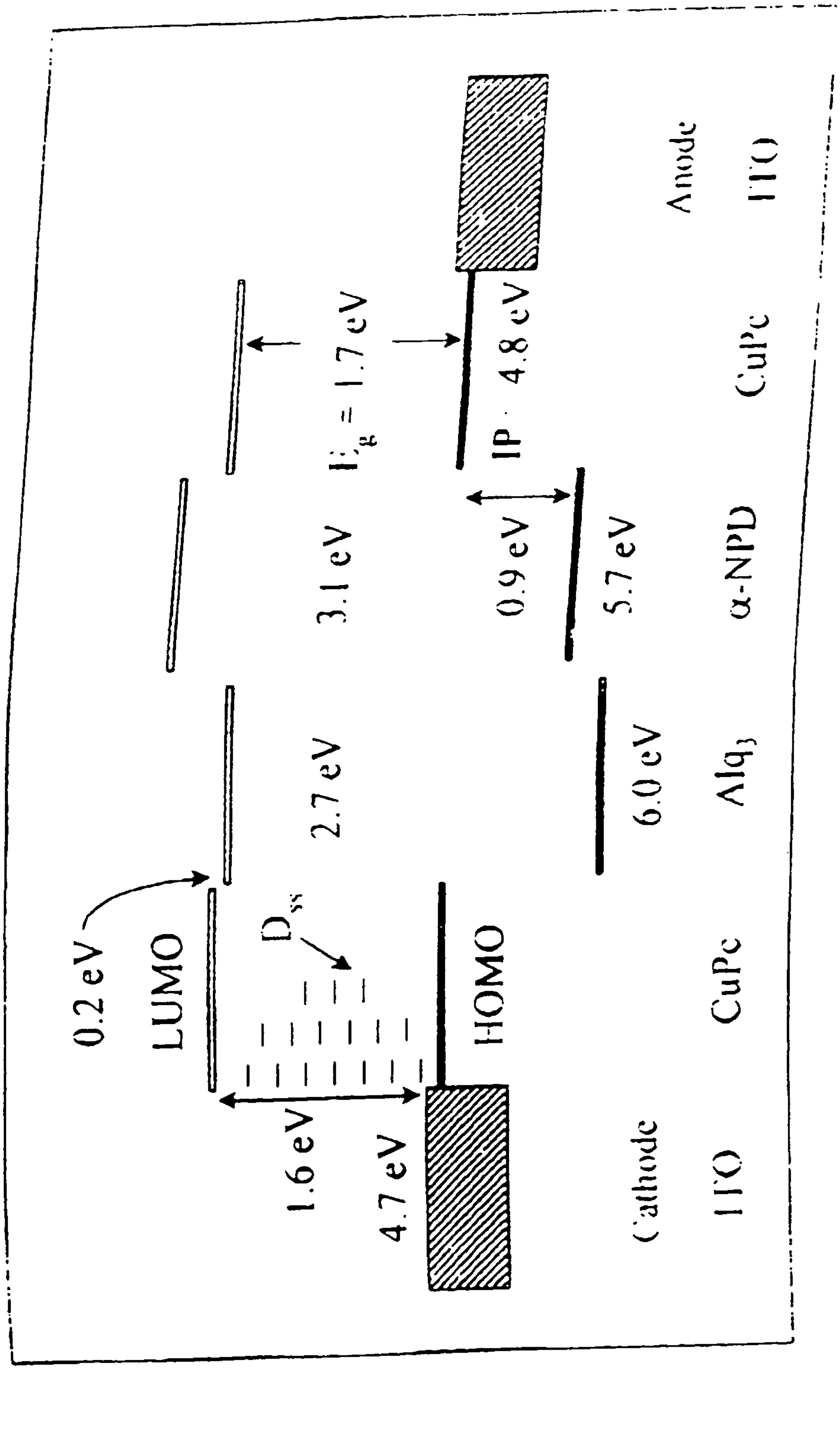
0.2 eV
LUMO
D$_{ss}$
HOMO
1.6 eV
4.7 eV
Cathode
ITO
CuPc
2.7 eV
6.0 eV
Alq$_3$
3.1 eV
0.9 eV
5.7 eV
α-NPD
E$_g$ = 1.7 eV
IP = 4.8 eV
CuPc
Anode
ITO

Figure A17

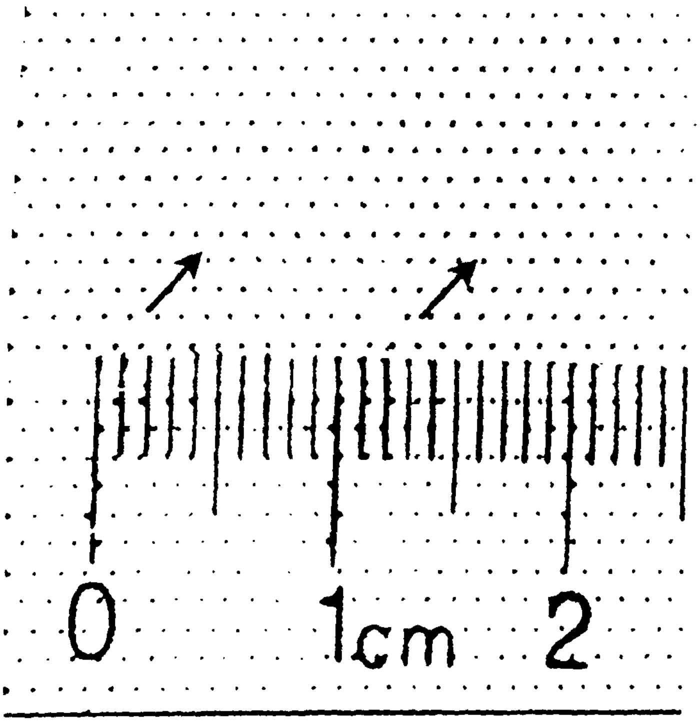

Figure B1

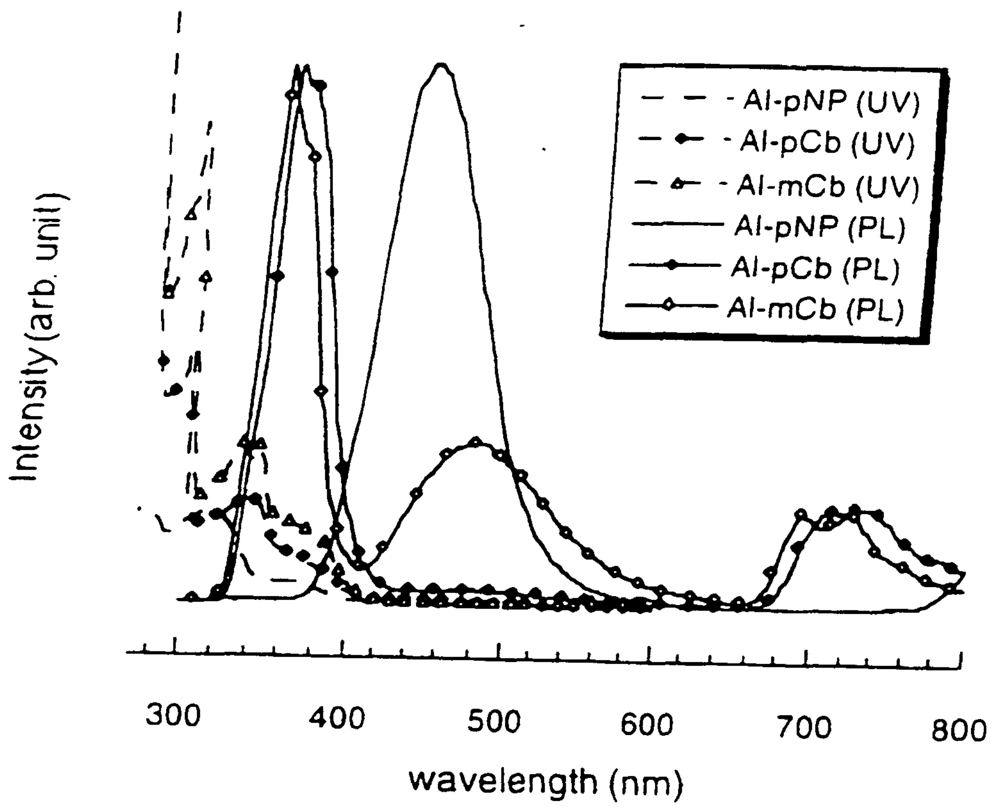


Absorption and Fluorescent Spectra of Al-pNP, Al-pCb, and Al-mCb in $CH_2Cl_2$ Solution

Figure B2

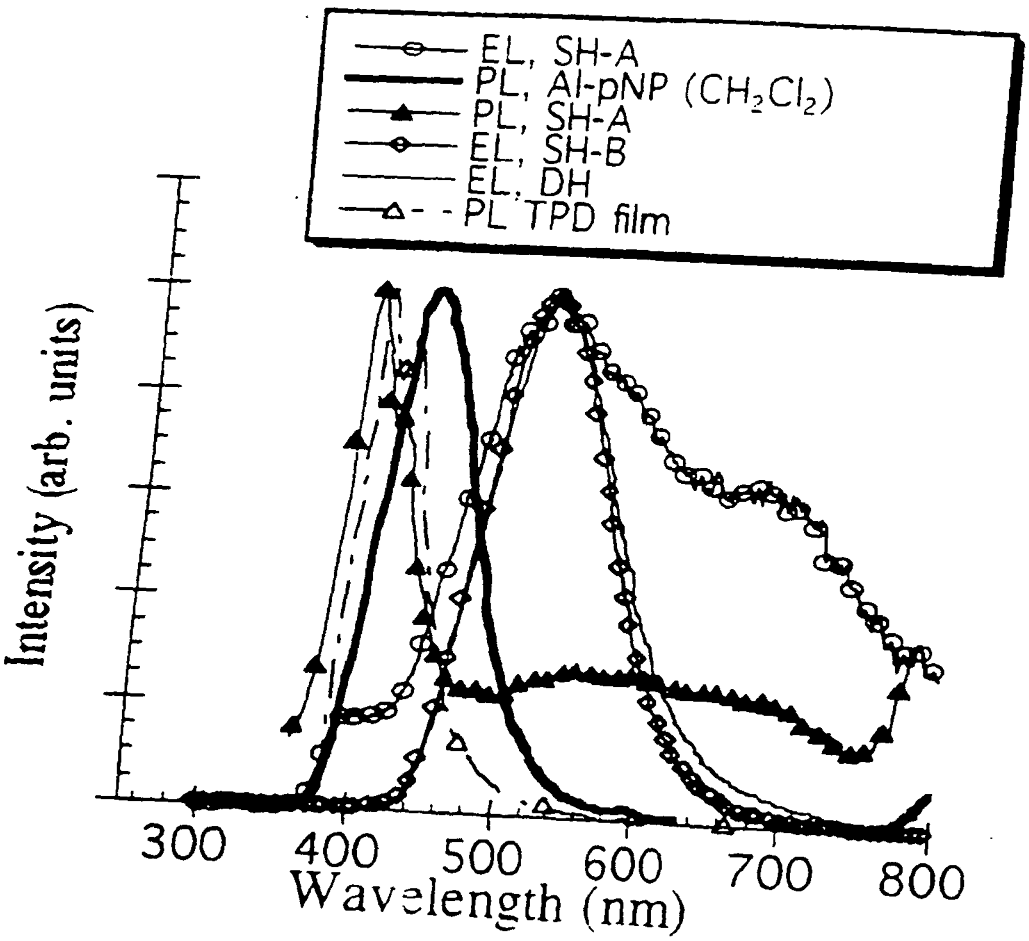


EL and PL of Al-pNP de·. ces.

Figure B3

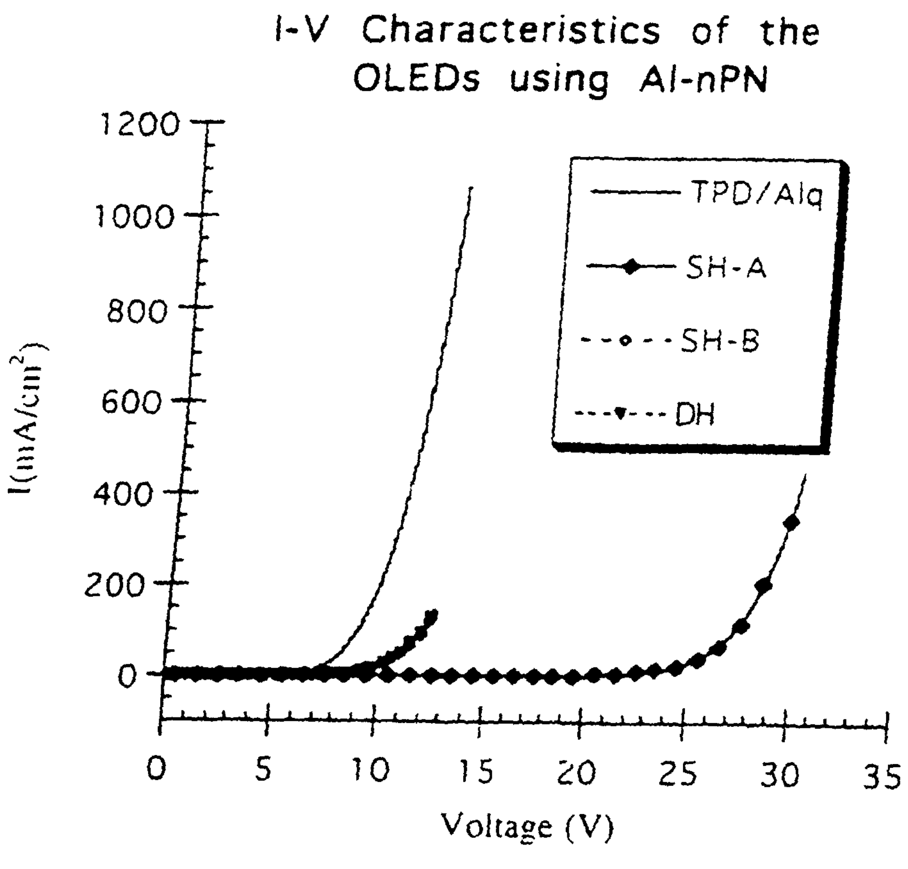
I-V Characteristics of the
OLEDs using Al-nPN
1200
1000
800
600
400
200
0
I(mA/cm²)
0
5
10
15
20
25
30
35
Voltage (V)
TPD/Alq
SH-A
SH-B
DH

Figure B4

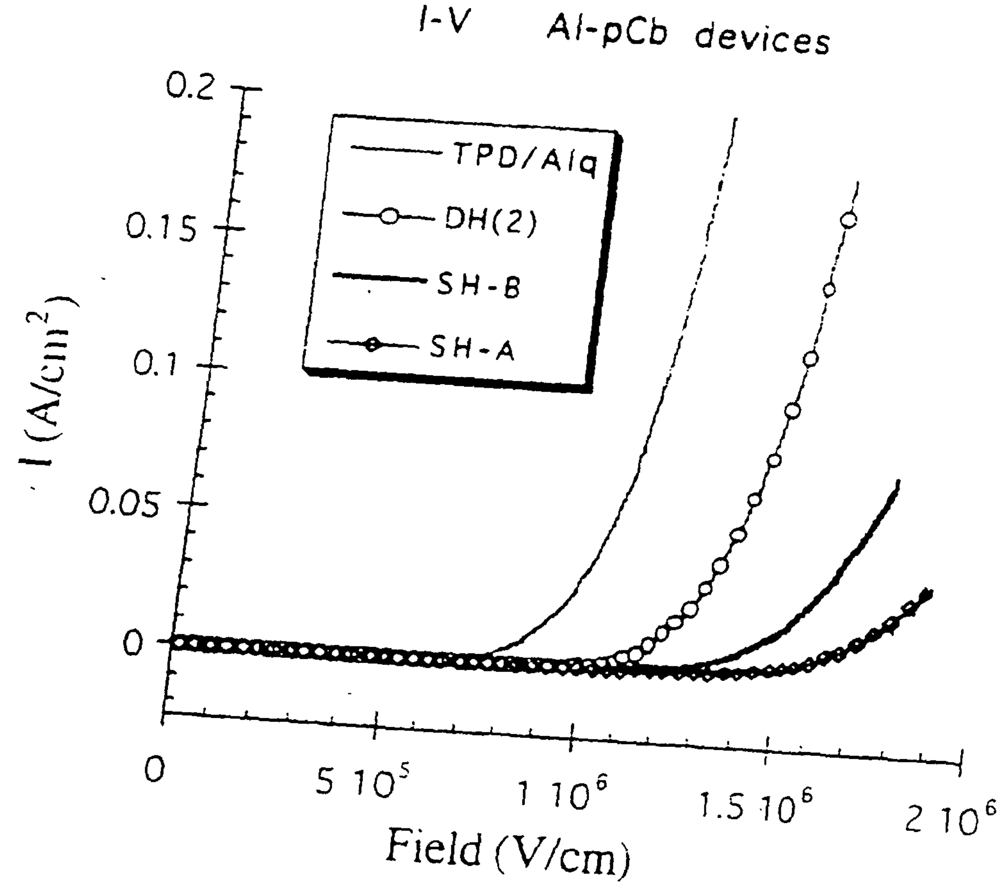
I-V Al-pCb devices
TPD/Alq
DH(2)
SH-B
SH-A
0.2
0.15
0.1
0.05
0
I (A/cm2)
0
5 10^5
1 10^6
1.5 10^6
2 10^6
Field (V/cm)

Figure B5

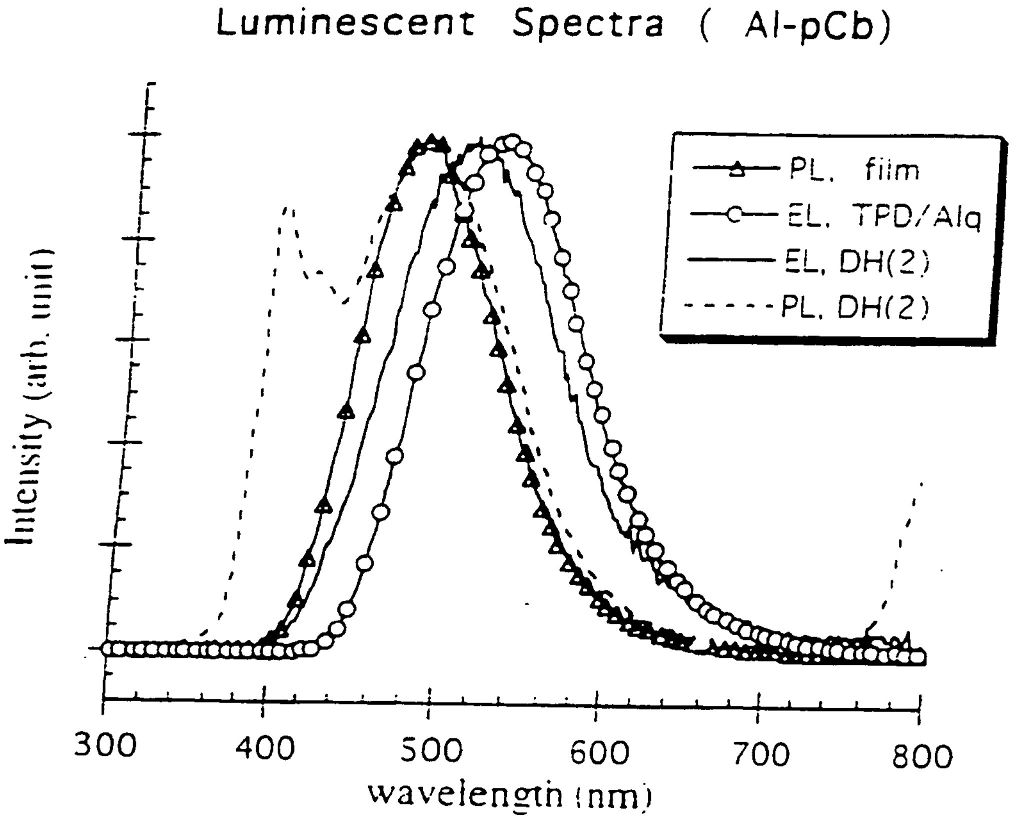
Luminescent Spectra ( Al-pCb)
PL. film
EL. TPD/Alq
EL, DH(2)
PL, DH(2)
Intensity (arb. unit)
300
400
500
600
700
800
wavelength (nm)

Figure B6

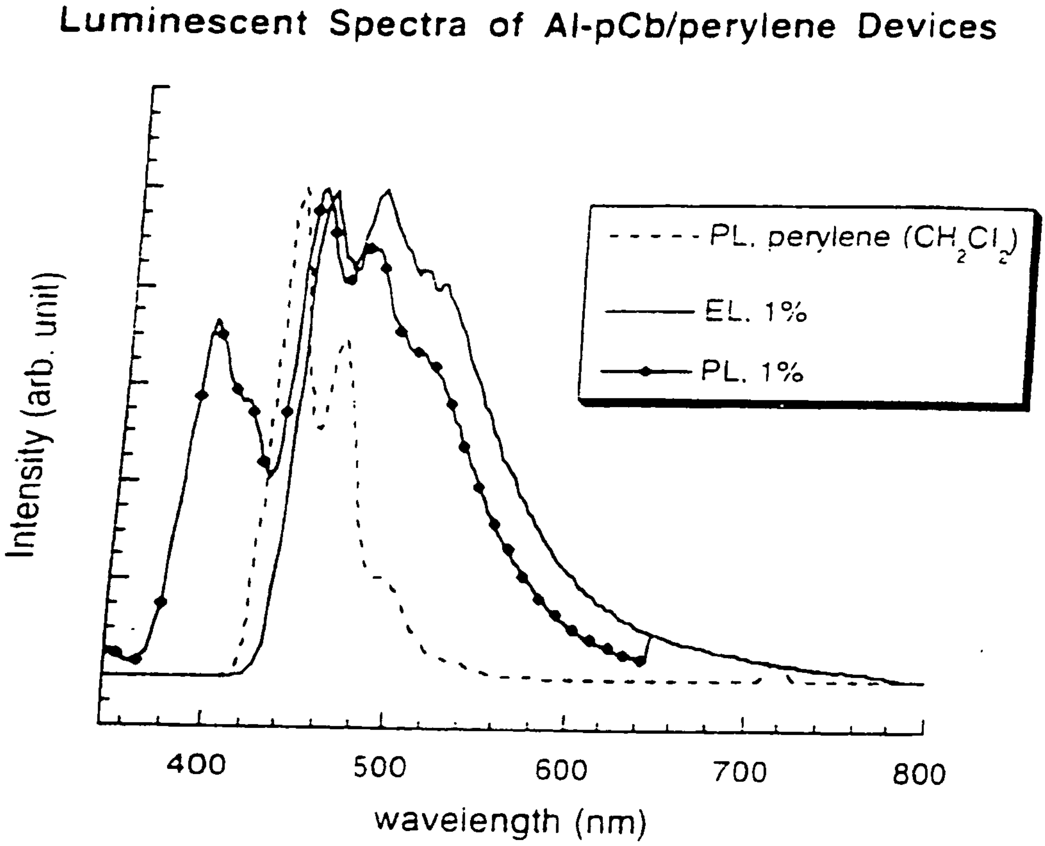
Luminescent Spectra of Al-pCb/perylene Devices
Intensity (arb. unit)
PL. perylene ($CH_2Cl_2$)
EL. 1%
PL. 1%
400
500
600
700
800
wavelength (nm)

Figure B7

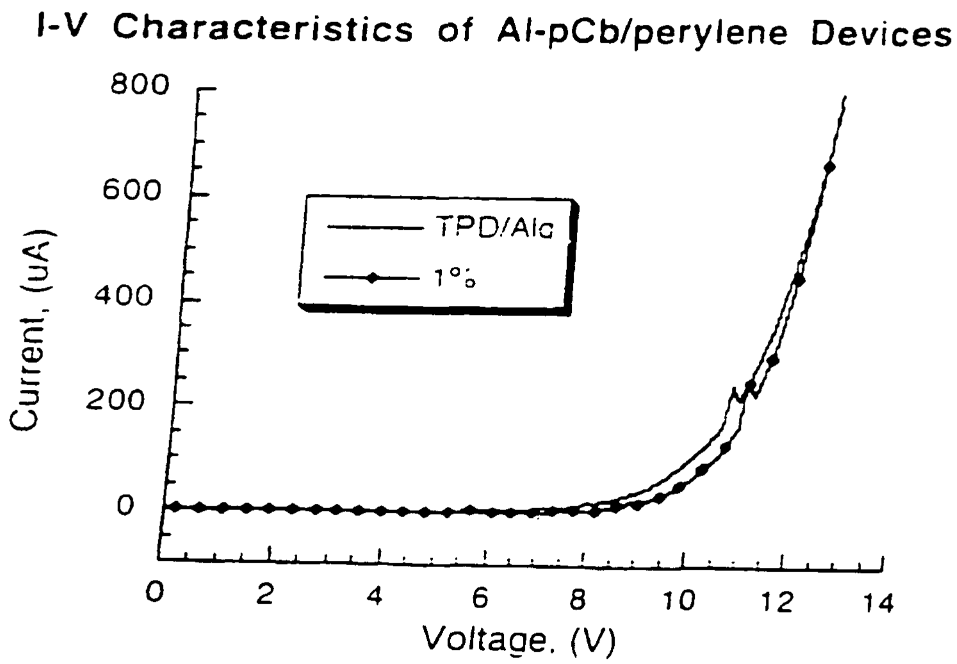
I-V Characteristics of Al-pCb/perylene Devices
800
600
400
200
0
Current, (uA)
TPD/Alq
1%
0
2
4
6
8
10
12
14
Voltage, (V)

Figure C1

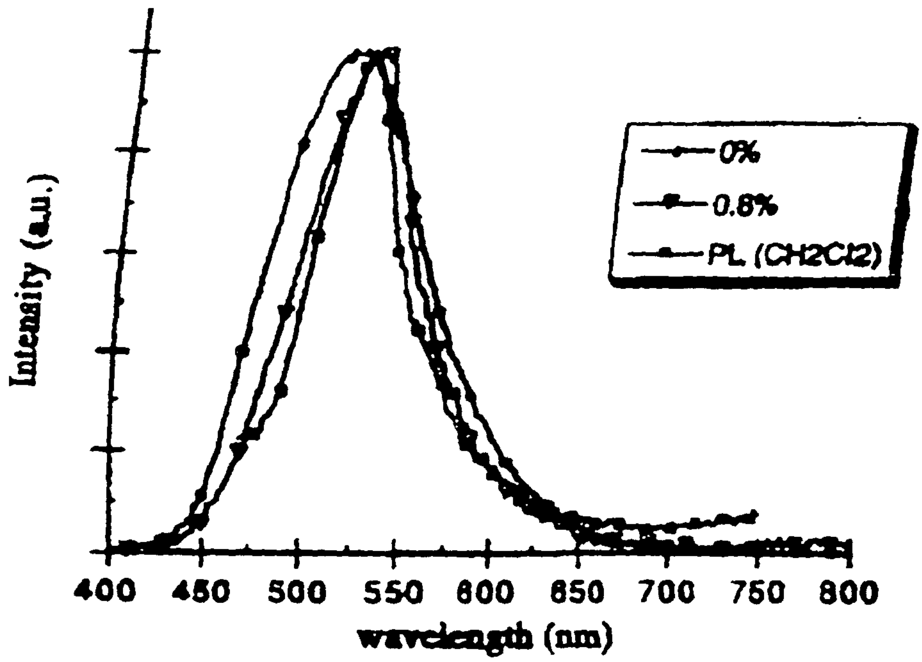
Intensity (a.u.)
0%
0.8%
PL (CH2Cl2)
400
450
500
550
600
650
700
750
800
wavelength (nm)

Figure C2

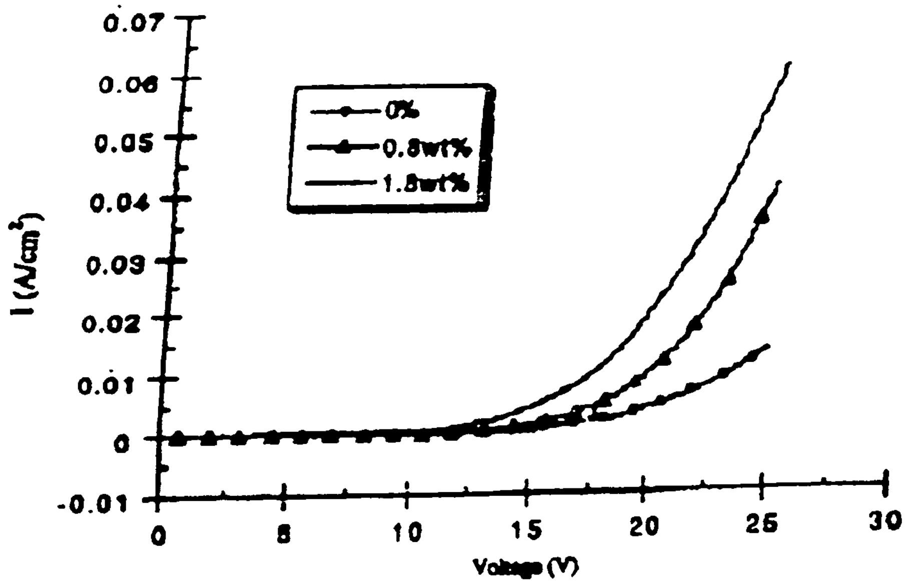
0.07
0.06
0.05
0.04
0.03
0.02
0.01
0
-0.01
I (A/cm$^2$)
0%
0.8wt%
1.8wt%
0
5
10
15
20
25
30
Voltage (V)

Figure D1

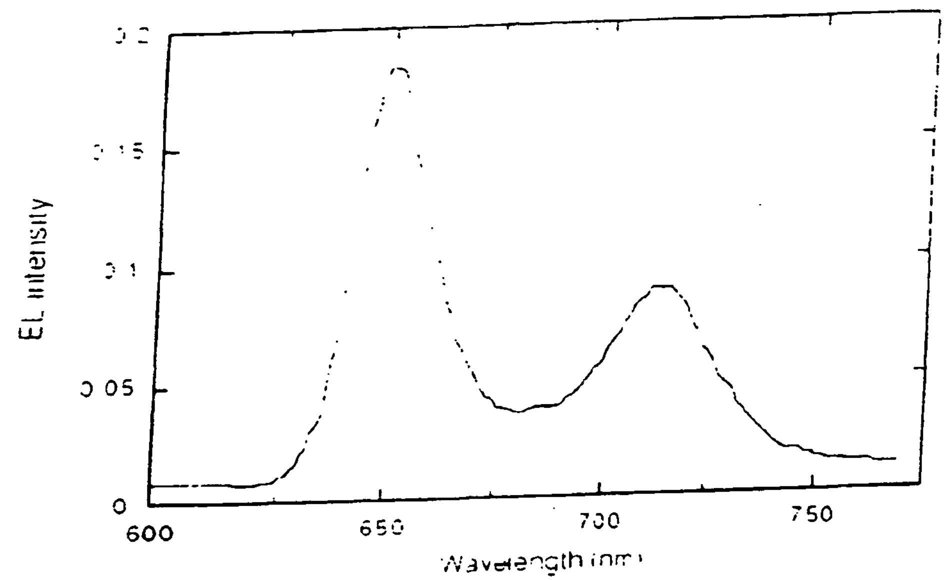

Figure D2

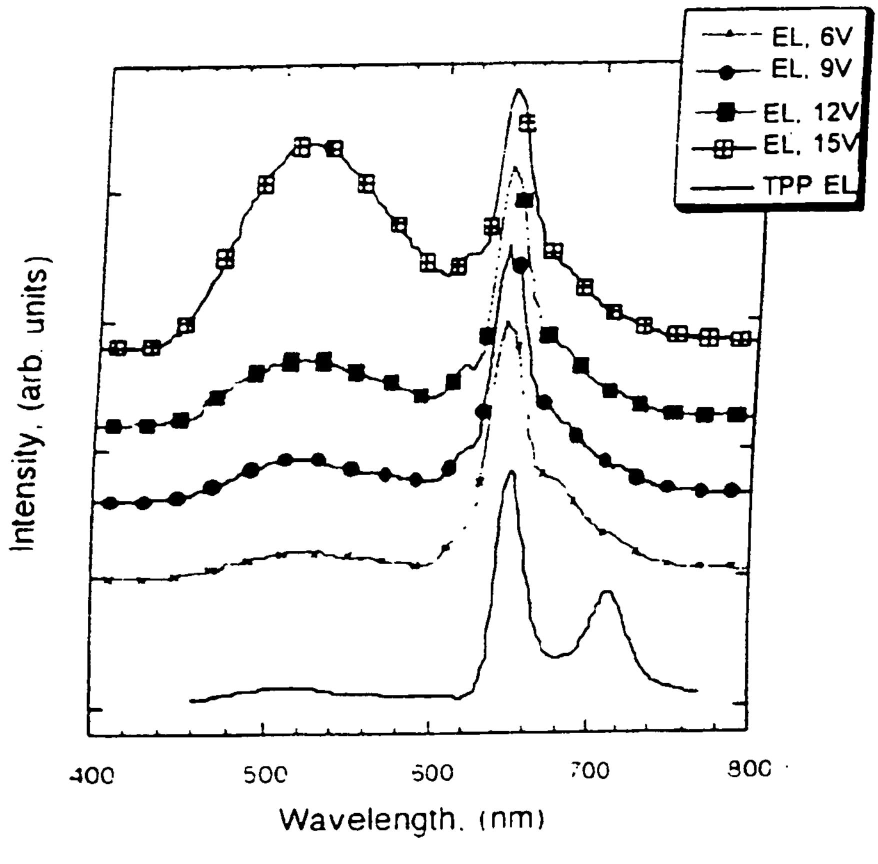
EL, 6V
EL, 9V
EL, 12V
EL, 15V
TPP EL
Intensity, (arb. units)
400
500
600
700
800
Wavelength, (nm)

Figure D3

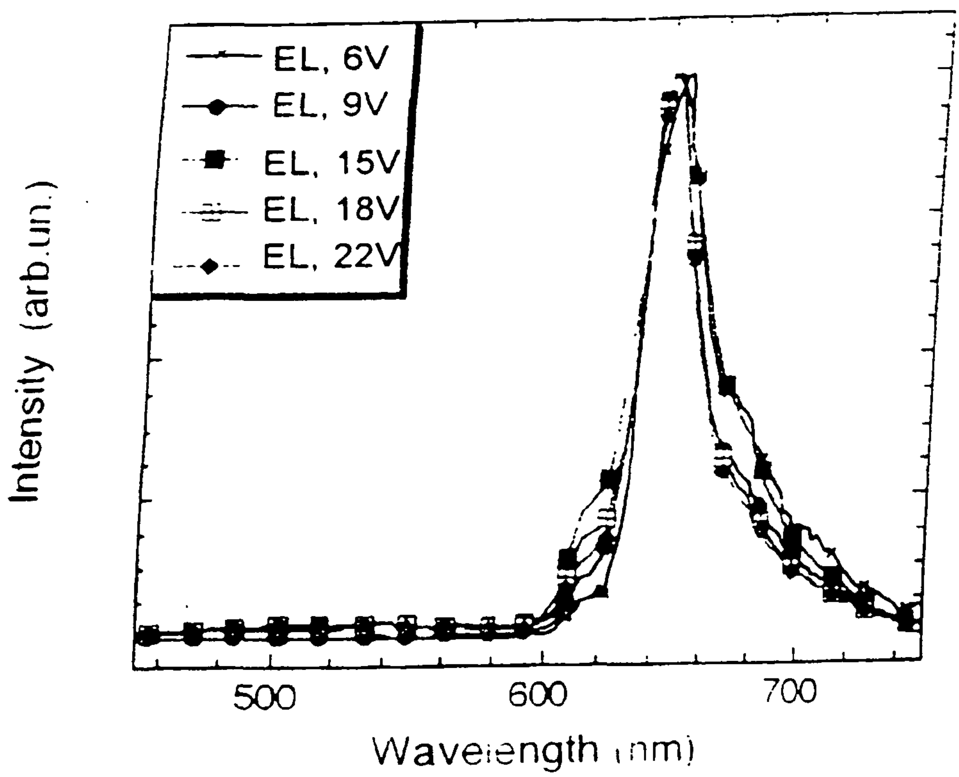
EL, 6V
EL, 9V
EL, 15V
EL, 18V
EL, 22V
Intensity (arb.un.)
500
600
700
Wavelength (nm)

Figure D4

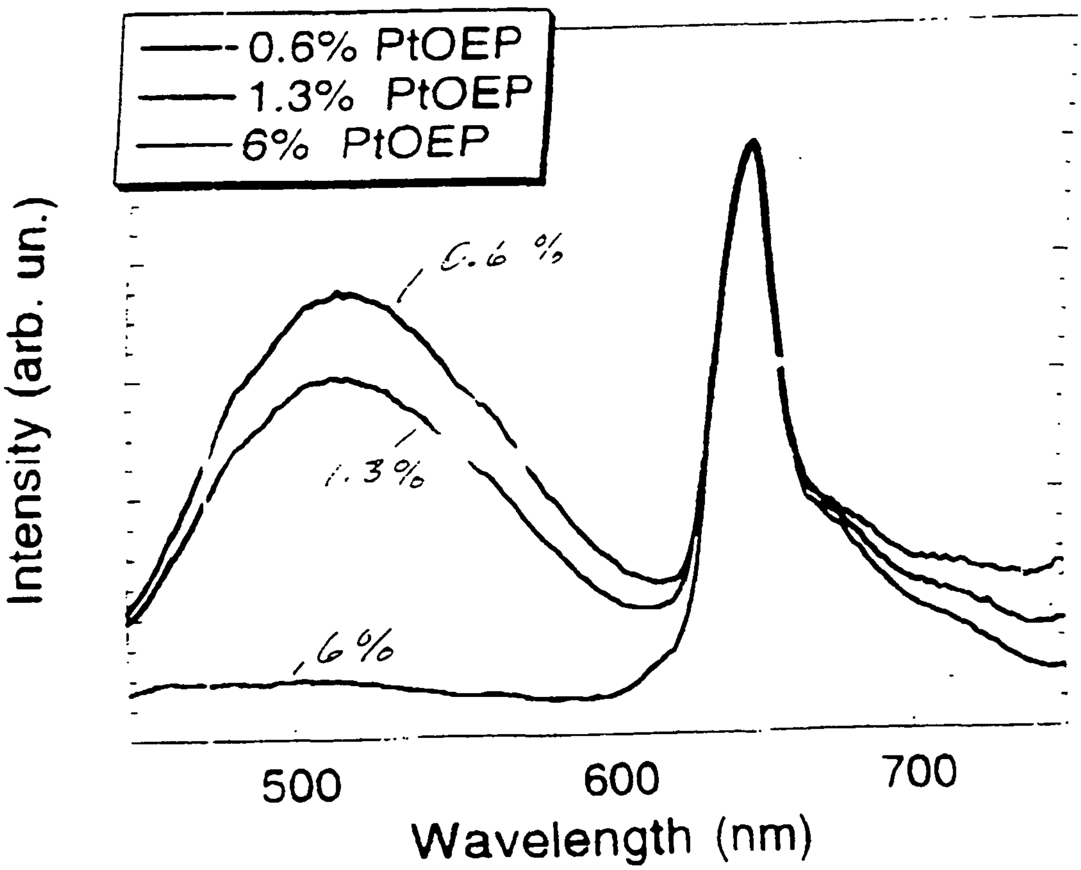
0.6% PtOEP
1.3% PtOEP
6% PtOEP
0.6 %
1.3%
6%
Intensity (arb. un.)
500
600
700
Wavelength (nm)

Figure D5

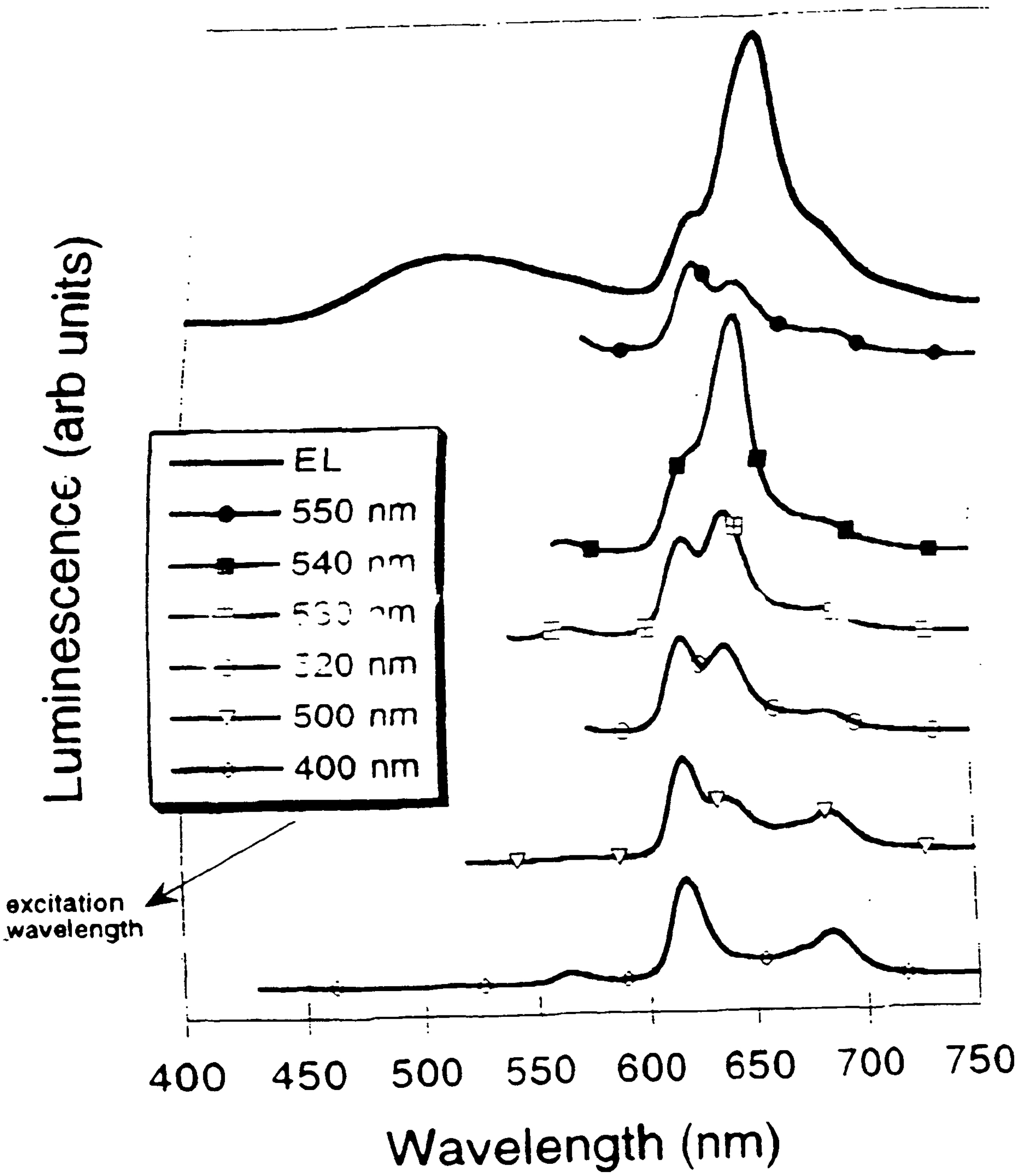
Luminescence (arb units)
EL
550 nm
540 nm
500 nm
400 nm
excitation wavelength
400
450
500
550
600
650
700
750
Wavelength (nm)

# Figure E1

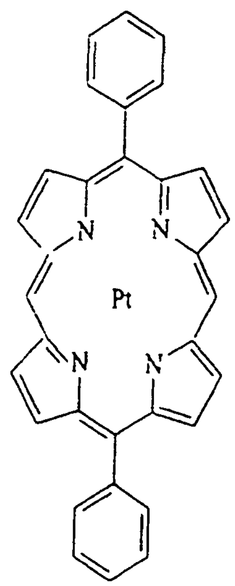

PtDPP

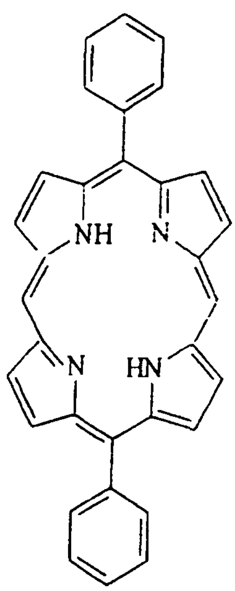

P$_2$DPP

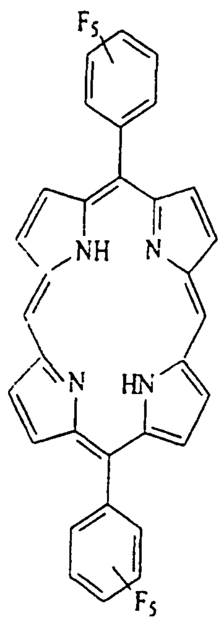

H$_2$BPFPP

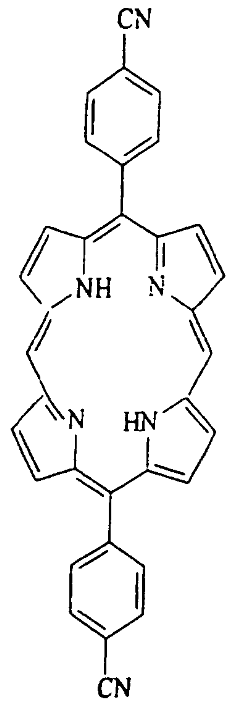

H$_2$BCPP

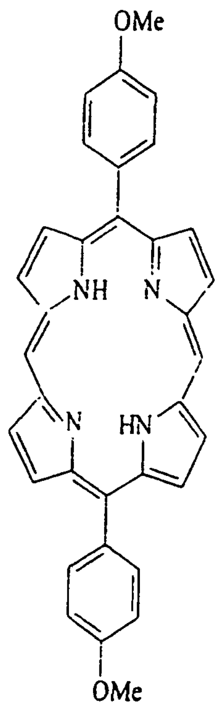

H$_2$BAP

Figure E2

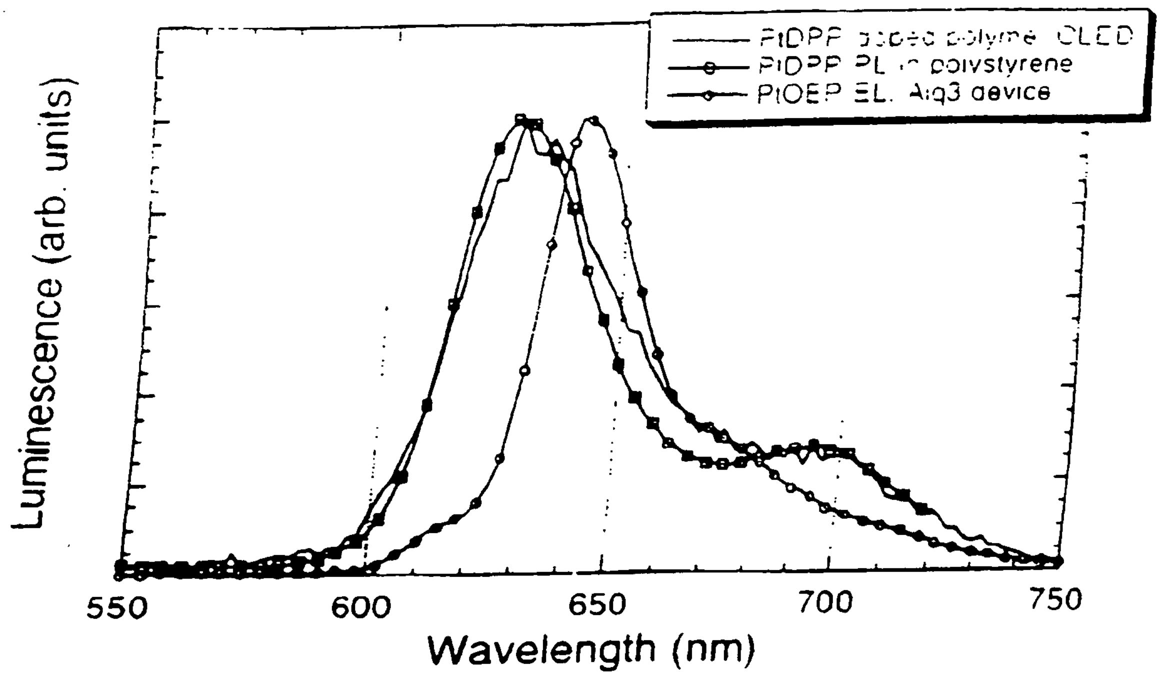
PtDPP
PtDPP PL polystyrene
PtOEP EL, Alq3 device
Luminescence (arb. units)
550
600
650
700
750
Wavelength (nm)

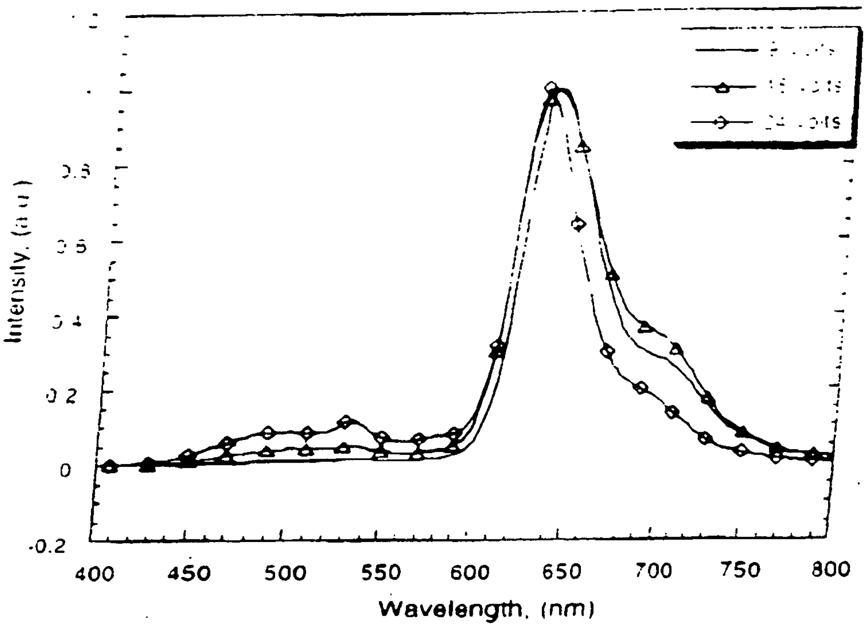


Figure E3a

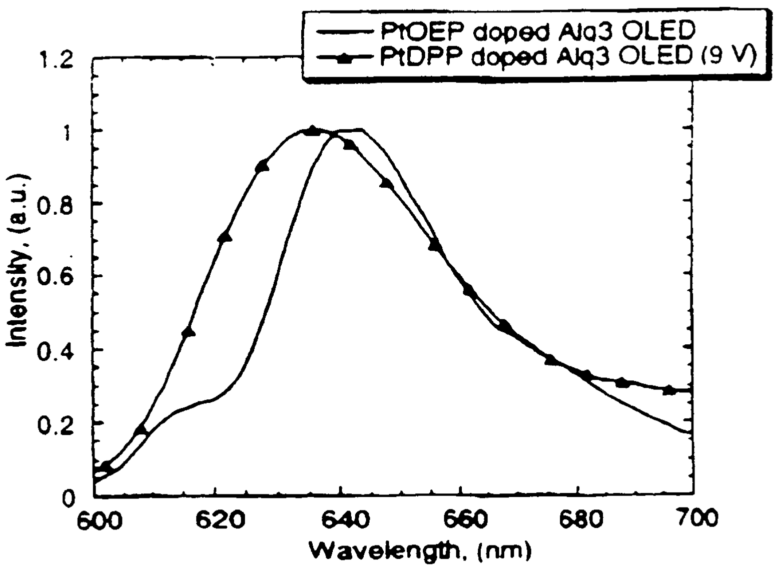


Figure E3b

Figure E4

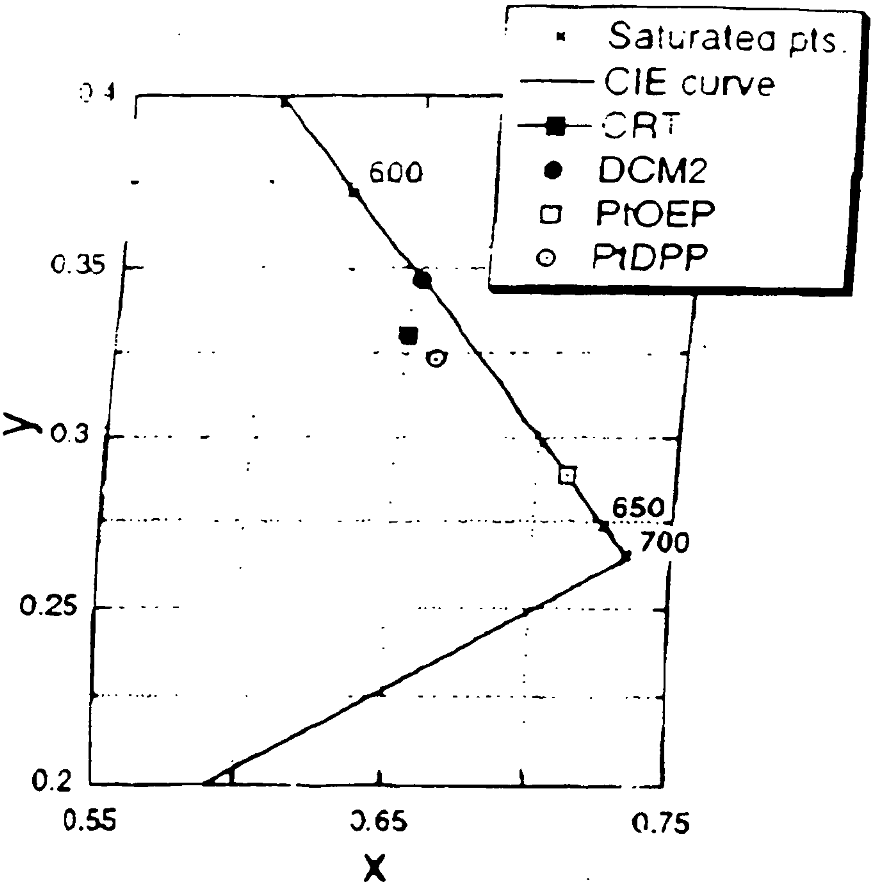
Saturated pts.
CIE curve
CRT
DCM2
PtOEP
PtDPP
0.4
0.35
0.3
0.25
0.2
y
600
650
700
0.55
0.65
0.75
x

Figure E5

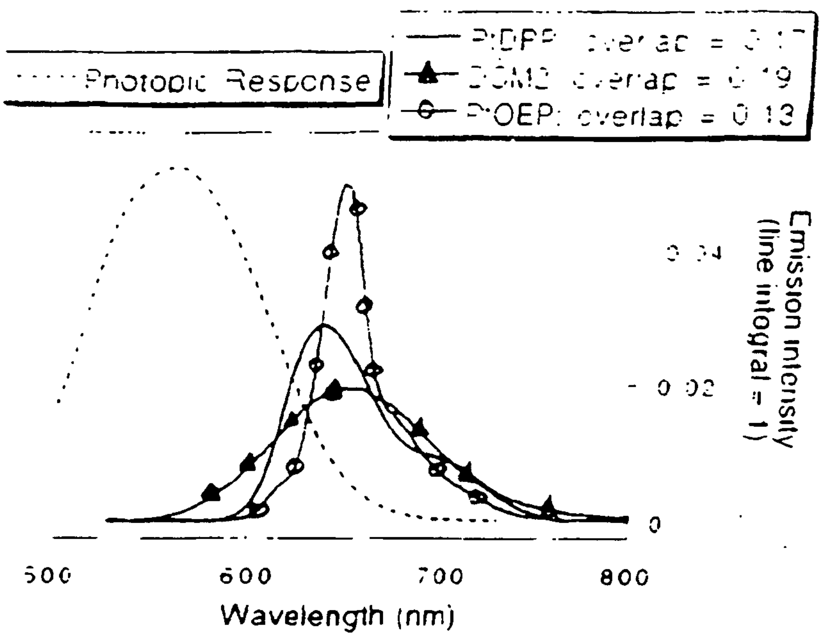
Photopic Response
OEP: overlap = 0.13
Emission Intensity
(line integral = 1)
0.02
0
500
600
700
800
Wavelength (nm)

Figure F1

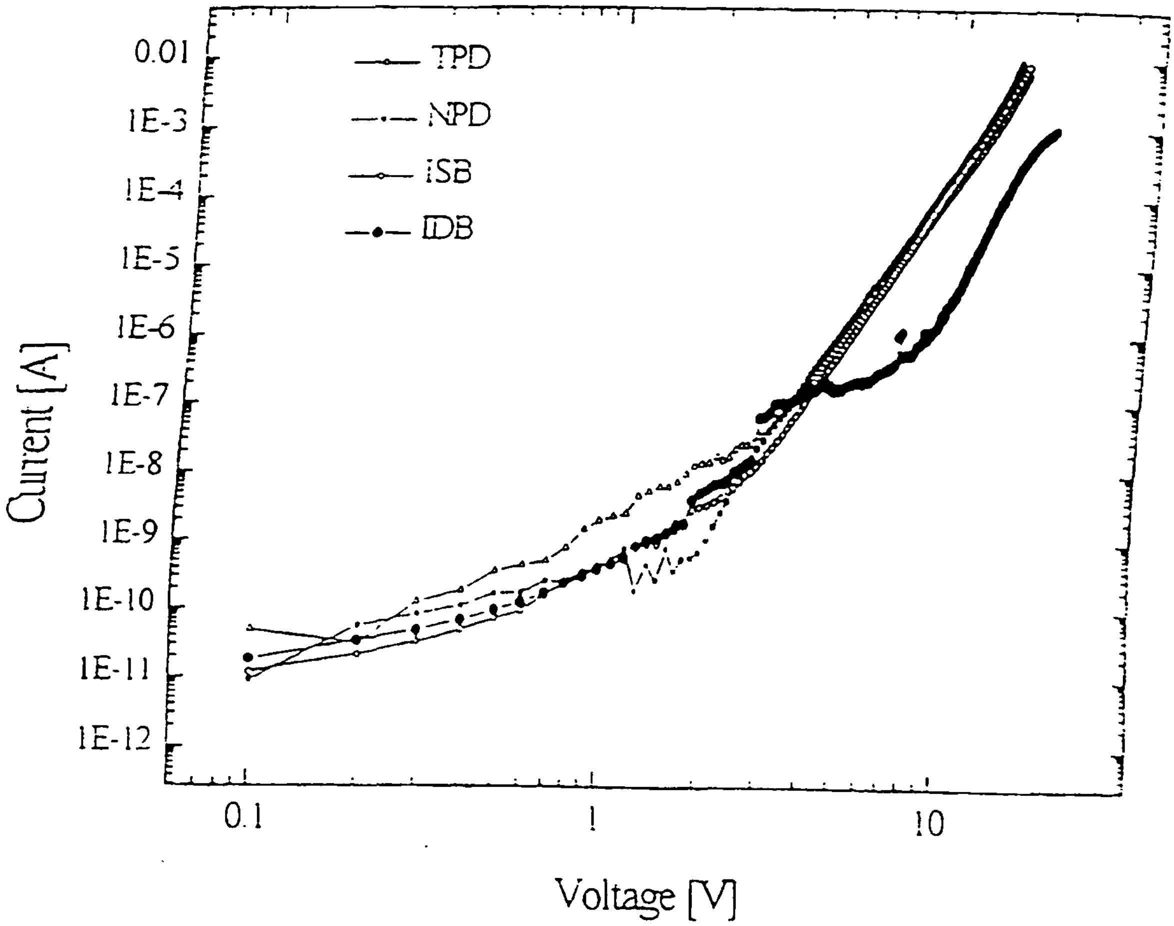
TPD
NPD
ISB
IDB
0.01
1E-3
1E-4
1E-5
1E-6
1E-7
1E-8
1E-9
1E-10
1E-11
1E-12
Current [A]
0.1
1
10
Voltage [V]